(19) Europäisches Patentamt
European Patent Office
Office européen des brevets

(11) EP 1 658 853 A1

# (12) EUROPEAN PATENT APPLICATION
published in accordance with Art. 158(3) EPC

(43) Date of publication:
**24.05.2006 Bulletin 2006/21**

(21) Application number: **04746855.8**

(22) Date of filing: **25.06.2004**

(51) Int Cl.:
***A61K 35/28*** *(1985.01)* ***A61K 35/14*** *(1985.01)*
***A61K 35/34*** *(1985.01)* ***A61P 25/00*** *(2000.01)*
***A61P 25/28*** *(2000.01)*

(86) International application number:
**PCT/JP2004/009386**

(87) International publication number:
**WO 2005/007176 (27.01.2005 Gazette 2005/04)**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **27.06.2003 JP 2003185260**
**26.12.2003 JP 2003432329**

(71) Applicants:
- **Renomedix Institute Inc.**
  **Ishikari-shi,**
  **Hokkai-do 0613241 (JP)**
- **Mitsui Sumitomo Insurance Co., Ltd.**
  **Tokyo 104-0033 (JP)**
- **Teikoku Hormone Mfg. Co., Ltd.**
  **Tokyo 108-8532 (JP)**
- **HITACHI, LTD.**
  **Chiyoda-ku**
  **Tokyo 100-8280 (JP)**

(72) Inventors:
- **HONMOU, Osamu**
  **Sapporo-shi,**
  **Hokkaido 064-0801 (JP)**
- **HAMADA, Hirofumi**
  **Sapporo-shi,**
  **Hokkaido 064-0959 (JP)**

(74) Representative: **Vossius & Partner**
**Siebertstrasse 4**
**81675 München (DE)**

## (54) REMEDY FOR INTERNAL ADMINISTRATION AGAINST CRANIAL NERVE DISEASES CONTAINING MESENCHYMAL CELLS AS THE ACTIVE INGREDIENT

(57) Intravenous administration of bone marrow cells collected from rat bone marrow to a rat cerebral infarction model was found to be effective in treating cerebral infarction. Human and murine bone marrow stem cells showed similar effects. Mesenchymal cells such as bone marrow cells, cord blood cells, or peripheral blood cells can be used as agents for *in vivo* administration against cranial nerve diseases.

EP 1 658 853 A1

## Description

### Technical Field

The present invention relates to cranial nerve disease therapeutic agents for *in vivo* administration, which comprise mesenchymal cells, particularly bone marrow cells, cord blood cells, or peripheral blood cells, or cells derived from these cells as active ingredients.

### Background Art

In recent years regenerative medical techniques have been in the limelight. In regenerative medical techniques, disorders that the natural, inherent regenerative-healing ability of the human body cannot cure can be cured by regenerating organs and such using artificial proliferation of autologous cells, and then surgically conjugating these at the site of the lesions. Such cures have been successful in a wide variety of fields.

Transplantation of oligodendroglia (oligodendrocytes) (see Non-patent Documents 1 to 3), or myelin-forming cells, such as Schwann cells (see Non-patent Documents 4, 2, and 5) or olfactory ensheathing cells (see Non-patent Documents 6 to 8), can elicit remyelination in animal models and electrophysiological function may be recovered (see Non-patent Documents 9 and 5). It is not impossible to prepare such cells from patients or other persons for use in cell therapy; however, it is problematic since tissue material must be collected from either the brain or nerves.

Neural progenitor cells or stem cells derived from the brain have the ability to self-proliferate, and are known to differentiate into neurons and glial cells of various lineages (see Non-patent Documents 10 to 13). Upon transplantation into newborn mouse brains, human neural stem cells collected from fetal tissues differentiate into neurons and astrocytes (see Non-patent Documents 14 to 16), and can remyelinate axons (Non-patent Document 17). There have been reports of the remyelination and recovery of impulse conduction when neural progenitor cells derived from adult human brains are transplanted into demyelinated rodent spinal cords (Non-patent Document 18).

These studies have evoked great interest since they indicate the possibility of applying the above-mentioned cells in reparative strategies for neurological diseases (see Non-patent Documents 18, 14 to 16, and 19).

Recent studies have revealed that neural stem cells can produce hematopoietic cells *in vivo*, indicating that neural progenitor cells are not limited to nervous system cell lineages (see Non-patent Document 20). Further, when bone marrow interstitial cells are injected into newbom mouse lateral ventricles, they differentiate, to a very small extent, into cells expressing astrocyte markers (see Non-patent Document 21). Under appropriate cell culture conditions bone marrow interstitial cells are reported to produce a very small number of cells that express nervous system cell markers *in vitro*; however, it is unclear whether these cells are useful for neural regeneration (see Non-patent Document 22).

The present inventors have previously extracted and cultured nervous system cells (neural stem cells, neural progenitor cells) from adult human brains, and established some cell lines. By studying the functions of these cells, the inventors discovered that neural stem cells are pluripotent and can self-reproduce (see Non-patent Document 18). Specifically, single-cell expansion of neural progenitor (stem) cells obtained from adult human brains was conducted to establish cell lines; the established cells were then subjected to *in vitro* clonal analysis. The results demonstrated that the cell lines were pluripotent (namely, had the ability to differentiate into neurons, astroglia (or astrocytes), and oligodendroglia (i.e., oligodendrocytes)) and had self-reproducing ability (namely, proliferation potency). Thus, these cells were confirmed to possess the characteristics of neural stem cells.

Transplantation of cultured neural stem cells, which were extracted from small amounts of neural tissue collected from the cerebrum of an individual, into a lesion of the brain or spinal cord of the individual, seems to be a widely applicable therapeutic method in autotransplantation therapy. However, although it doesn't cause symptoms of neurological deficiency, collecting tissues that contain neural stem cells from the cerebrum is not easy. Thus, considering the current need to establish therapeutic methods for various complicated diseases of the nervous system, it is crucial to establish safer and simpler methods for autotransplantation therapy. Thus, to obtain donor cells, the present inventors have developed techniques for collecting mononuclear cell fractions and the like from bone marrow cells, cord blood cells, or fetal liver cells, which is simpler than collecting neural stem cells (see Patent Document 1). Specifically, the present inventors have shown that mononuclear cell fractions prepared from bone marrow cells have the ability to differentiate into nervous system cells. They also have shown that cell fractions containing mesodermal stem cells (mesenchymal stem cells), stromal cells, and AC133-positive cells, which were separated from the mononuclear cell fraction, also had the ability to differentiate into nervous system cells.

Cranial nerve diseases can be treated by directly administering an affected part in the brain with cells that have the ability to differentiate into the above-mentioned nervous system cells. This technique, however, is very complicated and dangerous. There is therefore much demand for the development of simple and safe methods and agents for treating cranial nerve diseases.

[Patent Document]WO 02/00849

[Non-patent Document 1]Archer DR, *et al*. 1994. Exp Neurol 125:268-77.
[Non-patent Document 2]Blakemore WF, Crang AJ. 1988. Dev Neurosci 10:1-11.
[Non-patent Document 3]Gumpel M, *et al*. 1987. Ann New York Acad Sci 495:71-85.
[Non-patent Document 4]Blakemore WF. 1977. Nature 266:68-9.
[Non-patent Document 5]Honmou O, *et al*. 1996. J Neurosci 16:3199-208.
[Non-patent Document 6]Franklin RJ, *et al*. 1996. Glia 17:217-24.
[Non-patent Document 7]Imaizumi T, *et al*. 1998. J Neurosci 18(16):6176-6185.
[Non-patent Document 8]Kato T, *et al*. 2000. Glia 30:209-218.
[Non-patent Document 9]Utzschneider DA, *et al*. 1994. Proc Natl Acad Sci USA 91:53-7.
[Non-patent Document 10]Gage FH, *et al*. 1995. Proc Natl Acad Sci USA 92:11879-83.
[Non-patent Document 11]Lois C, Alvarez-Buylla A. 1993. Proc Natl Acad Sci USA 90:2074-7.
[Non-patent Document 12]Morshead CM, *et al*. 1994. Neuron 13:1071-82.
[Non-patent Document 13]Reynolds BA, Weiss S. 1992. Science 255:1707-10.
[Non-patent Document 14]Chalmers-Redman RM, *et al*. 1997. Neurosci 76:1121-8.
[Non-patent Document 15]Moyer MP, *et al*. 1997. Transplant Proc 29:2040-1.
[Non-patent Document 16]Svendsen CN, *et al*. 1997. Exp Neurol 148:135-46.
[Non-patent Document 17]Flax JD, *et al*. 1998. Nat Biotechnol 16:1033-9.
[Non-patent Document 18]Akiyama Y, *et al*. 2001. Exp Neurol.
[Non-patent Document 19]Yandava BD, *et al*. 1999. Proc Natl Acad Sci USA 96:7029-34.
[Non-patent Document 20]Bjornson CR, *et al*. 1999. Science 283:534-7.
[Non-patent Document 21]Kopen GC, *et al*. Proc Natl Acad Sci USA 96:10711-6.
[Non-patent Document 22]Woodbury *D, et al*. 2000. J Neurosci Res 61:364-70.

Disclosure of the Invention

The present invention was achieved under these circumstances, and an objective of the present invention is to provide safe techniques and agents for treating cranial nerve diseases. More specifically, an objective is to provide agents for treating cranial nerve diseases for *in vivo* administration, particularly for intravenous administration, comprising mesenchymal cells, particularly bone marrow cells, cord blood cells, or peripheral blood cells, or cells derived from these cells as an active ingredient.

The present inventors made intensive investigations to achieve the above objectives. Initially, they investigated the therapeutic effect on cranial nerve diseases of: collecting bone marrow cells from mouse bone marrow, separating only the mononuclear cell fraction therefrom, and using this isolated fraction as donor cells for intravenous administration to a rat cerebral infarction model. Consequently, they surprisingly found that not only local administration, but also intravenous administration of bone marrow cells exhibits a therapeutic effect on cranial nerve diseases such as cerebral infarction, spinal cord injuries, and demyelinating diseases.

The present inventors made further studies on the therapeutic effects of bone marrow stem cells (mesenchymal stem cells) on cranial nerve diseases by intravenously administering them to the above mentioned model animal in the same manner. They found that intravenous administration of bone marrow stem cells is very effective for treating cranial nerve diseases.

They also found that intravenous administration or local administration of autologous bone marrow cells or mesenchymal stem cells is effective for treating cranial nerve diseases. Compared to allotransplantation and xenotransplantation, autotransplantation is extremely advantageous in terms of therapeutic effects and further does not require immunosuppressive drugs.

As described above, the present inventors discovered the therapeutic effects of intravenously administering mesenchymal cells (mesenchymal stem cells), particularly bone marrow cells or mesenchymal stem cells, on cranial nerve diseases. The present invention has been achieved based on these findings. As shown below in the Examples, the present inventors have verified the therapeutic effects on cranial nerve diseases of intravenously administering the mesenchymal cells of the present invention by carrying out various medical or biological experiments and detailed analysis.

Specifically, mesenchymal cells (mesenchymal stem cells), and particularly bone marrow cells themselves, can become intravenously administered agents for therapies of cranial nerve diseases.

The above mentioned therapeutic effects are considered to be synergistic, including the neuroprotective and neural regenerative effects of bone marrow cells or mesenchymal stem cells. Accordingly, bone marrow cells or mesenchymal stem cells are expected to be intravenously administered cranial nerve protectants or cranial nerve regenerants.

The present invention relates to agents for treating cranial nerve diseases that are administered *in vivo,* and particularly that are intravenously administered, where the agents comprise mesenchymal cells, particularly bone marrow cells, cord blood cells, or peripheral blood cells, or cells derived from these cells as an active ingredient. The present invention also relates to agents *for in vivo* administration that exhibit neuroprotective or regenerative actions on cranial nerves, which comprise the above mentioned mesenchymal cells as an active ingredient, and use of the agents. More specifically, the present invention provides:

[1] A cranial nerve disease therapeutic agent for *in vivo* administration, comprising a mesenchymal cell as an active ingredient.
[2] The agent of [1], wherein the cranial nerve disease is cerebral infarction.
[3] An agent *for in vivo* administration, exhibiting neuroprotection and comprising a mesenchymal cell as an active ingredient.
[4] An agent *for in vivo* administration, exhibiting cranial nerve regeneration and comprising a mesenchymal cell as an active ingredient.
[5] The agent of any one of [1] to [4], wherein the *in vivo* administration is intravenous.
[6] The agent of any one of [1] to [5], wherein the mesenchymal cell is:

(a) a mesenchymal cell introduced with a BDNF gene, PLGF gene, GDNF gene, or IL-2 gene; or
(b) an immortalized mesenchymal cell introduced with an hTERT gene.

[7] The agent of any one of [1] to [6], wherein the mesenchymal cell is a mesenchymal stem cell.
[8] The agent of any one of [1] to [6], wherein the mesenchymal cell is a bone marrow cell, a cord blood cell, or a peripheral blood cell.
[9] A method for treating a cranial nerve disease comprising the *in vivo* administration to a patient of a therapeutically effective amount of the agent of any one of [1] to [8].
[10] The method of [9], wherein the bone marrow cell is an autologous cell of the patient.
[11] The method of [9] or [10], wherein the cranial nerve disease is cerebral infarction.
[12] The method of any one of [9] to [11], wherein the *in vivo* administration is intravenous administration.
[13] The method of any one of [9] to [12], wherein the mesenchymal cell is a bone marrow cell, a cord blood cell, or a peripheral blood cell.

The present invention provides cranial nerve disease therapeutic agents for *in vivo* administration, wherein the agents comprise mesenchymal cells (for example, bone marrow cells, cord blood cells, peripheral blood cells, mesenchymal stem cells, or cells derived from these cells) as an active ingredient.

Herein the term "*in vivo* administration" generally means administration at a site other than the head (brain). The *in vivo* administration includes intravenous administration, intramuscular administration, subcutaneous administration, and intraperitoneal administration, and of these intravenous administration is most preferred.

Herein the term "mesenchymal cells" preferably refers to, for example, bone marrow cells (mononuclear cell fraction of bone marrow cells; MCF (mononuclera cell fraction)), cord blood cells, peripheral blood cells, mesenchymal stem cells (MSCs), or cells derived from these cells. The mesenchymal cells of the present invention include, for example, mesenchyme-related cells, mesoblastic stem cells, and so on. Even if cells referred to as "mesenchymal cells" in the present invention are classified as cells other than mesenchymal cells in the future, the cells can still be suitably used in the present invention.

The stem cells included in bone marrow are hematopoietic stem cells and "mesenchymal stem cells (MSCs)". Herein "stem cells" generally mean undifferentiated cells with self-proliferation ability and the ability to differentiate into cells which have specific functions in physiological processes, such as the proliferation and differentiation of cells constituting living bodies. Hematopoietic stem cells are stem cells that differentiate into red blood cells, white blood cells, or thrombocytes. Mesenchymal stem cells may differentiate via neural stem cells into nerves, differentiate directly into nerves without going via neural stem cells, differentiate via stromal cells into nerves (but with low efficiency), differentiate into viscera, differentiate into the blood vascular system, or differentiate into bone, cartilage, fat, or muscle.

The present invention mainly uses mesenchymal stem cells (MSCs), but there is also the possibility of using hematopoietic stem cells and other stem cells (progenitor cells) in the body. The mesenchymal stem cells can be obtained from bone marrow cells, collected from the bone marrow. Bone marrow cells from which mesenchymal stem cells are not separated can also be used for the treatments, as for the mesenchymal stem cells, although the efficacy of the former is somewhat less the latter.

Preparing cells such as mesenchymal stem cells from the peripheral blood is also thought possible. In fact, the present inventors have successfully induced cultured cells, derived from cells contained in the peripheral blood, to differentiate into cells capable of developing cell markers of neural stem cells and nervous system cells (neurons and glial cells). G-CSF or SCF is not always necessary when inducing cells derived from the peripheral blood to differentiate into nervous system cells. Specially, the present inventors have found that, when mesoblastic stem cells (mesenchymal stem cells) prepared from a mononuclear cell fraction separated from bone marrow fluid or umbilical cord blood, or embryonic stem cells (ES cells), are cultivated in a basal culture medium, the mesoblastic stem cells (mesenchymal stem cell) or ES cells are induced to differentiate into neural stem cells, neurons, or glial cells. Accordingly, cells with functions equivalent to mesenchymal stem cells can be prepared by cultivating cells, from peripheral blood, and such cells can be used in the present invention. The "basal culture media" is not limited, as long as they are regular culture media used in cell cultivation, and they are preferably DMEM (Dulbecco's modified essential medium) or NPBM (Neural progenitor cell basal medium: Clonetics). Other components of the above mentioned basal culture medium are not particularly limited, and preferably contains F-12, FCS, and/or Neural survival factors (Clonetics), and so on. The concentration within this culture medium may be, for example, 50% for F-12 and/or 1% for FCS. The $CO_2$ concentration of the culture medium is preferably 5%, but is not limited thereto.

As used herein, the term "mesodermal stem cell" refers to a cell constituting tissues embryologically categorized into the class of mesoderm, including blood cells. A "mesodermal stem cell" is also a cell that can make copies of itself (divide and proliferate), with the same potency as that of the original cell, and with the ability to differentiate into all cell types constituting mesodermal tissues. The mesodermal stem cell expresses, for example, the cell markers SH2(+), SH3(+), SH4(+), CD29(+), CD44(+), CD14(-), CD34(-), and CD45(-), but such cells are not limited to these markers. Furthermore, so-called mesenchyme-related stem cells are also included in the mesodermal stem cells of the present invention.

The above term "mesenchyme-related cell" refers to mesenchymal stem cells, mesenchymal cells, precursor cells of mesenchymal cells and cells derived from mesenchymal cells.

The term "mesenchymal stem cell" refers to stem cells that can be obtained from bone marrow, peripheral blood, skin, hair root, muscle tissue, uterine endometrium, blood, cord blood and primary cultures of various tissues. Furthermore, cells functionally equivalent to mesenchymal stem cells obtainable by culturing cells in the peripheral blood are also comprised in the mesenchymal stem cells of the present invention.

Preferred mesenchymal cells in the present invention are bone marrow cells and bone marrow stem cells (mesenchymal stem cell). Cord blood cells, peripheral blood cells, and fetal liver cells are also preferable examples in the present invention.

A preferred embodiment of bone marrow cells, cord blood cells, peripheral blood cells, and fetal liver cells in the present invention is a cell fraction which is isolated from bone marrow cells, cord blood cells, peripheral blood, or fetal liver and comprises cells capable of differentiating into nervous system cells.

In another embodiment, the cell fraction is a cell fraction containing mesoblastic stem cells characterized by SH2 (+), SH3 (+), SH4 (+), CD29 (+), CD44 (+), CD 14 (-), CD34(-), and CD45(-).

Other examples of the cell fraction are cell fractions containing interstitial cells characterized by Lin(-), Sca-1 (+), CD10(+), CD11D(+), CD44(+) CD45(+), CD71(+), CD90(+), CD105(+), CDW123(+), CD127(+), CD164(+), fibronectin (+), ALPH(+), and collagenase-1 (+), or cell fractions containing cells characterized by AC133(+).

Cells contained in the above mentioned cell fractions are preferably cells capable of differentiating into nervous system cells.

The cell fractions in the present invention comprise mononuclear cell fractions, which were separated from bone marrow cells, and which contain cells characterized by their ability to differentiate into nervous system cells. Another embodiment is a mononuclear cell fraction separated from, for example, cord blood cells, peripheral blood cells, or fetal liver cells which contain cells characterized by their ability to differentiate into nervous system cells. Yet another embodiment is mesenchymal stem cells from the bone marrow that are released into the peripheral blood, and which are characterized by their ability to differentiate into nervous system cells. Active substance or agents, for example, can be used when mesenchymal stem cells are released into the peripheral blood, but these substances are not always necessary.

Mesenchymal stem cells collected from the bone marrow and those derived from the peripheral blood possess common characteristics in the development of markers of neural stem cells and/or nervous system cells, but differ from each other in some properties, such as proliferation rate and rate of differentiation induction. The mesenchymal stem cells for use in the present invention are not limited to those collected from the bone marrow but also include those derived from the peripheral blood. Specifically, the "mesenchymal stem cells" in the present invention include both of these cells. In the present invention, the mesenchymal stem cells derived from the peripheral blood may also be simply referred to as "mesenchymal cells".

It is unclear whether the differentiation of cells contained in the cell fractions of the present invention into neural cells is caused by the transformation of so-called hematopoietic cells into neural cells, or, alternatively, by the differentiation of immature cells capable of differentiating into neural cells that are comprised in bone marrow cells, cord blood cells, or peripheral blood cells. However, the majority of the cells differentiating into neural cells are assumed to be stem or precursor cells, namely, cells having pluripotency and the ability to self-propagate. Alternatively, the cells differentiating into neural cells may be stem or precursor cells which have differentiated to some extent into endoderm or mesoderm.

Cells in a cell fraction of the present invention do not have to be proliferated with any trophic factors (but proliferation in the presence of trophic factors is possible). Thus, these cells are simple and practical from the standpoint of the development of autotransplantation technique for nervous system, and are very beneficial to the medical industry. In general, bone marrow cells, cord blood cells, or peripheral blood cells (cell fractions) of the present invention are derived from vertebrates, preferably from mammals (for example, mice, rats, rabbits, swine, dogs, monkeys, humans, etc.), but are not especially limited.

A cell fraction of the present invention can be prepared, for example, by subjecting marrow cells or cord blood cells collected from vertebrate animals to density-gradient centrifugation at 2,000 rpm in a solution for a sufficient time to ensure separation, depending on specific gravity, and then recovering the cell fraction with a certain specific gravity in the range of 1.07 to 1.1 g/ml. Herein, the phrase "a sufficient time to ensure separation, depending on specific gravity" refers to a time, typically about ten to 30 minutes, sufficient for the cells to shift to positions in the solution for density-gradient centrifugation that accord with their specific gravity. The specific gravity of the cell fraction to be recovered is within the range of 1.07 to 1.08 g/ml (for example, 1.077 g/ml). Solutions such as Ficoll solution and Percoll solution can be used for the density-gradient centrifugation, but there is no limit thereto. Furthermore, cord blood cells collected from vertebrate animals may be prepared in a similar manner as described above, and can be used as a cell fraction.

Specifically, first, bone marrow (5 to 10 μl) collected from a vertebrate animal is combined with a solution (2 ml L-15 plus 3 ml Ficoll), and then centrifuged at 2,000 rpm for 15 minutes to isolate a mononuclear cell fraction (approx. 1 ml). The mononuclear cell fraction is combined with culture solution (2ml NPBM) to wash the cells, and then the cells are again centrifuged at 2,000 rpm for 15 minutes. Then, after removing the supernatant, the precipitated cells are recovered. In addition to the femur, sources for obtaining a cell fraction of the present invention include the sternum and the ilium, which constitutes the pelvis. Any other bone can serve as a source, as long as it is large enough. A cell fraction of the present invention can also be prepared from bone marrow fluid stored in a bone marrow bank, or from cord blood. When using cord blood cells, the cells can be obtained from cord blood stored in a bone marrow bank.

Another embodiment of the cell fractions of the present invention includes mononuclear cell fractions isolated and purified from bone marrow cells, cord blood cells, or peripheral blood cells, which contains mesodermal (mesenchymal) stem cells capable of differentiating into neural cells. A cell fraction containing mesodermal stem cells can be obtained, for example, by selecting cells with a cell surface marker, such as SH2 as described above, from the above-mentioned cell fraction obtained by centrifuging bone marrow cells, cord blood cells, or peripheral blood cells.

Furthermore, a cell fraction containing mesodermal stem cells (mesenchymal stem cells) capable of differentiating into neural cells can be prepared by subjecting bone marrow cells or cord blood cells collected from vertebrate animals to density-gradient centrifugation at 900 G in a solution for a sufficient time to ensure separation, depending on specific gravity, and then recovering the cell fraction with a certain specific gravity within the range of 1.07 to 1.1 g/ml. Herein, the phrase "a sufficient time to ensure separation, depending on specific gravity" refers to a time, typically about ten to 30 minutes, sufficient for the cells to shift to positions in the solution for density-gradient centrifugation that accord with their specific gravity. The specific gravity of a cell fraction to be recovered varies depending on the type of animal (for example, human, rat, or mouse) from which the cells have been derived. Solutions for density-gradient centrifugation include Ficoll solution and Percoll solution, but are not limited thereto.

Specifically, first, bone marrow (25 ml) or cord blood collected from a vertebrate animal is combined with an equal volume of PBS solution, and then centrifuged at 900 G for ten minutes. Precipitated cells are mixed with PBS and then recovered (cell density = approx. $4x\ 10^7$ cells /ml) to remove blood components. Then, a 5-ml aliquot thereof is combined with Percoll solution (1.073 g/ml), and centrifuged at 900 G for 30 minutes to extract a mononuclear cell fraction. The extracted mononuclear cell fraction is combined with a culture solution (DMEM, 10% FBS, 1% antibiotic-antimycotic solution) to wash the cells, and is centrifuged at 2,000 rpm for 15 minutes. Finally, the supernatant is removed, and the precipitated cells are recovered and cultured at 37°C under 5% $CO^2$ atmosphere.

Another embodiment of a cell fraction of the present invention is a fraction of mononuclear cells isolated from bone marrow cells or cord blood cells, which contains stromal cells capable of differentiating into neural cells. Examples of stromal cells include cells characterized by Lin(-), Sca-1(+), CD10(+), CD11D(+), CD44(+), CD45(+), CD71(+),CD90 (+), CD105(+), CDW123(+), CD127(+), CD164(+), fibronectin (+), ALPH(+), and collagenase-1(+). A cell fraction containing stromal cells can be prepared, for example, by selecting cells with a cell surface marker, such as Lin as described above, from the above-mentioned cell fraction obtained by centrifuging bone marrow cells or cord blood cells.

Furthermore, such cell fractions can be prepared by subjecting bone marrow cells or cord blood cells collected from vertebrate animals to density-gradient centrifugation at 800 G in a solution for a sufficient time to ensure separation, depending on specific gravity, and then recovering the cell fraction with a certain specific gravity within the range of 1.07 to 1.1 g/ml. Herein, "a sufficient time ensuring separation depending on the specific gravity" indicates a time, typically about ten to 30 minutes, sufficient for the cells to shift to positions in the solution for density-gradient centrifugation that accord with their specific gravity. The specific gravity of the cell fraction to be recovered is preferably in the range of 1.07 to 1.08 g/ml (for example, 1.077 g/ml). Solutions for density-gradient centrifugation include Ficoll solution and Percoll solution, but are not limited thereto.

Specifically, first, bone marrow or cord blood collected from vertebrate animals is combined with an equal volume of a solution (PBS, 2% BSA, 0.6% sodium citrate, and 1% penicillin-streptomycin). A 5-ml aliquot thereof is combined with Ficoll + Paque solution (1.077 g/ml) and centrifuged at 800 G for 20 minutes to obtain a mononuclear cell fraction. The mononuclear cell fraction is combined with a culture solution (Alfa MEM, 12.5% FBS, 12.5% horse serum, 0.2% i-inositol, 20 mM folic acid, 0.1 mM 2-mercaptoethanol, 2 mM L-glutamine, 1 μM hydrocortisone, 1% antibiotic-antimycotic solution) to wash the cells, and then centrifuged at 2,000 rpm for 15 minutes. After centrifugation the supernatant is removed. The precipitated cells are collected and then cultured at 37°C under 5% $CO^2$ atmosphere.

Another embodiment of a cell fraction of the present invention is a mononuclear cell fraction containing cells characterized by AC133(+) which can differentiate into neural cells, and which is isolated from bone marrow cells, cord blood cells, peripheral blood cells, or fetal liver tissues. Such cell fractions can be obtained, for example, by selecting cells with a cell surface marker of the above-mentioned AC133(+) from the cell fraction obtained as described above by centrifuging bone marrow cells, cord blood cells, or peripheral blood cells.

Further, in other embodiments, the cell fractions can be obtained by subjecting fetal liver tissues collected from vertebrate animals to density-gradient centrifugation at 2,000 rpm in a solution for a sufficient time to ensure separation, depending on specific gravity, then recovering a cell fraction with a specific gravity in the range of 1.07 to 1.1 g/ml, and then recovering cells with AC133(+) characteristics from the cell fraction. Herein, "a sufficient time ensuring separation depending on specific gravity" refers to a time, typically about ten to 30 minutes, sufficient for the cells to shift to positions in the solution for density-gradient centrifugation that accord with their specific gravity. The solutions for density-gradient centrifugation include Ficoll solution and Percoll solution, but are not limited thereto.

Specifically, first, liver tissue collected from vertebrate animals is washed in L-15 solution, and then enzymatically treated for 30 minutes at 37°C in an L-15 solution containing 0.01% DNaseI, 0.25% trypsin, and 0.1% collagenase. Then, the tissue is dispersed into single cells by pipetting. These single fetal liver cells are centrifuged by the same procedure as that described for the preparation of mononuclear cell fractions from femur in Example 1(1). The cells thus obtained are washed, and then AC133(+) cells are collected from the washed cells using an AC133 antibody. Thus, cells capable of differentiating into neural cells can be prepared from fetal liver tissues. The antibody-based recovery of AC133(+) cells can be achieved using magnetic beads or a cell sorter (FACS, etc.).

Transplanting any of these cell fractions containing mesodermal stem cells (mesenchymal stem cells), interstitial cells, or AC133-positive cells into demyelinated spinal cords can lead to efficient remyelination of demyelinated regions. In particular, the above-mentioned cell fractions containing mesodermal stem cells (mesenchymal stem cells) can favorably engraft and differentiate into nervous system cells or glial cells when transplanted into a cerebral infarction model.

The cells capable of differentiating into neural cells, which are contained in the above-mentioned cell fractions, include for example, neural stem cells, mesodermal stem cells (mesenchymal stem cells), interstitial cells, and AC133-positive cells which are contained in the above-mentioned cell fractions, but are not limited thereto as long as they can differentiate into neural cells.

The active ingredients of the cranial nerve disease therapeutic agents for *in vivo* administration of the present invention comprise not only bone marrow cells, cord blood cells, or peripheral blood cells, but also the above-mentioned cell fractions. In the present invention it is possible to administer mesenchymal cells such as bone marrow cells, cord blood cells, or peripheral blood cells without any modification. However, to improve the efficiency of therapy, they may be administered as agents (compositions) to which various agents have been added, or as cells to which genes with the function of increasing therapeutic effect have been introduced. The preparation of agents or transgenic cells of the present invention may comprise, but is not limited to:

(1) adding a substance that improves the proliferation rate of cells included in a cell fraction, or that enhances the differentiation of cells into nervous system cells, or introducing a gene having the same effect;
(2) adding a substance that improves the viability of cells in damaged neural tissues included in a cell fraction, or introducing a gene having the same effect (e.g., reduction of radicals);
(3) adding a substance that inhibits the adverse effects of damaged neural tissues on the cells in a cell fraction, or introducing a gene having the same effect;
(4) adding a substance that prolongs the lifetime of donor cells, or introducing a gene having the same effect (e.g., the hTERT gene);
(5) adding a substance that modulates the cell cycle, or introducing a gene having the same effect;
(6) adding a substance aimed at suppressing immunoreaction, or introducing a gene having the same effect;
(7) adding a substance that enhances energy metabolism, or introducing a gene having the same effect;
(8) adding a substance that improves the migration ability of donor cells in host tissues, or introducing a gene having the same effect;
(9) introducing a substance that improves blood flow (including the induction of angiogenesis), or a gene having the same effect (e.g., VEGF, angiopoietin, or PGF);

(10) adding a substance having neuroprotection activity, or introducing a gene having the same effect (e.g., BDNF, GDNF, NT, NGF, FGF, EGF, or PFG);

(11) adding a substance having an apoptosis inhibitory effect, or introducing a gene having the same effect; or

(12) adding a substance having an antitumor effect, or introducing a gene having the same effect (e.g., IL-2 or IF-β).

The present inventors verified that mesenchymal stem cells (MSCs) introduced with BDNF (brain-derived neurotrophic factor) gene, which is a nerve nutritional factor, have therapeutic effects on a rat cerebral infarction model, as shown in the Examples below. In addition, the present inventors confirmed that intravenous transplantation of MSCs introduced with the BDNF gene has therapeutic effects on cerebral infarction. Similarly, they have confirmed that intravenous transplantation of MSCs introduced with PLGF (placental growth factor) gene show therapeutic effects on cerebral infarction.

The present inventors have also found that MSCs introduced with genes other than the BDNF gene, such as the GDNF (glial cell line-derived neurotrophic factor), CNTF (cliary neurotrophic factor), or NT3 (neurotrophin-3) gene, show therapeutic effects on cerebral infarction. They have verified that mesenchymal stem cells introduced with IL-2 gene have therapeutic effects on a rat brain tumor model. Thus, preferred embodiments of the mesenchymal cells for use in the present invention are mesenchymal cells introduced with the BDNF gene, PLGF gene, GDNF gene, or the IL-2 gene. Specifically, mesenchymal cells with an exogenous BDNF gene, PLGF gene, GDNF gene, or IL-2 gene in an expressible condition are preferably used as mesenchymal cells in the present invention.

Apart from the above genes, mesenchymal stem cells introduced with a gene such as the CNTF or NT3 gene are also preferred as specific examples of the mesenchymal cells in the present invention. Hereinafter, a mesenchymal stem cell (MSC) introduced with the "XX" gene may be referred to as "MSC-XX".

Combining the mesenchymal cells of the present invention with factors (genes) that are responsible for angiogenesis is expected to show significant therapeutic effects on treatments of cerebral infarction, since cerebral infarction shows symptoms of vascular occlusion. The present inventors have found that direct injection of the angiopoietin gene into cerebral infarctions exhibits significant angiogenetic effects, as shown in the Examples below. Specifically, MSCs introduced with a gene involved in angiogenesis, such as the angiopoietin gene, are expected to have therapeutic effects, particularly on cerebral infarctions.

Mesenchymal cells introduced with a desired gene in an expressible manner can be suitably prepared using techniques known to those skilled in the art.

The bone marrow fluids to be used in the present invention can be collected, for example, by anesthetizing (locally or systemically) vertebrate animals (including humans), puncturing a bone with a needle, and then aspirating with a syringe. The bones include, but are not limited to, the femur, sternum, and osilium, which forms the pelvis. Further, a procedure that involves directly puncturing the umbilical cord with a needle, and aspirating with a syringe to collect and store the cord blood at birth, has also become an established technique. Bone marrow cells are collected from subjects under local anesthesia, in an amount of preferably several milliliters per collection. Please note that this amount does not apply to case A below, but does apply to cases B and C.

Procedures for bone marrow collection include, but are not limited to, the following procedures:

(A) Transplanting living bone marrow cells

When bone marrow cells are collected from humans, for example, bone marrow fluid is collected by an anesthetist from patients (the ilium and the like) under general anesthesia, after sufficient consideration of the safety of general anesthesia. A target number of cells for collection is 3x $10^9$ or more mononuclear leukocytes. It is assumed that the target cell number can be obtained from about 200 ml to about 400 ml of bone marrow fluid. The upper limit of the bone marrow amount to be collected is calculated in consideration of patient strain, and is calculated using the hemoglobin level (Hb level) immediately prior to bone marrow collection, and body weight of the patient, in consideration of patient strain. However, in the case of an elderly patient, where collection of the necessary amount of bone marrow fluid is problematic, a maximum amount should be collected based on the decision of the doctor in attendance at the collection of bone marrow fluid.

The upper limit of bone marrow to be collected depending on Hb level immediately prior to collection:

(1) Collect 12 ml or less per kg of patient body weight, when Hb level is less than 12.5 g/dl;

(2) Collect 15 ml or less per kg of patient body weight, when Hb level is less than 13.0 g/dl;

(3) Collect 18 ml or less per kg of patient body weight, when Hb level is less than 13.5 g/dl; or

(4) Collect 20 ml or less per kg of patient body weight, when Hb level is 13.5 g/dl or more.

Bone marrow fluid should not be collected when a patient suffers from cytopenia in the peripheral blood (a white blood cell count less than 2,000 per milliliter; neutrophil count less than 1,000 per milliliter; hemoglobin level less than 11.0 g/dl; platelet count less than 10x $10^4$ per milliliter) or when the patient suffers from hemorrhagic diathesis.

The collection of bone marrow from patients who use anticoagulants or antiplatelet agents should be carefully considered while performing, prior to bone marrow collection and general anesthesia, hemostasis-coagulation tests (FDP, fibrinogen, ATIII) which comprise bleeding time and ACT, and that can be performed as emergency tests.

(1) Patients using anti-platelet agents (such as Panaldine, Bufferin, and Bayaspirin):

Platelet function does not recover until seven days or more after discontinuation, and bone marrow collection in an acute stage may cause hemorrhage. Thus, bone marrow collection should be carefully performed. When bleeding time exceeds ten minutes, bone marrow collection should not be performed.

(2) Patients using anti-coagulants (such as warfarin):

Bone marrow should be collected after ACT has been normalized by intravenous injection of vitamin K (K1 or K2).

Bone marrow cells are intravenously administered by mixing bone marrow cells (3x $10^9$ cells or more) with an equal amount of a diluent (antibiotic-free RPMI 1640) and intravenously injecting the mixture, for example. The entire quantity is expected to be about 400 to 2000 cc. Administration is as rapid as possible, but to inhibit coagulation during the administration period, heparin is generally co-injected.

For example, 250 cc of the collected bone marrow cell fluid is mixed with an equal amount of a diluent and 2500 units of heparin to make up 500 cc, the mixture is immediately filtrated through a filter to yield an intravenously injectable preparation, and intravenous administration of the preparation to a patient is begun immediately. Collection of bone marrow cell fluid is also continued during this time. This operation is repeated two to six times, and a set amount of bone marrow cell fluid is administered. The amount of heparin to be thus administered is about 5,000 to 15,000 units which is substantially the same as the safe and effective amount for which evidence in the acute stage of cerebral infarction is already obtained. However, when considering the continuation of bone marrow collection during administration, ACT is determined, and treatment such as neutralization with protamine is conducted as necessary. The time needed for collection is about two hours. The total amount of bone marrow fluid (including diluent) to be administered intravenously is about 2000 cc. Intravenous administration is completed in about three to four hours while sufficiently monitoring strain to the right heart, indicated by central venous pressure and so on. Note that about 2000 ml of bone marrow fluid (including diluent) is intravenously administered, and about 1000 ml of the bone marrow fluid is collected. Thus the volume of fluid applied to the patient is 1000 ml in three to four hours, which is not so large considering the volume load applied during conventional treatment of cerebral infarction.

Patients are preferably selected according to the following requirements, but are not limited thereto:

1. Patients aged 20 to 70;
2. Patients within 24 hours of onset;
3. Patients for whom diffusion-weighted MRIs show abnormalities in the supratentorial cerebral cortex, perforating region, or both;
4. Patients whose NINDS-III category is any of atherothrombotic cerebral infarction, lacunar infarction, or cardiogenic cerebral embolism;
5. Patients whose Modified Rankin Scale for the present episode is 3 or more;
6. Patents whose impaired consciousness rates 0 to 100 on the Japan Coma Scale.

Patients under the following conditions are preferably excluded.

1. Patients with improving symptoms and diagnosed as substantially asymptomatic or as TIA (transient ischemic attack) patients;
2. Patients diagnosed as having a causative lesion of a disorder other than obliterative cerebrovascular disorders, such as an intracranial hemorrhage, based typically on CT or MRI;
3. Patients with cardiogenic embolus where hemophilic alterations have already been observed by CT;
4. Patients in a coma with a severe consciousness disorder of 200 or more on the Japan Coma Scale;
5. Pregnant patients or patients at risk of pregnancy;
6. Patents with grave renal diseases, liver diseases, or digestive organ diseases;
7. Patents with malignant tumors;
8. Patients in whom grave abnormalities, such as severe ischemic heart disease, are suspected in the cardiovascular system;
9. Patients meeting the subject exclusion criteria for bone marrow fluid collection;

10. Patients for whom general anesthesia is judged as risky;
11. Patients with cerebellar infarction or brainstem infarction;
12. Patients who have undergone endovascular surgical treatment in the acute stage; or
13. Patients judged by the doctor in charge of treatment as unsuitable subjects for this treatment.

(B) Culturing, preserving, and administering mesenchymal stem cells (MSCs) collected from the bone marrow fluid and so on:

Another embodiment of the intravenous administration of the present invention is, for example, the intravenous administration of MSCs collected from the bone marrow fluid and so on, then cultured, and preserved. Preferred conditions for collection, culture, and preservation of the bone marrow cells are as follows:

Specifically:

(1) Collect about 5 ml of bone marrow fluid from the ilium under local anesthesia;
(2) Extract MSCs from the collected bone marrow fluid, cultivate and proliferate, for example, using the method described in WO 02/00849;
(3) Cryopreserve in a preservation medium;
(4) Thaw the frozen MSCs as needed and intravenously administer thawed MSCs intact (2 x $10^8$ cells or more).

In the present invention, bone marrow fluid can be safely and easily collected under local anesthesia from almost all patients, since the amount of the bone marrow to be collected in the initial stages is about 3 ml to about 5 ml, and the strain on the body is small.

Since the collected mesenchymal stem cells, such as bone marrow stem cells, can be proliferated, they can be proliferated in advance to an amount required for treatment. The MSCs to be used in the present invention are preferably primary culture MSCs, and more preferably, primary culture MSCs of 2 x $10^8$ cells or more. The mesenchymal stem cells proliferated by the above methods, or the therapeutic agents of the present invention can be preserved for long periods using a predetermined procedure, such as freezing. Preservation and thawing methods are as follows:

The cells are thawed in the following manner. First, equipment and materials such as a program freezer, a freezing bag F-100, liquid nitrogen, and a tube sealer are prepared. Reagents such as Trypsin/EDTA, DMSO, dextran autoserum, and D-MEM are also prepared.

After removing the culture medium, T/E is added, adherent cultured MSC cells are recovered, an equal amount of a cell washing fluid (D-MEM containing 2% autoserum) is added, and this is then centrifuged at 400 g for five minutes. The cell pellets are stirred with a cell-washing fluid (D-MEM containing 2% autoserum) and centrifuged at 400 g for five minutes. Next, the cells are stirred in 41 ml of a cell-preservation medium (D-MEM containing 50% autoserum). In this procedure, two 0.5 ml portions of the cell suspension are sampled using a 1 ml syringe, and the cells are counted. The stirred fluid is subjected to bacteriological and virological examinations to confirm it is uncontaminated by bacteria or viruses. Next, 10 ml of a cryoprotective fluid (5 ml of DMSO (Cryoserv) and 5 ml of 10% dextran 40) is added. The resulting suspension is packed into freezing bags at 50 ml per bag, and the specimen number is indicated on each bag. The bags are frozen in a program freezer, and the frozen bags are transferred to and stored in a liquid nitrogen tank.

The cells are thawed and washed as follows: First, equipment and materials such as a warm water bath, a clean work station, a centrifugal separator, a separating bag, and a tube sealer are prepared, and reagents such as 20% human serum albumin (or autoserum), physiological saline, and 10% dextran 40 are prepared. A freezing bag comprising the cells is removed from the liquid nitrogen tank and left to stand in the gaseous phase for five minutes, and at room temperature for two minutes. The bag is left to stand in the gaseous phase and at room temperature to prevent its explosion caused by the vaporization of liquid nitrogen. The bag is placed in a sterilized plastic bag to prevent leakage of its contents due to, for example, pinholes in the bag. The plastic bag is placed in a warm water bath and is thawed. After thawing, the entire quantity of the cell suspension is recovered in a blood bag (closure system) or tube (open system). The recovered cell suspension is added to an equal amount of a washing fluid (25 ml of 20% human serum albumin, 75 ml of physiological saline, and 100 ml of 10% dextran 40). The mixture is left to stand for five minutes to reach equilibrium, intracellular DMSO is removed, and the mixture is then centrifuged at 400 g for five minutes. The cell pellets are stirred with cell-washing fluid. The resulting cell suspension is administered *in vivo* to a patient, and again, two 0.5 ml portions of the cell suspension are sampled using a 1 ml syringe and subjected to a viability assay and bacteriological examination.

In the present invention, the primary culture MSCs, which were collected, cultivated, and preserved in advance, can be immediately thawed to an active state as needed, and can be immediately administered intravenously for treatment. Heparin is not used herein. The patient to be administered has no specific limitations.

(C) Administering mesenchymal stem cells immortalized by hTERT:

The present inventors succeeded in developing a method for stably inducing the differentiation and proliferation of large cell numbers (WO 03/038075). Generally, mesodermal stem cells (mesenchymal stem cells) are useful in the medical field of neural regeneration; however, the proliferation of such cells under culture conditions is limited to some extent. However, according to the studies of the present inventors, the *in vitro* introduction into stromal cells or mesenchymal stem cells of a viral vector containing, as an insert, an immortalization gene such as telomerase, was revealed to result in the continuation of cell proliferation, even after cycles of cell division, greatly extending the life span of the cells, and still retaining the same morphology as normal cells. The present inventors found that mesodermal stem cells (mesenchymal stem cells) immortalized by introducing an immortalization gene can be efficiently induced to differentiate into neural stem cells and nervous system cells under appropriate culture conditions.

Specifically, the inventors succeeded in inducing mesodermal stem cells (mesenchymal stem cells), which had been immortalized through the introduction of the immortalization gene hTERT, to differentiate into fat cells, chondroblasts, and osteoblasts, for example. Furthermore, the inventors induced the efficient differentiation of mesodermal stem cells (mesenchymal stem cells) immortalized by the introduction of the hTERT gene, into nervous system cells containing neural stem cells. The present inventors further revealed that demyelinated areas in the spinal cord can be repaired by transplanting these cells themselves (the mesodermal stem cells (mesenchymal stem cells)); neural stem cells differentiated from the mesodermal stem cells (mesenchymal stem cells); nervous system cells differentiated from neural stem cells which had been differentiated from the mesodermal stem cells (mesenchymal stem cells); and nervous system cells differentiated from the mesodermal stem cells (mesenchymal stem cells).

In addition, neural stem cells and nervous system cells whose differentiation was induced according to the above-described methods of the present invention, or by the mesodermal stem cells (mesenchymal stem cells) themselves having an immortalization gene introduced therein, are expected to be very useful in achieving neural regeneration.

When a cell is immortalized by introducing an oncogene or such, the character of the cell is also transformed. In contrast, when a cell is immortalized by introducing an immortalization gene, as in the present invention, the cell retains its original character. In addition, in cases where an immortalization gene has been introduced, the gene can be removed after sufficient proliferation.

Mesenchymal cells introduced with an immortalization gene can also be used for intravenous administration in the present invention as appropriate.

Since technique (C) can yield a large quantity of cells, a larger number of cells can be intravenously administered. Preferably, $1 \times 10^9$ or more cells can be administered. This is a tremendous advantage, since therapeutic effect increases as the number of administered cells increases.

The cranial nerve disease therapeutic agents for in *vivo* administration of the present invention, comprising mesenchymal cells as an active ingredient, can be formulated according to methods known to those skilled in the art. For example, the agents can be used parenterally in the form of an abacterial solution or suspension for injection, combined with water or other pharmaceutically acceptable liquid. The agents can be formulated, for example, by appropriate combination with pharmacologically acceptable carriers or vehicles (specifically, sterile water, physiological saline, vegetable oils, emulsifiers, suspending agents, surfactants, stabilizers, fillers, vehicles, antiseptic agents, and binders), into the form of generally acceptable unit dosages as required in drug manufacturing procedures. The amount of active ingredient in these pharmaceutical preparations is set so as to yield an appropriate volume within an indicated range. An aseptic composition for injection can be formulated using a vehicle such as distilled water for injection, according to regular preparation procedures.

Aqueous solutions for injection include, for example, physiological saline and isotonic solutions containing other adjuvants such as glucose, D-sorbitol, D-mannose, D-mannitol, or sodium chloride. These aqueous solutions may be used in combination with appropriate solubilizers, such as alcohols, more specifically ethanol and polyalcohols, such as propylene glycol and polyethylene glycol; and nonionic surfactants such as Polysorbate 80™ and HCO-50.

Oily liquids include sesame oil and soy bean oil. These can be used in combination with a solubilizer such as benzyl benzoate or benzyl alcohol. Buffers such as phosphate buffer and sodium acetate buffer; soothing agents such as procaine hydrochloride; stabilizers such as benzyl alcohol and phenol; and antioxidants may also be combined. Injections prepared in this way are generally packaged into appropriate ampules.

*In vivo* administration of the agents to patients is preferably parenteral administration. Specifically, it is a single dose intravenous administration, but can be a multiple dose administration. The administration can be conducted over a short period or continuously over a long period. More specifically, the administration includes injection-type and dermal administration-type administrations. Injection-type administration includes intravenous injection, intraarterial injection, selective intraarterial injection, intramuscular injection, intraperitoneal injection, hypodermic injection, intracerebroventricular injection, intracranial injection, and intraspinal injection, and of these intravenous injection is preferred.

Intravenous injection enables transplantation by a regular blood transfusion procedure, does not require surgery or local anesthesia of the patient, and reduces the burden on both patient and doctor. Intravenous injection is preferable in that it also enables bedside transplantation. Considering future advances in emergency medicine, administration may also be possible during ambulance transportation or at the scene of an episode.

Further, due to their high capacity for migration, cells comprised in a cell fraction of the present invention can be used as carriers (vectors) for genes. For example, the cells are expected to be useful as vectors for the gene therapy of various neurological diseases, such as cerebral infarction and brain tumor.

Cranial nerve diseases of the present invention include cerebral infarction, cerebral stroke, encephalorrhagy, subarachnoidal hemorrhage, and brain tumor, of which cerebral infarction is preferred. The cause can be any of atherothrombotic cerebral infarction, cardiogenic cerebral embolism, and lacunar stroke categorized in NINDS-III (Classification of Cerebrovascular Diseases (the third edition) by NINDS (National Institute of Neurological Disorders and Stroke)). The cranial nerve diseases also include neurological diseases associated with head injuries, such as head injuries and cerebral contusion; ischemic cranial nerve injuries; traumatic cranial nerve injuries; cranial nerve degenerative diseases; and metabolic nerve diseases, but are not limited to these, as long as they are diseases caused by abnormalities in the cranial nerve.

*In vivo* administration, such as intravenous administration of the mesenchymal cells of the present invention enables neuroprotection in the brain and cranial nerve regeneration. Accordingly, the present invention provides agents *for in vivo* administration that exhibit neuroprotective effects and comprise mesenchymal cells as active ingredients. The term "neuroprotection" herein refers to the effect of saving cranial neurons that would be damaged or die without treatment.

In addition, the present invention provides agents for *in vivo* administration that exhibit cranial nerve regeneration and that comprise mesenchymal cells as active ingredients. Herein the term "cranial nerve regeneration" means the effect of regenerating cranial nerve cells to recover their function, or therapeutic effects obtained from this effect.

Furthermore, the present invention relates to methods of treating cranial nerve diseases, including the *in vivo* administration (preferably intravenous) of a therapeutically effective amount of an agent of the present invention to a patient.

To reduce the risk of transplant rejection, mesenchymal cells such as bone marrow cells, cord blood cells, or peripheral blood cells in the agents for use in the above mentioned therapeutic methods are preferably cells collected from the patient, or cells derived therefrom (autologous cells derived from the patient) (autotransplantation treatment), unless a special operation such as immunosuppression is conducted. This is preferable there is no need for the concomitant use of immunosuppressive drugs. Allotransplantation is possible if immunosuppression is carried out; however, autotransplantation treatments can be expected to exhibit significantly greater therapeutic effect.

When autotransplantation treatment is difficult, cells derived from another person or from another animal for medical use can be used. These cells may be cryopreserved.

The autologous cells can be any undifferentiated cells collected from a patient, cells prepared by subjecting undifferentiated mesenchymal stem cells collected from a patient to gene manipulation, and cells prepared by inducing differentiation of undifferentiated mesenchymal stem cells collected from a patient.

The agents of the present invention (mesenchymal cells such as bone marrow cells) can be suitably administered to patients by the above-mentioned methods, for example. Doctors can administer the agents of the present invention to patients by appropriately modifying the above-described methods.

The therapeutic methods of the present invention are not limited to humans. In general, the methods of the present invention can also be conducted in the same manner, using mesenchymal cells, on non-human mammals, such as mice, rats, rabbits, pigs, dogs, and monkeys.

All prior art documents cited herein are hereby incorporated by reference into this specification.

Brief Description of the Drawings

FIG. 1 is a photograph showing the therapeutic effect of intravenous administration of MCF cells on a rat cerebral infarction model (transient middle cerebral artery occlusion model). The intravenously administered MCF cells accumulated in the cerebral infarction area.

FIG. 2 is a graph showing the results of investigating the therapeutic effect of locally and intravenously administered MCFs, in which open bars indicate local administration and filled bars indicate intravenous administration.

FIG. 3 shows photographs depicting the therapeutic effect of transplanting autologous MCFs (1x$10^7$ cells) to a rat cerebral infarction model (transient middle cerebral artery occlusion model: 45 minutes) three hours after cerebral infarction (A), six hours after cerebral infarction (B), 12 hours after cerebral infarction (C), 24 hours after cerebral infarction (D), or 72 hours after cerebral infarction (E), or the untreated group (F).

FIG. 4 shows a graph of the results of the above-mentioned FIG. 3, in which open bars indicate data of the untransplanted group, and filled bars indicate data of the transplanted group. *<0.001.

FIG. 5 shows photographs depicting the result of intravenously administering autologous MCFs ($1x10^7$ cells) to a rat cerebral infarction model, in which A shows transplanted MCFG cells (blue) accumulating in a cerebral infarction area. B is a photograph obtained by high power magnification of the region indicated by the open square in A (HE staining). C is a visualized image of the same region as in B (in blue) after treatment with x-gal; many transplanted MCF cells (blue) have accumulated; LacZ-positive cells (D) are found to be NSE-positive (E), and F is a merged view of D and E; LacZ-positive cells (G) are found to be GFAP-positive (H), and I is a merged view of G and H.

FIG. 6 shows photographs depicting the very frequent migration of transplant MCF cells into the brain. The cells were transplanted three hours after cerebral infarction (A), 12 hours after cerebral infarction (B), or 72 hours after cerebral infarction (C). D and G show the region indicated by the open square in A after staining; E and H show the region indicated by the open square in B after staining; and F and I show the region indicated by the open square in C after staining.

FIG. 7 shows graphs indicating the therapeutic effects of transplantation. (A) shows the results of investigating higher brain functions (memory and learning) in a Morris water maze test and (B) is a graph indicating the results of a treadmill stress test. Filled triangles indicate the untreated group, and open squares indicate the treated group. *$p<0.05$, **$p<0.01$.

FIG. 8 shows photographs depicting the MRI test results of rats with cerebral infarction. The upper row shows data immediately after cerebral infarction and the lower row shows data one week after cerebral infarction. Scale bar: 5 mm.

FIG. 9 shows photographs depicting the therapeutic effects of using MSCs. The upper row shows data immediately after cerebral infarction and immediately before treatment, and the lower row shows data after treatment conducted one week after the cerebral infarction. Scale bar: 5 mm.

FIG. 10 shows photographs and a graph indicating the results when MSCs ($1x\ 10^4$ to $1x\ 10^7$ cells) were intravenously administered 12 hours after the cerebral infarction.

FIG. 11 shows photographs and a graph of results that histologically support the results of FIG. 10. Compared to the untreated group (A), a marked therapeutic effect can be seen in the treated group (B: transplantation of $1x\ 10^6$ cells). (C) shows quantified histological results.

FIG. 12 shows photographs indicating the results of intravenous administration of MSCs ($1x\ 10^6$ cells) to a rat cerebral infarction model. The transplanted donor cells accumulated at areas of cerebral infarction (A, B, C, and D). Photographs A and C are photofluorograms, and photographs C and D are photofluorograms merged with regular photographs. The untransplanted group showed no donor cells (E and F). Some of the transplanted donor cells differentiated into neurons (G, I, and K) and glial cells (H, J, and L). LacZ-positive cells (G) were found to be NSE-positive (I). Photograph K shows photograph G merged with photograph I. LacZ-positive cells (H) were found to be GFAP-positive (J). Photograph L shows photograph H merged with photograph J. Scale bars: 250 $\mu$m (A and B), 10 $\mu$m (C to F), and 5 $\mu$m (G to L).

FIG. 13 shows photographs and graphs indicating the therapeutic effects of intravenous MSC administration, as investigated by magnetic resonance spectroscopy (MRS).

FIG. 14 shows graphs indicating the therapeutic effects of MSC transplantation, as investigated by ethological examination.

FIG. 15 shows photographs indicating the results of investigating the therapeutic effects of MSCs on a rat permanent middle cerebral artery occlusion model.

FIG. 16 shows photographs indicating that abnormal signals are also detected in concordance with a cerebral infarction area in MRI examination of severe cerebral infarction (rat permanent middle cerebral artery occlusion model).

FIG. 17 shows photographs indicating that when cerebral infarction is not treated the clarity of the above-mentioned abnormal signal of FIG. 16 in cerebral infarction (HIA in MRI) increases with time (12 hours, three days, and seven days after cerebral infarction).

FIG. 18 shows photographs of MRI images showing the results of intravenous administration of mesenchymal stem cells (MSCs) ($1x\ 10^6$ cells) to a rat permanent middle cerebral artery occlusion model. Results are divided according to time elapsed from the onset of disorder to the administration of MSCs. The images show data without treatment, and for treatment three hours, six hours, 12 hours, 24 hours, and 72 hours after onset, indicated sequentially from the upper row. Each image was obtained by MRI examination ($T_2$WI) one week after the onset of cerebral infarction. The cerebral infarction is white in these images.

FIG. 19 shows a graph indicating the results of intravenously administering mesenchymal cells ($1x\ 10^6$ cells) to severe cerebral infarctions (a rat permanent middle cerebral artery occlusion model), in which the cerebral infarction area is quantitatively determined in terms of the infarct volume.

FIG. 20 shows photographs indicating the therapeutic effect over time of intravenous MSC administration in the hyperacute stage of severe cerebral infarction.

FIG. 21 shows photographs indicating examples of the therapeutic effect over time of intravenous MSC administration in the acute stage of severe cerebral infarction:

FIG. 22 shows a graph of the viability after the onset of disorder upon intravenous administration of mesenchymal stem cells (MSCs) (1x $10^6$ cells) to severe cerebral infarction (rat permanent middle cerebral artery occlusion model). The results are divided according to the time elapsed from the onset of disorder until the administration of mesenchymal stem cells. An "n" denotes the number of samples.

FIG. 23 shows a graph indicating clinical symptoms after MSC transplant therapy for severe cerebral infarction.

FIG. 24 shows photographs of adherent cultured cells, such as mesenchymal stem cells obtained from the peripheral blood in an untreated group or in a group pre-administered with G-CSF or SCF factor by hypodermic injection.

The left view of FIG. 25 is a photograph showing that it was possible to induce the differentiation of adherent cultured cells into neural stem cells (Neurospheres). The right view of FIG. 25 is a photograph showing that it was also possible to confirm nestin expression using RT-PCR.

The upper half of FIG. 26 shows photographs indicating that the cells shown in FIG. 25 could be induced to differentiate into neurons (NF-positive cells) and glial cells (GFAP-positive cells). The lower half of FIG. 26 shows photographs indicating that the expression of both NF and GFAP could also be confirmed by RT-PCR.

FIG. 27 is a graph showing that culturing MSCs results in BDNF production. MSCs transfected with AxCAhBDNF-F/RGD (MSC-BDNF) at MOIs of 100, 300, 1000, and 3000 pu/cell secreted $0.230 \pm 0.110$, $0.434 \pm 0.122$, $0.931 \pm 0.101$, and $1.860 \pm 0.41$ ng/$10^5$-cells of BDNF, respectively, 48 hours later. Untransfected MSCs also produced BDNF ($0.0407 \pm 0.0059$ ng/$10^5$ cell/48-hr).

FIG. 28 shows graphs indicating evaluations of cerebral ischemia-induced neural deficiency.

A: Leg placement impairment was evaluated according to the following scale: 0: severe neural deficiency, 16: no neural deficiency. The four ischemia groups showed no statistical difference in leg-placement score, one day after MCAO and before intracranial administration of MSCs. Eight days after MCAO, MSC-BDNF-treated rats had significantly higher leg-placement scores than control DMEM rats (P=0.0001) and fibroblast-treated rats (P=0.003). Fifteen days after MCAO, the scores of MSC-BDNF-treated rats had similarly increased compared to the scores of the DMEM group (P=0.024).

B: Prior to MCAO the average treadmill speeds were compared between the groups. Eight days after MCAO, rats in the MSC-BDNF group achieved a significantly higher speed than those in the control DMEM (P=0.001) and the fibroblast-treated (P=0.017) group. The speed in MSC-BDNF group remained different (significantly higher than the control DMEM (P=0.002) and the fibroblast group (P=0.023)) until Day 15.

FIG. 29A is a photograph of T2-weighted images (T2W) of rats administered with DMEM, fibroblasts, MSCs, or MSC-BDNF taken two, seven, and 14 days after MCAO. Seven days after MCAO, the MSC-BDNF-treated rats showed a significant reduction in HLV (%) as compared to rats treated with DMEM (P=0.002), fibroblasts (P=0.015), or MSCs (P=0.028). Fourteen days after MCAO, the MSC-BDNF-treated rats showed a significant reduction in HLV (%) compared with the DMEM-treated rats (P=0.011).

FIG. 29B shows photographs of representative T2W images of rats administered with DMEM, MSCs, or MSC-BDNF, taken two and seven days after MCAO. Compared to the other groups on Day 7, the MSC-BDNF group showed a reduction in ischemic injury volume.

FIG. 30 is a graph showing *in vivo* BDNF production levels. The MSC-BDNF-transplanted rats showed a significantly increased BDNF level in the ischemic hemisphere seven days after MCAO compared to rats treated with DMEM (P=0.0002) or MSCs (P=0.0006). Compared to the DMEM-treated rats, the MSC-treated rats also showed a significantly increased BDNF level in the ischemic hemisphere (P=0.0124).

FIG. 31 shows diagrams indicating the presence of cells having a DNA fragment in the ischemic penumbra and at the site of application after MCAO.

A: Photographs showing that compared to the DMEM-treated rats, the MSC-BDNF-treated rats had virtually no TUNEL-positive cells. FITC = green (TUNEL-positive), PI = red (nucleus), magnification x 200.

B: Photographs A magnified by 630 times.

FIG. 31C is a graph showing that animals treated with MSC-BDNF in the ischemic boundary zone showed a significant reduction in TUNEL-positive cells compared to DMEM-administered animals (P=0.013).

FIG. 31D shows photographs indicating that fewer positive cells were detected in the MSC-BDNF-treated rats than in the MSC-treated rats. A large number of DsR-positive MSCs were detected within 2 mm of the administration site. FITC (green, TUNEL-positive), DsR (red, MSC).

FIG. 32 shows micrographs indicating morphological characteristics of exogenous MSCs and endogenous brain cells in the rat brain. Double-immunofluorescence staining revealed that EGFP cells were localized near the administration site. In the brains of recipient rats, EGFP cells (green), neurogenic nucleus antigen (NeuN; A) and glial fibrillary acidic protein (GFAP; B) were found using confocal laser scanning microscopy. Scale bar: 20 μm.

FIGS. 33a to 33e are photographs showing the expression of surface antigens in rat MSCs analyzed by flow cytometry. The MSCs were labeled with monoclonal antibodies specific to the antigen to be presented. Dead cells were removed by front and side scattering. FIGS. 33f to 33i are photographs indicating the differentiation of rat MSCs into typical mesenchymal cells. Osteogenic differentiation of primary MSCs or MSC-IL2s was detected by von Kossa staining. Adipogenic differentiation of primary MSCs (h) or MSC-IL2s (i) was detected by Oil Red O staining.

FIG. 34 shows graphs indicating the antitumor effect and migration capability of MSCs. Filled bars represent NRK cells, and open bars represent MSC cells. (a): 9L cells (5x $10^4$ cell/well) were co-cultured with MSC or NRK cells (5x $10^3$ cell/well). (b): MSC or NRK cells were inoculated at a concentration of 1x $10^5$ cells in a Transwell Insert, and 9L cells (5x $10^3$ cell/well) were placed in wells. The 9L cells were counted four days later. All data are expressed by proliferation inhibitory percentage (%) = [1-(number of 9L cells co-cultured with MSC or NRK cells/number of 9L cells cultivated alone)]x 100. Graph (c) shows the results of a migration assay. $^{125}$I-deoxyuridine-labeled cells (5x $10^4$) isolated using a filter of 8 μm pore size were then placed in the upper chamber of a Transwell, and 9L cells were placed in the lower chamber. After 24-hours of incubation, radioactivity in the lower chamber was determined. The results of the cell migration assay are expressed as ratios of the cell number in the lower chamber to the total cell number in the chamber.

FIG. 35 shows photographs indicating the distribution and migration of MSCs in rats with glioma. 9L-DsR cells (4x $10^4$) were transplanted, and 4x $10^5$ of MSC-EGFP were administered into the tumor or to the contralateral hemisphere three days after the inoculation of the tumor. The rats were euthanized 14 days after tumor inoculation and their brains were excised. (a) and (b) are micrographs of a brain preparation where MSC-EGFP were administered into the tumor. (c) and (d) are micrographs of a brain preparation where MSC-EGFP were administered to the contralateral hemisphere. (a) and (c) are H-E stained, and (b) and (d) are immunohistochemically stained using an anti-GFP monoclonal antibody. (e) to (h) are fluorescent micrographs of the brain where MSC-EGFP were administered into the tumor. (e) shows a boundary zone between glioma and normal parenchyma. (f) shows the inside of the tumor, and (g) shows a terminal microsatellite. (h) is a fluorescent micrograph of a boundary zone between tumor and normal parenchyma where MSC-EGFP were administered to the contralateral hemisphere.

FIG. 36 shows graphs indicating the effects of IL2 genetically modified MSCs on surviving rats to which 9L cells were inoculated. Survivorship was analyzed using a log-rank test based on the Kaplan-Meier method. (a) shows the viabilities of rats, with or without MSC inoculation, after inoculation of 9L cells. (b) shows the viabilities of rats, with or without inoculation of MSC, into the tumor three days after tumor inoculation.

FIG. 37 shows photographs of representative MRIs (Gd-DTPA-enhanced T1-weighted coronal images). 9L glioma were inoculated, or not inoculated, with MSCs three days after tumor inoculation. All animals were subjected to magnetic resonance imaging analysis every seven days. The tumor volume ($mm^3$) was calculated as the sum of image thickness and the area ($mm^2$) of Gd-DTPA-enhanced portions in each imaged region.

FIG. 38 shows photographs indicating the results of histological analysis of glioma administered with genetically modified MSCs. Glioma inoculated with unmodified MSCs (a and b) or with MSC-IL2s (c and d) were histologically analyzed using hematoxylin and eosin staining. The invasion of CD4-positive lymphocytes in glioma after inoculation with unmodified MSCs (e) or MSC-IL2s (f) was detected using a monoclonal antibody W3/25. The invasion of CD8-positive lymphocytes in glioma after inoculation of unmodified MSCs (g) or MSC-IL2s (h) was detected using a monoclonal antibody OX-8.

FIG. 39 shows graphs indicating the results of investigating the production of BDNF, GDNF, CNTF, and NT3 by MSCs introduced with the BDNF, GDNF, CNTF, and NT3 genes. The y-axis indicates cytokine production (ng/$10^5$ cell/48-hr), and the x-axis indicates the multiplicity of infection (pu/cell).

FIG. 40 is a graph showing the results of assessing neurological disorders induced by cerebral ischemia. In addition to the BDNF gene, the GDNF, CNTF, or NT3 gene was introduced into MSCs, the resulting cells were transplanted to a cerebral infarction region, and a limb placement test was conducted. The y-axis indicates the leg-placement score, and the x-axis indicates data before MCAO, one day after MCAO (before injection), eight days after MCAO, and 15 days after MCAO, respectively.

FIG. 41 is a graph showing the infarct volume (HLV) after local transplantation treatment of MSC-BDNF and MSC-GDNF. The y-axis indicates the infarct volume (%), and the x-axis indicates data two days, seven days, and 14 days after MCAO, respectively.

FIG. 42 shows photographs of representative T2-weighted (T2W) images of rats after local administration of DMEM, MSC-BDNF, MSC-GDNF, MSC-CNTF, or MSC-NT3, taken two days and seven days after MCAO.

FIG. 43 shows photographs of MRI images of the group intravenously administered with MSC-BDNF, the group intravenously administered with MSC, and an untreated group (control), taken 24 hours, 72 hours, and seven days after MCAO.

FIG. 44 is a graph showing changes in cerebral infarct volume after MCAO of the group intravenously administered with MSC-BDNF, the group intravenously administered with MSC, and an untreated group (control). The y-axis indicates the infarct volume, and the x-axis indicates data six hours, 24 hours, 72 hours, and seven days after MCAO, respectively.

FIG. 45 is a graph showing treadmill test results of the group intravenously administered with MSC-BDNF, the group intravenously administered with MSC, and an untreated group (control) after MCAO. The y-axis indicates the highest running speed, and the x-axis indicates data 24 hours, 72 hours, and seven days after MCAO, respectively.

FIG. 46 shows photographs of DW2 (b=1000) images and $T_2$WI images in MRI analysis of the cerebral infarctions of an untreated group (control) and a group intravenously administered with MSC-PLGF (administered three hours after MCAO), observed at three hours, 24 hours, three days, and seven days after MCAO, respectively.

FIG. 47 shows graphs indicating the results of quantifying the volume of a region showing abnormal signals developed after MCAO, observed in MRI analysis over time. The upper graph shows the results using DWI images, and the lower graph shows the results using $T_2$WI images.

FIG. 48 shows photographs of the brain tissues of the untreated group (control) and the group intravenously administered with MSC-PLGF, which tissues were stained with TTC seven days after MCAO. The upper photographs show the MSC-PLGF treated group, and the lower photographs show the untreated group (control).

FIG. 49 shows photographs of the blood vascular system of a normal rat visualized by staining with Evans Blue and FITC dextran. The left photograph shows the results using Evans Blue, and the right photograph shows the result using FITC dextran.

FIG. 50 shows photographs of the results of using FITC to visually compare angiogenesis induction in an MCAO-model rat, locally injected with the angiopoietin gene using an adenoviral vector, and in an untreated MCAO-model rat. The left images show results for the MCAO-model rat which was injected with the gene (Angiopoietin), and the right images show results for the MCAO-model rat that was not injected with the gene (control).

FIG. 51 is a graph showing the results of quantifying the ipsilateral/contralateral ratio using FITC. In FIG. 51, "ANG" represents angiopoietin treatment.

FIG. 52 shows photographs indicating the results of using Evans Blue staining to visually compare angiogenesis induction in MCAO-model rats injected, and not injected, with a gene. The left photograph shows results for the MCAO-model rat injected with the gene (Angiopoietin), and the right photograph shows results for the MCAO-model rat not injected with the gene (control).

FIG. 53 is a graph showing the results of a treadmill test on an MSC-administered group, in which MSCs were locally administered in the chronic stage after cerebral infarction, and an untreated group (control). The y-axis indicates the highest running speed, and the x-axis indicates the number of days after MSC administration.

Best Mode for Carrying Out the Invention

The present invention will be illustrated in further detail with reference to several Examples below, which by no means limit the scope of the invention.

[Example 1] Transient middle cerebral artery occlusion model

A rat middle cerebral artery occlusion model was used as a stroke model. Transient middle cerebral artery occlusion (MCAO) was induced for 45 minutes using the intravascular occlusion method (E.Z. Longa, P.R. Weinstein, S. Carlson, R. Cummins, Reversible middle cerebral artery occlusion without craniectomy in rats, Stroke 20 (1989) 84-91).

Adult male Sprague-Dawley rats (n=113) weighing 250 to 300 g were anaesthetized with 5% isoflurane, and the anesthesia was mechanically maintained with 1.5% isoflurane in a gaseous mixture of 70% $N_2O$ and 30% $O_2$ under artificial ventilation. The rectal temperature was maintained at 37°C using an infrared heat lamp. A cannula was inserted into the left femoral artery during surgery, for measuring blood pH, $pO_2$, and $pCO_2$. The tip of a 20.0 to 22.0 mm long 3-0 surgical suture (Dermalon: Sherwood Davis & Geck, UK) was rounded by heating near a flame, and was advanced from the external carotid artery into the lumen of the internal carotid artery, to thereby occlude the origin of the middle cerebral artery (MCA). The tip of the surgical suture was extracted from the internal carotid artery 45 minutes after MCAO, and reperfusion was conducted.

The physiological parameters (rectal temperature, blood pH, $PO_2$, $PCO_2$, and blood pressure) of all mice were maintained within normal ranges during surgery and transplant treatment, and no statistical difference was found between experimental groups.

[Example 2] Preparation of bone marrow cells

Autologous bone marrow was collected from the femur of MCAO rats, one and a half hours prior to bone marrow cell transplant.

The rats were anaesthetized with ketamine (75 mg/kg) and xylazine (10 mg/kg; i.p.). A 1 cm incision was made in the skin, a small hole (2x 3 mm) was punctured in the femur using an air drill, and 1 ml of bone marrow was aspirated using a 22-gauge needle. The collected samples were diluted and suspended in a medium containing 2 ml of L-15 medium and 3 ml of Ficoll (Amersham Biosciences). After centrifugation at 2,000 rpm for 15 minutes, mononuclear cell fractions were collected and resuspended in 2 ml serum-free medium (NPMM: Neural Progenitor Cell Maintenance Medium; Clonetics, San Diego, CA, USA). Following a second centrifugation (2,000 rpm, 15 minutes), cells were suspended in 1 ml of NPMM.

[Example 3] Experimental groups

The experiment was conducted using 11 groups (n=88). Nothing was administered to the Group 1 (control) rats after MCAO (n=8). The rats in Groups 2 to 6 were intravenously administered with just the medium (without donor cell administration), 3, 6, 12, 24, and 72 hours after MCAO (n=8 for each group). The rats in Groups 7 to 11 were intravenously administered with the autologous bone marrow cells (1.0x $10^7$ cells), 3, 6, 12, 24, and 72 hours after MCAO (each group n=8). Six rats in each group were used to calculate the infarct volume, and the others were used for other histological analyses.

[Example 4] Intravenous administration of autologous bone marrow cells to rat cerebral infarction model

LacZ gene was introduced into the bone marrow cells (mononuclera cell fraction: MCF) before transplantation to the rat cerebral infarction model (transient middle cerebral artery occlusion model).

Adex1CAlacZ adenovirus was used to transduce the LacZ gene into the bone marrow cells. The details of the construction procedures are described in another document (I. Nakagawa, M. Murakami, K. Ijima, S. Chikuma, I. Saito, Y. Kanegae, H. Ishikura, T. Yoshiki, H. Okamoto, A. Kitabatake, T. Uede, Persistent and secondary adenovirus-mediated hepatic gene expression using adenoviral vector containing CTLA4IgG, Hum. Gene Ther. 9 (1998) 1739-1745. Y. Nakamura, H. Wakimoto, J. Abe, Y. Kanegae, I. Saito, M. Aoyagi, K. Hirakawa, H. Hamada, Adoptive immunotherapy with murine tumor-specific T lymphocytes engineered to secrete interleukin 2, Cancer Res. 54 (1994) 5757-5760. M. Takiguchi, M. Murakami, I. Nakagawa, I. Saito, A. Hashimoto, T. Uede, CTLA4IgG gene delivery prevents autoantibody production and lupus nephritis in MRL/lpr mice, Life Sci. 66 (2000) 991-1001.). This adenoviral vector has an adenovirus serotype-5 genome that lacks the E1A, E1B, and E3 regions to prevent viral replication. Instead of the E1A and E1B domains, the vector comprises the lacZ gene, which is a β-galactosidase gene of *Escherichia coli.* The lacZ gene is comprised between a CAG promoter, which comprises a cytomegalovirus enhancer and a chicken β-actin promoter (H. Niwa, K. Yamamura, J. Miyazaki, Efficient selection for high-expression transfectants with a novel eukaryotic vector, Gene 108 (1991) 193-199.), and a rabbit β-globin polyadenylation signal. This recombinant adenovirus was propagated in 293 cells and was then isolated. Viral solutions were stored at -80°C until use. The autologous bone marrow cells (1.0x $10^7$ cells), together with 50 MOI of Adex1CalacZ, were placed in DMEM containing 10% fetal bovine serum at 37°C to allow the adenovirus to infect *in vitro*.

The same rats from which bone marrow cells were collected were then subjected to MCAO. Then, a total volume of 1 ml of a liquid (NPMM) containing about 1x $10^7$ mononuclear cells, just prepared from the autologous bone marrow, was administered to the left femoral vein.

Two weeks after transplantation, cells that expressed β-galactosidase were detected *in vivo.*

First, the brains of the deeply anaesthetized rats were removed, fixed by leaving to stand in a phosphate buffer to which 0.5% glutaraldehyde had been added. The brain was cut into slices (100 μm) with a vibratome, and the sections were incubated at 37°C overnight in a X-Gal developer (phosphate buffered saline containing 35 mM $K_3Fe(CN)_6$/35 mM $K_4Fe(CN)_6.3H_2O$/2 mM $MgCl_2$) with X-Gal in a final concentration of 1 mg/ml. Blue reaction products were formed within the cells and thus those cells expressing β-galactosidase were detected.

The cross section of each brain slice was observed under a dissecting microscope, and recorded with an image analyzer. The slices were then fixed by being left to stand overnight in a phosphate buffer to which 4% paraformaldehyde had been added, being dehydrated, and then embedded in paraffin. Slices (5 μm) were cut, and the presence of the blue reaction product (the β-galactosidase reaction product) was evaluated using a light microscope (Zeiss: Axioskop FS). Some sections were counter stained with hematoxylin and eosin.

X-gal develops a blue color in the host brain tissue, and thus donor MCF cells were visualized as blue cells in host brain tissue (FIG. 1). Intravenously administered MCF cells accumulated in the cerebral infarction area.

[Example 5] The effect of local and intravenous administration of MCF on therapeutic effects

The experimental results show that therapeutic effect increases with an increasing number of transplanted cells (FIG. 2). The results also demonstrate that intravenous administration requires about one hundred times as many cells as local administration to attain substantially the same therapeutic effect. Conversely, when one hundred times as many cells are intravenously administered than locally administered, intravenous administration can be expected to exhibit substantially the same therapeutic effect as local administration.

[Example 6] Therapeutic effects of autologous MCF transplant on rat cerebral infarction model

Autologous MCF (1x $10^7$ cells) were transplanted to the rat cerebral infarction model (transient middle cerebral artery occlusion model: 45 minutes).

The extent of infarction lesions was examined using 2,3,5-triphenyltetrazolium chloride (TTC) staining (J.B. Bederson, L.H. Pitts, S.M. Germano, M.C. Nishimura, R.L. Davis, H.M. Bartkowski, Evaluation of 2,3,5-triphenyltetrazolium chloride as a stain for detection and quantification of experimental cerebral infarction in rats, Stroke 17 (1986) 1304-1308.). Normal brain is stained red by this method.

Two weeks after transplantation, the rat was deeply anaesthetized with sodium pentobarbital (50 mg/kg, i.p.). The brain was carefully removed and was sliced into 1 mm coronal sections using a vibratome. Fresh brain sections were immersed for 30 minutes in 37°C physiological saline containing 2% 2,3,5-triphenyltetrazolium chloride (TTC).

As a result, the cerebral infarcted area (including both the cortex and basal ganglia) was slightly stained, and a white image of the cerebral infarction was clearly visualized in the brain of the MCAO model rats (FIG. 3).

The cross-sectional area of infarction in each brain section was examined with a dissecting microscope, and was measured using NIH image, which is image analyzing software. Infarct areas in all brain sections were added, and the total infarct volume of each brain was calculated.

The infarct volumes were statistically analyzed. Data are expressed as "mean$\pm$SD". Differences between the groups were assessed by ANOVA using the Scheffes post hoc test for identifying differences between groups. Differences were deemed statistically significant at $p<0.05$.

Histological analysis of ischemic lesions to which no cells had been administered (the controls) revealed that ischemic lesions were found with reproducibility and consistency, and that their average volume was 258$\pm$55 $mm^3$ (n=6) (FIG. 3F). Of the occlusion indices used for the infarct model, ischemia as determined by TTC was highest in the striatuin (caudate-putamen), globus pallidus, and septal nucleus, and was relatively mild in the cortex.

Using the same infarction parameters, the bone marrow cells were intravenously administered 3, 6, 12, 24 and 72 hours after infarct induction. At all these time points the transplantations reduced the infarct volume, but better results were obtained when transplantation was conducted in the early stages after ischemia induction. When the autologous bone marrow cells were intravenously administered three hours after MCAO, virtually no infarct was detected (FIG. 3A); changes in TTC staining were barely detected, but a slight inflammatory response was detected in the target infarcted lesion. When the cells were administered six hours after MCAO, the intensity of TTC staining was reduced in the infarct at the basal ganglia (40$\pm$28 $mm^3$, n=6) (FIG. 3B). The infarct gradually increased when the cells were administered 12 hours (80$\pm$25 $mm^3$, n=6, FIG. 3C), 24 hours (140$\pm$18 $mm^3$, n=6, FIG. 3D), and 72 hours (180$\pm$22 $mm^3$, n=6, FIG. 1E) after MCAO.

The therapeutic effect was more remarkable when the transplant was conducted earlier. However, it is noticeable that a certain degree of therapeutic effect was obtained even when the treatment was conducted 72 hours after cerebral infarction.

The therapeutic effect is considered to be a synergy of the effects of neuroprotection and neural regeneration. The sooner after cerebral infarction that the transplant is conducted, the greater the neuroprotection exhibited. Further, when treatment is conducted relatively late, the neuroprotective effect is relatively weak, but the neural regeneration effect becomes stronger instead.

The obtained results were quantified and shown in the histogram of FIG. 4 as the infarct volumes in the control (group without cell implants), and in the infarction model animals (groups to which cells were transplanted), which were administered with the cells 3, 6, 12, 24, and 72 hours after MCAO.

[Example 7] Effects of intravenous administration of autologous MCF to rat cerebral infarction model

Autologous MCFs (1x $10^7$ cells) introduced with LacZ were intravenously administered to the rat cerebral infarction model after induction of MCAO. The bone marrow cells were identified *in vivo.*

The phenotype of the transplanted cells *in vivo* was analyzed using a laser scanning confocal microscope (n=5). Rats were deeply anaesthetized with sodium pentobarbital (50 mg/kg, i.p.), and the heart was perfused first with PBS, then with a fixative solution containing 4% paraformaldehyde in 0.14 M Sorensen's phosphate buffer (pH 7.4). The brain was removed, fixed for 24 hours in a 4°C phosphate buffer containing 4% paraformaldehyde, and dehydrated in 0.1M PBS solution containing 30% sucrose. The tissue was placed in O.C.T. compound (Miles Inc.), frozen in liquid nitrogen, and sliced into 10 $\mu$m thick coronal sections using a cryostat. The sections were dried on silane-coated slide glass.

To identify the type of cells derived from the donor bone marrow, a double labeling study was conducted using antibodies against β-galactosidase (polyclonal rabbit anti-β-galactosidase antibody (IgG) labeled with Alexa Fluor 594, CHEMICON), neurons (monoclonal mouse anti-neuron-specific enolase antibody (IgG) labeled with Alexa Fluor 488 [NSE], DAKO) and astrocytes (monoclonal mouse anti-glial fibrillary acidic protein antibody (IgG) labeled with Alexa Fluor 488 [GFAP], SIGMA). The primary antibodies were labeled with Alexa Fluor 488 or Alexa Fluor 594, using a Zenon mouse or rabbit IgG labeling kit (Molecular Probes) according to the manufacturer's instruction. The tissue sections were dried on silane-coated slide glass, then washed with PBS (three times for five minutes), treated for 30 minutes with PBS containing 0.1% Triton-X at room temperature, and incubated for ten minutes with a blocking solution (Protein Block Serum Free, DAKO) at room temperature. The tissue sections were further reacted with two types of primary antibodies at room temperature for 60 minutes, then washed with PBS (three times for five minutes). After immunostaining, the slide glass was covered with a glass cover using a fluorescence mounting medium (DAKO). Alexa Fluor 488 (green) and Alexa Fluor 594 (red) were excited using a 488 nm laser beam derived from an argon laser, and a 543 nm laser beam derived from an He-Ne laser, respectively. Confocal images were obtained using a laser scanning confocal microscope (Zeiss) and software (Zeiss).

The transplanted MCFG cells were treated with X-gal to visualize the donor cells in blue.

The results showed the transplanted donor cells accumulated inside and around the cerebral infarction. FIG. 5A shows a coronal section of the infarcted region comprising accumulated LacZ-positive cells. Examination with a light microscope indicates that many cells are present in and around the ischemic lesion (FIG. 5B), and most of these cells were LacZ-positive donor cells (FIG. 5C). Immunohistochemical analysis showed that some of the LacZ-positive donor cells express NSE, a neuron marker (FIG. 5E) or GFAP, an astrocyte marker (FIG. 5H). FIGS. 5F and 51 each show composite images of the LacZ, NSE, and GFAP images. No clear fluorescence signal was found in the control group. These results indicate that at least some of transplanted bone marrow cells can differentiate into neuronal (FIGS. 5E and 5F) and glial cell lineages (FIGS. 5H and 51).

[Example 8] Migration of transplanted MCF cells into the brain

The transplanted MCF cells migrated into the brain at a high rate (FIG. 6). This migration varies with transplant time after cerebral infarction. For example, in cases where the cells were intravenously administered three hours after MCAO and infarct volume was reduced (FIG. 6A), LacZ-positive cells were observed both in the blood vessel tissue and in the parenchymal brain tissue of the protected lesion, indicating that the transplanted cells migrated to sites that would undergo cerebral infarction and be irreversibly damaged unless treated, and these cells exhibited remarkable neuroprotective effects, saving nervous system cells which would ordinarily have been killed (FIGS. 6D and G). When autologous bone marrow cells were administered 12 hours after MCAO (FIG. 6B), the pathophysiological features were more complex. A relatively large number of blue donor cells were found in areas thought to be severely damaged due to ischemic stress, but a smaller number of donor cells were present in non-damaged regions of the lesions (FIGS. 6E and 6H). In addition to the neuroprotective effect that was observed above, a neural regenerative effect was also found (FIGS. 6A, 6D, and 6G). In contrast, when the autologous bone marrow cells were administered 72 hours after MCAO, ischemic damage was much greater (FIG. 6C), and fewer transplanted cells were found in the lesions (FIGS. 6F and 1). The neuroprotective effect observed above (FIGS. 6A, 6D, and 6G) was relatively small; however, a strong neural regenerative effect was observed. It should be noted, however, that even in this group TTC assays showed that cerebral infarction was suppressed by bone marrow cell transplantation.

[Example 9] Confirmation of therapeutic effects of MCF transplantation by ethological examination

The therapeutic effects of MCF transplantation were verified by two ethological examinations: a Morris water maze test to evaluate learning and memory behaviors, and a treadmill stress test to evaluate motor function.

Higher brain functions (memory, learning) were studied by a modified water maze test (n=10) based on Morris's method (R.G.M. Morris, Spatial localization does not depend upon the presence of local cues, Learn Motiv. 12 (1981) 239-260.). Intravenous administration of autologous bone marrow or sham administration was conducted 12 hours after infarction induction.

The device comprised a white steel tank, 1.3 m in diameter and filled with water until 30 cm deep. The water was opacified with white tempera paint, and was held at 24°C. The walls of the space comprised visual cues, and these remained in the same positions during the experiment. In every training trial, a round ceramic platform 8 cm in diameter was placed 2.5 cm from the water surface in one quadrant of the tank. On Day 1 of training, a single habituation trial was conducted by placing each rat on the hidden platform for 60 seconds. If a rat fell or jumped from the platform, the rat was saved from the water and returned to the platform. Quadrant search and swimming speed were monitored with a video camera mounted to the ceiling and connected to a computer tracked image analysis system.

Treadmill stress tests were also conducted. Intravenous administration of the autologous bone marrow or sham administration was conducted 12 hours after infarction induction.

Rats were trained by making them run at a speed of 20 m/min on a motor-driven treadmill with a slope of 0° for 20 minutes per day, two days a week. The rats were placed on a moving belt that faced away from an electrified grid, and the rats were made to run in a direction opposite to that of the belt's movement. Namely, the rats need to run forwards to avoid a shock (intensity 1.0 mA) to the paw. Only those rats which had learned to avoid the weak electric shock were included in the test (n=10). The maximum speeds of the rats running on the motor-driven treadmill were recorded.
The behavioral scores recorded in the Morris water maze test and the treadmill stress test were statistically analyzed. Data are expressed as "mean±SD". Differences between the groups were evaluated by ANOVA using Scheffe's post hoc test for identifying differences between groups. The difference was deemed statistically significant at $p<0.05$.
The experimental data show that improvements in behavior were observed in both tests (each n=10) (FIGS. 7A and 7B). No dyskinesis was apparent in normal time observation of both the untransplanted group and the transplanted group. However, the treadmill test revealed that the treated rats had higher running speeds on the motor-driven treadmill than the untreated rats (FIG. 7B). This reveals that transplantation markedly improved the motor function deterioration due to cerebral infarction. Severe dyskinesis can potentially affect swimming speed, but the mild hypokinesis of the present invention is not thought to lead to poor performance in the Morris water maze test.

[Example 10] Chronological MRI analysis of therapeutic effects

MRI was used to chronologically examine the therapeutic effects on living animals. This method is used in clinical examinations and treatments, and data obtained by this method can be clinically applied without modification, and are very useful.
Initially, rats were anaesthetized with ketamine (75 mg/kg) and xylazine (10 mg/kg, i.p). Each rat was placed in an animal holder/MRI probe apparatus and positioned inside a magnet. The rat's head was fixed in an imaging coil. A superconducting magnet (Oxford Magnet Technologies) of 7 Tesla, having an internal diameter of 18 cm, interfaced to a Biospec I spectrometer (Bruker Instruments) was used in every MRI determination. T2-weighted images were obtained from coronal sections 0.5 mm thick using visual field 3 cm, TR=3000 ms, TE=30 ms, and reconstructed using a 128 x 128 image matrix.
Rats in which a cerebral infarction had been induced were examined using MRI, and abnormal signals were detected from about three hours after the cerebral infarction. Specifically, a cerebral ischemic region was detected as a High Intensity Area (HIA) in MRI ($T_2$WI) (FIG. 8, upper row). The abnormal signals remained in the untreated group (FIG. 8, lower row) to eventually form a cerebral infarcted area.

[Example 11] Therapeutic effects of using mesenchymal stem cells

The intravenous administration of bone marrow cells (mononuclera cell fraction: MCF) exhibited significant therapeutic effects on cerebral infarction. Mesenchymal stem cells (MSCs), which exist in about 0.1% of MCF, were also used for treatment and their therapeutic effects were confirmed. Mesenchymal stem cells can be easily sampled, cultivated, proliferated, and preserved.
MSCs (1x $10^7$ cells) were administered to the rats with cerebral infarction of FIG. 8. The cells were intravenously administered 12 hours after cerebral infarction, and abnormal signals (HIA), having appeared in MRI tests after cerebral infarction (FIG. 9, upper row), then disappeared from re-tests one week after treatment (FIG. 9, lower row).
Thus, intravenous administration of MSCs was proven to treat cerebral infarction, which is untreatable at current medical levels.

[Example 12] Relationship between number of transplanted MSC cells and therapeutic effects

MSCs (1x $10^4$ to 1x $10^7$ cells) were intravenously administered 12 hours after cerebral infarction.
To clarify the efficacy of MSCs and hTERT-MSCs transplants in reducing ischemic lesion volume, cells in different concentrations (1x $10^4$ to 1x $10^7$ cells) were intravenously administered 12 hours after infarction induction, and cerebral images (T2-weighted images) of all tested animals were obtained 12 hours after MCAO and one week after intravenously administering different concentrations of hTERT-MSCs. Initial infarct volumes were estimated using *in vivo* MRI.
The left row of FIG. 10(A1-E1) shows simple cerebral images obtained from five rats, 12 hours after injury. These coronal forebrain sections were obtained at the caudate-putamen complex level. An ischemic damaged site is seen as a high-intensity region. The contralateral brain tissue shows normal signals, enabling comparison.
The infarct volume ($mm^3$) was evaluated by analyzing the high-intensity regions in a series of images collected from the entire cerebrum. Estimated infarct volumes were constant among the tested animals (214±23 $mm^3$, n=25).
MRI analysis did not find any change in infarct size when the vehicle (medium) alone was administered (FIG. 10A2). The infarct volume decreased with an increasing number of intravenously administered hTERT-MSCs. When

$10^4$ hTERT-MSCs were administered, the infarct volume slightly decreased, showing a slight therapeutic effect (FIG. 10B2) (176±21 $mm^3$, n=5). The reduction in infarct volume escalated and the therapeutic effects became apparent upon administration of $10^5$ cells (138±36 $mm^3$, n=5) and $10^6$ cells (56±18 $mm^3$, n=5) (FIGS. 10C2 and 10D2). The infarct volume was most reduced when $10^7$ cells were administered; a virtually complete therapeutic effect could be expected (FIG. 10E2) (23±31 $mm^3$, n=5). The abnormal signals (HIA) observed before treatment (FIGS. 10A1, B1, C1, D1, and E1) remained unchanged if treatment was not conducted (FIG. 10A2), but disappeared partially or almost completely after treatment (FIGS. 10B2, C2, D2, and E2).

In another test, primary MSCs were also intravenously transplanted. Transplantation of $10^6$ primary MSCs reduced the infarct volume to the same extent as in tests using the same number of hTERT-MSCs (FIG. 10F) (61±18 $mm^3$, n=5, vs. 56±18 $mm^3$, n=5, p=0.69). A supplemental sham control experiment was conducted using $10^6$ dermal fibroblasts (FIG. 10F). Transplantation of $10^6$ dermal fibroblasts did not show a reduction in infarct volume (240±27 $mm^3$, n=5, p=0.95).

The therapeutic effect on cerebral infarction of intravenously administering MSCs correlated with the number of transplanted cells. Namely, the therapeutic effect was higher when more cells were transplanted.

Histological verification was also conducted (FIG. 11).

After evaluation of lesion volume by MRI analysis, and before and after cell administration, the rats were perfused, stained with 2,3,5-triphenyltetrazolium chloride (TTC), and second independent measurements of infarct volume were obtained. Normal brain tissues were generally stained with TTC, but the infarction lesions were not stained or were slightly stained. FIG. 11A shows a TTC stain result obtained one week after MCAO without cell transplantation. Staining on the lesion side was mainly observed in the corpus-striatum. The lesion volume was calculated by measuring the region with reduced TTC staining in the forebrain. As with MRI analysis, infarct size decreased with an increasing number of transplanted cells. Evaluation by TTC staining showed that intravenous administration of $10^7$ hTERT-MSCs markedly decreased the lesion volume (FIG. 11B and C).

[Example 13] Accumulation of transplanted donor cells

MSCs (1x $10^6$ cells) introduced with LacZ or GFP were intravenously administered to the rat cerebral infarction model 12 hours after MCAO.

The cultured cells were rinsed with phosphate buffered saline (PBS) and fixed at 4°C for 15 minutes in a fixative solution containing 4% paraformaldehyde in 0.14M Sorensen's phosphate buffer (pH 7.4). The fixed cells were incubated in a blocking solution containing 0.2% Triton X-100 and 5% normal goat serum for 15 minutes, then incubated with primary antibodies. The primary antibodies used were anti-neuron specific enolase (NSE; 1:1000 polyclonal rabbit anti-NSE, Nitirei) antibody, anti-glial fibrillary acidic protein (GFAP; 1:200 polyclonal rabbit anti-GFAP, Nitirei) antibody, and anti-Nestin (Nestin; 1:5000 murine monoclonal anti-Nestin, Chemicon) antibody. For visualizing the primary antibodies, goat anti-mouse IgG antibody and goat anti-rabbit IgG antibody with fluorescein (FITC) (1:100, Jackson ImmunoResearch Laboratories, Inc.), or an alkaline phosphatase reaction (Zymed) were used according to the manufacturer's instruction. After immunostaining, a glass cover was placed on a microscopic slide glass using a mounting medium (Dako) cell-side down. Photographs were taken using an immunofluorescent microscope (Axioskop FS; Zeiss).

The FITC fluorochrome (green) and rhodamine fluorochrome (red) were excited using a 488 nm laser beam derived from an argon laser and a 543 nm laser beam derived from a He-Ne laser, respectively. Confocal images were obtained using a confocal laser scanning microscope (Zeiss) and software (Zeiss).

As shown in FIGS. 10 and 11, transplant treatment significantly reduced infarct volume, however, hTERT-MSCs expressing GFP were found mainly at the striatum (most infarction was identified in the untransplanted rats).

FIGS. 12A and 12B show merged confocal images of GFP fluorescence and transmitted light image, at low and high power, respectively, where the images are of the striatum on the infarcted side. Note the abundance of GFP-positive cellular-like elements. GFP-expressing cells were mainly concentrated in the corpus-striatum, but a few were found throughout the affected hemisphere. No GFP expression was found in images obtained from the contra lateral striatum, to which infarction had not been introduced. These data indicate that systemically administered cells reached the lesion site.

An immunohistochemical study was conducted to identify immature neurons (NeuN) and astrocytes (GFAP) in the infarcted sites of rats transplanted with LacZ-transferred hTERT-MSCs.

As a result, a small number of NeuN-positive cells and GFAP-positive cells were co-stained with LacZ (FIGS. 12G to L).

The transplanted donor cells accumulated in the cerebral infarction region (FIGS. 12A, B, C, and D). Under a fluorescent microscope, MSC in host brain tissue becomes green. Donor cells were not found in the untransplanted group (FIGS. 12E and F). Some of the donor cells differentiated into neurons (FIGS. 12G, I, and K) and glial cells (FIGS. 12H, J, and L).

[Example 14] Confirmation of therapeutic effects of MSC intravenous administration by metabolic analysis

The therapeutic effects of intravenous administration of MSCs on cerebral infarction were examined in terms of metabolism.

Specifically, NAA and lactate levels in the brain before and after cell transplantation were determined using magnetic resonance spectroscopy (MRS). Correlations have been reported between NAA signals and the presence of normal neurons, and between an increase in lactate and neuronal death (Barker, P.B., Gillard, J.H., van Zijl, P.C., Soher, B.J., Hanley, D.F., Agildere, A.M., Oppenheimer, S.M. and Bryan, R.N., Acute stroke: evaluation with serial proton MR spectroscopic imaging, Radiology, 192(3) (1994) 723-32.). MRS analyses of NAA and lactate levels were conducted in the lesioned and non-lesioned hemispheres 12 hours after MCAO induction.

MRS was conducted at TR=1500 msec, TE=20 msec, average =1024, voxel size 2.5 x 2.5 x 2.5 $mm^3$. The brain was accurately positioned by holding the rat's head in the flat skull position, and locating the center of an imaging section 5 mm posterior to the rhinal fissure.

Consequently, the therapeutic effects of intravenous administration of MSCs on cerebral infarction were also verified in terms of metabolism. Normal hemispheres showed the highest NAA levels and no lactate signal (FIGS. 13A and D). In contrast, the lesioned sides showed low NAA levels and high lactate signals (FIGS. 13A and E). Without cell transplant, NAA signals were low and lactate signals were high one week after infarction induction (FIGS. 13B and F). In contrast, after intravenous administration of $10^7$ hTERT-MSCs, NAA signals were present, and lactate signals were low, indicating that the brain tissue was protected by the transplant treatment (n=5) (FIGS. 13C and G).

[Example 15] Confirmation of therapeutic effects of MSC transplantation by ethological analysis

Two tests were conducted to evaluate the behavioral abilities of infarction-induced rats and transplanted rats: a Morris water maze test and a treadmill stress test. These behavioral tests were started one week after infarction induction, and were conducted alone or together with cell transplantation.

The Morris water maze test was conducted every other day. Control rats learned to mount the platform within several seconds (Morris, R.G.M., Spatial localization does not depend upon the presence of local cues., Learn Motiv, 12 (1981) 239-260.). It took about 140 seconds for MCAO-induced rats to execute the test. Rats without transplants showed stepwise improvement, and had learned to mount the platform in about 40 seconds on Day 26 of the test. The time required for MCAO-induced rats intravenously injected with hTERT-MSCs to get on the platform gradually decreased, and they mounted the platform within several seconds by Day 26 of the test, indicating that transplantation results in remarkable improvement (FIG. 14A).

In the treadmill stress test, the maximum treadmill velocity of control rats (without infarcts) reached about 60 m/min. The maximum velocity in the treadmill test one week after MCAO induction alone, or one week after MCAO induction along with transplantation, was about 35 m/min (FIG. 14B). The untreated rats showed an increase in treadmill velocity from 11 days after infarction induction, and gradually improved over 25 days at the most (46.3+6.1, n=10). The cell-transplanted group showed an even greater improvement in treadmill velocity, with their maximum speed 25 days after injury approaching that of the control group ($62.0 \pm 7.2$, n=10). These results revealed that the transplants remarkably improve the motor function deterioration due to cerebral infarction.

[Example 16] Therapeutic effects of MSC on severe cerebral infarction

The therapeutic effects of the regenerative medical technique using MSCs were studied on rats with severe cerebral infarction (permanent middle cerebral artery occlusion model), to determine from what level of tissue damage the treatment can facilitate recovery. In contrast to the transient middle cerebral artery occlusion model used in Examples 1 to 15, the model used was one in which the middle cerebral artery was permanently occluded. This model was prepared under the same conditions as the model used in Examples 1 to 15, except for occlusion time.

Compared to the previously mentioned rat cerebral infarction model (the transient middle cerebral artery occlusion model: 45 min), those rats with severe cerebral infarction showed a broader cerebral infarct area (FIG. 15, the portion stained white by TTC staining).

Abnormal signals in concordance with cerebral infarction were also detected in the severe cerebral infarction by MRI analysis (FIG. 16). Without treatment, the abnormal signals (HIA in MRI) due to cerebral infarction became more clear with time (12 hours, three days, and seven days after) (FIG. 17).

MSCs (1x $10^6$ cells) were intravenously administered to rats with severe cerebral infarction (rat permanent middle cerebral artery occlusion model). Therapeutic effects were investigated by MRI analyses ($T_2$WI) one week after the cerebral infarction. The cerebral infarction appeared white. Transplants were timed three hours, six hours, 12 hours, 24 hours, and 72 hours after cerebral infarction. Cerebral infarction lesions were clearly observed in the untreated group (uppermost row), but hardly observed in the group intravenously administered with MSC three hours after cerebral infarction (FIG. 18). Specifically, intravenous administration of MSCs also had a remarkable therapeutic effect on severe cerebral infarction. This therapeutic effect was more prominent the earlier that transplant was conducted. It should be noted, however, that some degree of therapeutic effect is observed even when treatment is conducted 24 hours or more after the cerebral infarction.

The therapeutic effect is considered to be a synergy of the effects of neuroprotection and neural regeneration. The sooner after cerebral infarction that transplant is conducted, the stronger the neuroprotective and antihydropic actions. When the transplant is conducted relatively late, the neuroprotection becomes relatively weak, but the neural regeneration becomes strong instead.

FIG. 19 is a graph quantifying results of intravenous administration of MSCs (1x $10^6$ cells) to severe cerebral infarctions (rat permanent middle cerebral artery occlusion model), determined as cerebral infarct volume. The results are divided by the time elapsed between the disorder onset and MSC administration. This graph shows that the untreated group had an infarct volume of about 500 $mm^3$, but the group treated three hours after MCAO had an infarct volume of only 200 $mm^3$, indicating significant effect. When treatment was conducted within 12 hours of MCAO, the infarct volume was clearly reduced compared to that in the untreated group. The earlier the treatment, the greater the reduction in infarct volume, which means a good prognosis.

[Example 17] Effects of intravenous administration of MSC on severe cerebral infarction in hyper acute stage

The chronological therapeutic effects of intravenous MSC administration on severe cerebral infarction in the hyper acute stage were investigated.

When MSCs (1x $10^6$ cells) were intravenously administered to severe cerebral infarction three hours after induction of the cerebral infarction, the abnormal signals (HIAs) that appeared in MRI tests then disappeared several days into the treatment, and this effect continued (FIG. 20). The therapeutic effects of MSCs are exhibited relatively early after administration, and rather than continuing these effects, the predominant therapeutic effects of intravenous MSC administration in the acute stage of cerebral infarction may be neuroprotection and antihydropic action.

[Example 18] Effects of intravenous MSC administration on severe cerebral infarction in the acute stage

The chronological therapeutic effects of intravenous MSC administration on severe cerebral infarction in the acute stage were examined. When MSCs (1x $10^6$ cells) were intravenously administered to severe cerebral infarction six hours after the cerebral infarction, the abnormal signals (HIAs) that appeared in MRI tests immediately before treatment gradually disappeared after treatment (18 hours, one week, two weeks, and four weeks after the transplant treatment) (FIG. 21). These therapeutic effects were not observed in the untreated group (no data).

[Example 19] Viability in severe cerebral infarction upon intravenous MSC administration

MSCs (1x $10^6$ cells) were intravenously administered to severe cerebral infarctions (rat permanent middle cerebral artery occlusion model), and changes in viability after disorder onset were examined and plotted on a graph.

The graph demonstrates that treatment by intravenous MSC administration markedly improved viability in severe cerebral infarction (rat permanent middle cerebral artery occlusion model) (FIG. 22). Without treatment, 90% of these same cerebral infarction model rats died; however, when MSCs (1x $10^6$ cells) were intravenously administered three hours after cerebral infarction, 80% of the rats survived. This revealed that viability increases the earlier that treatment is started. Viability after treatment is also outstanding.

[Example 20] Effects of MSC transplantation treatment on clinical symptoms of severe cerebral infarction

MSC transplant treatment was conducted on severe cerebral infarction, and clinical symptoms were studied.

The MSC transplant treatment significant improved clinical symptoms of severe cerebral infarction (FIG. 23). Treadmill stress tests demonstrated that transplantation markedly improved motor function, once deteriorated by cerebral infarction.

[Example 21] Induction of differentiation of adherent cultured cells derived from peripheral blood into neural stem cells or nervous system cells

Adherent cultured cells such as mesenchymal stem cells were obtained from the peripheral blood. A large number of these cells were revealed to be obtainable by hypodermically injecting a factor such as g-CSF or SCF in advance (FIG. 24).

The obtained adherent cultured cells could be induced to differentiate into neural stem cells (Neurosphere).

The expression of Nestin could be verified through RT-PCR (FIG. 25).

The obtained adherent cultured cells could also be induced to differentiate into neurons (NF-positive cells) and glial cells (GFAP-positive cells). The expressions of NF and GFAP could each be verified by RT-PCR (FIG. 26).

The following Examples 22 to 31 investigate the therapeutic effects of transplanting transgenic stem cells to the brain parenchyma a relatively long time after the onset of cerebral infarction.

[Example 22] Preparation of cells

Human bone marrow (BM) was obtained from the posterior iliac crest of healthy adult volunteers after obtaining their informed consents. This test was approved by the Institutional Review Board of Sapporo Medical University. BM mononuclear leukocytes were plated on 150 $cm^2$ plastic tissue culture flasks and incubated overnight. After washing away the free cells, the adherent cells were cultured at 37°C in Dulbecco's modified essential medium (DMEM) containing 10% heat-inactivated fetal bovine serum (FBS) (GIBCO BRL, Rockville, Maryland) in a humidified atmosphere of 5% $CO_2$.

After reaching confluence, the cells were harvested and used for gene transfection with a retroviral vector, BABE-hygro-hTERT (Kawano, Y*., et al*. (2003). *Ex vivo* expansion of human umbilical cord hematopoietic progenitor cells using a co-culture system with human telomerase catalytic subunit (hTERT)-transfected human stromal cells. Blood 101, 532-540.). MSCs within 40 population doublings (PD) were used in this study.

The morphological features of the MSCs were the same as those previously described by Kobune *et al*. (Kobune, M*., et al*. (2003). Telomerized human multipotent mesenchymal cells can differentiate into hematopoietic and cobblestone area-supporting cells. Exp Hematol 31, 715-722.). Adult normal human dermal fibroblasts (NHDF-Ad) were obtained from TAKARA BIO INC. (Japan) and were cultured in DMEM containing 10% FBS as described above.

[Example 23] Adenoviral vector

An adenoviral vector (AxCAEGFP-F/RGD) carrying a gene for RGD-mutated fiber together with a humanized variant *of Aequoria victoria* green fluorescent protein (enhanced GFP: EGFP) under the control of CA promoter (chicken β-actin promoter with CMV-IE enhancer) was constructed according to known procedures (Nakamura, T., Sato, K. and Hamada, H. (2002). Effective gene transfer to human melanomas via integrin-targeted adenoviral vectors. Hum Gene Ther 13, 613-626., Dehari, H., *et al*. (2003). Enhanced antitumor effect of RGD fiber-modified adenovirus for gene therapy of oral cancer. Cancer Gene Ther 10, 75-85.).

The EGFP gene fragment was isolated from the pEGFP vector (BD Biosciences Clontech, Palo Alto, CA) and inserted into the pCAcc vector (pCAEGFP) (Yamauchi, A., *et al*. (2003). Pre-administration of angiopoietin-1 followed by VEGF induces functional and mature vascular formation in a rabbit ischemic model. J Gene Med in press). The cosmid vector pWEAxCAEGFP-F/RGD so generated, together with ClaI- and EcoT221-digested DNA-TPC from Ad5dlx-F/RGD, were co-transfected into human embryonic kidney 293 cells. Adenoviral EGFP expression vector, AxCAEGFP-F/RGD, obtained from isolated plaques, was expanded in these cells and purified by cesium chloride ultracentrifugation (Kanegae, Y*., et al*. (1995). Efficient gene activation in mammalian cells by using recombinant adenovirus expressing site-specific Cre recombinase. Nucleic Acids Res 23, 3816-3821.).

Another adenoviral vector (AxCADsR-F/RGD) carrying a gene for RGD-mutated fiber together with a humanized Discosoma red fluorescent protein (DsR) under the control of the CA promoter was constructed as described above.

Human BDNF cDNA was cloned by a polymerase chain reaction using total RNA extracted from primary culture MSCs as the template (RT-PCR). The identity of the BDNF cDNA obtained in this manner was verified by sequencing and comparing with the GenBank sequence XM_006027. The human BDNF primer sequence was: forward 5'-CGGAATTCCACCATGACCATCCTTTTCCTTACTATGGTTA-3' (SEQ ID NO: 1); and reverse 5'-CCAGATCTATCTTCCCCTTTTAATGGTCAATGTA- 3' (SEQ ID NO: 2).

A plasmid was obtained by inserting the BDNF cDNA into the pCAcc vector between the EcoRI site and the Bgl II site, and was named pCAhBDNF. The plasmid pCAhBDNF was digested by ClaI and the fragment containing the BDNF cDNA expression unit was isolated by agarose gel electrophoresis. The adenoviral BDNF expression vector pWEAxCAhBDNF-F/RGD was prepared using Lipofectamine 2000 (Invitrogen Corporation, Tokyo, Japan).

Before using the viral vector, the concentration and titer of the virus was evaluated, and the viral stocks were examined for potential contamination with replication competent viruses. To determine viral concentration (particle unit (pu)/ml), the viral solution was incubated in 0.1 % sodium dodecylsulfate, and $A_{260}$ was measured (Nyberg-Hoffman, C., Shabram, P., Li, W., Giroux, D. and Aguilar-Cordova, E. (1997). Sensitivity and reproducibility in adenoviral infectious titer determination. Nat Med 3, 808-811.). The viral titers of AxCAhBDNF-F/RGD, AxCAEGFP-F/RGD, and AxCADsR-F/RGD were 4.35x $10^{11}$, 5.38x $10^{11}$, and 1.03x $10^{12}$ pu/ml, respectively.

[Example 24] Adenovirus infection

Adenovirus-mediated gene transfection was performed as previously described (Tsuda, H., *et al.* (2003). Efficient BMP2 gene transfer and bone formation of mesenchymal stem cells by a fiber-mutant adenoviral vector. Mol Ther 7, 354-365.).

Briefly, the cells were seeded onto 15 cm plates at a density of 2x $10^6$ cells per plate. MSCs were exposed to 7.5 ml of a DMEM suspension containing infectious viral particles at 37°C for 60 minutes. The cells were infected with AxCAhBDNF-F/RGD, AxCAEGFP-F/RGD, and AxCADsR-F/RGD at MOIs of 1x $10^3$, 4x $10^3$, and 4x $10^3$ pu/cell, respectively. The medium was then removed, and the cells washed with DMEM once, and then re-cultured with normal medium for 24 hours, and transplanted into the brain.

[Example 25] *In vitro* detection and quantitative analysis of immunoreactive human BDNF

MSC cells transfected with AxCAhBDNF-F/RGD (MSC-BDNF) at MOIs of 100, 300, 1000, and 3000 pu/cell secreted BDNF at rates of $0.230\pm0.110$, $0.434\pm0.122$, $0.931\pm0.101$, and $1.860\pm0.410$ ng/$10^5$ cell/48-hr, respectively. Untransfected MSCs also produced BDNF protein at a rate of $0.0407\pm0.0059$ ng/$10^5$ cell/48-hr. BDNF production level of MSC-BDNF cells transfected at an MOI of 1000 pu/cell was 23 times more than in uninfected MSCs (FIG. 27).

[Example 26] Transient MCAO animal model and intracerebral transplantation

The use of animals in this study was approved by the Animal Care and Use Committee of Sapporo Medical University, and all procedures were conducted according to institutional guidelines.

Rats were anaesthetized with 3.5% halothane, and were kept unconscious using a face mask and 1.0% to 2.0% halothane in 70% $N_2O$ and 30% $O_2$. After surgery, the animals were placed under a heat lamp to maintain their body temperatures at 37°C. Local cerebral ischemia was induced in male Wistar rats (each 250 to 300 g) by endovascular middle cerebral artery occlusion (Tamura, A., Gotoh, O. and Sano, K. (1986). [Focal cerebral infarction in the rat: I. Operative technique and physiological monitorings for chronic model]. No To Shinkei 38, 747-751.). A 5-0 monofilament nylon suture with a silicone-coated tip was gradually inserted through an arteriotomy in the right common carotid artery through the internal carotid artery to a point about 18 mm distal to the bifurcation of the carotid artery. The nylon suture was extracted 90 minutes into the transitory occlusion, to recover blood flow in the brain.

Donor MSCs were transplanted to the brain according to the method described by Goto et *al.* (Goto, S., Yamada, K., Yoshikawa, M., Okamura, A. and Ushio, Y. (1997). GABA receptor agonist promotes reformation of the striatonigral pathway by transplant derived from fetal striatal primordia in the lesioned striatum. Exp Neurol 147, 503-509.).

After confirming the induction of ischemic brain injury using the behavioral tests described below, the animals were randomized for transplantation. The animals were anaesthetized with intraperitoneal (IP) injection of ketamine (2.7 to 3 mg/100-g) and xylazine (0.36 to 0.4 mg/100-g) and positioned in a Narishige stereotaxic frame (Model SR-6N, Narishige Co., Ltd., Japan). Using a 26-gauge Hamilton syringe, 5 μl of a suspension of 5x $10^5$ MSCs in serum-free DMEM was injected to the right dorsolateral striatum 4 mm beneath the skull surface and 3 m lateral to the bregma level over 2.5 minutes (Paxinos, G., Watson, C., Pennisi, M. and Topple, A. (1985). Bregma, lambda and the interaural midpoint in stereotaxic surgery with rats of different sex, strain and weight. J Neurosci Methods 13, 139-143.). This position was approximately the ischemic boundary zone. To prevent rejection of human MSCs transplants, the transplanted rats were intraperitoneally administered with cyclosporine A (10 mg/kg/day).

[Example 27] Therapeutic effects of MSC-BDNFs (Experiment 1)

Experiment 1 was conducted 14 days after MCAO to test the therapeutic effectiveness of MSC-BDNF. Experimental groups were as follows:

Group 1 (control): Rats in which the ischemic boundary zone was injected with DMEM 24 hours after MCAO (n=7);
Group 2: Rats in which the ischemic boundary zone was transplanted with fibroblasts 24 hours after MCAO (NHDF-Ad) (n=6);
Group 3: Rats in which the ischemic boundary zone was injected with MSCs 24 hours after MCAO (n=7); and
Group 4: Rats in which the ischemic boundary zone was transplanted with MSC-BDNFs 24 hours after MCAO (n=7).

LPT was performed one, eight, and 15 days after MCAO, and a treadmill stress test was performed eight and 15 days after MCAO. MRI was performed on days two, seven, and 14.

(1) Limb Placement Test (LPT) (FIG. 28A)

LPTs included eight subtests, described by Johansson and coworkers (Ohlsson, A. L. and Johansson, B. B. (1995). Environment influences functional outcome of cerebral infarction in rats. Stroke 26, 644-649.), and were conducted 24 hours after ischemia induction.

Briefly, the four limbs of the rats were evaluated using the top and edges of a counter top. For each subtest, animals were scored as follows: 0=unable to place their limbs; 1 =partial and/or delayed (by more than 2 seconds) placement of their limbs; and 2=immediate and correct limb placement.

The neurological scores prior to MCAO were similar for all animals. One day after MCAO, prior to intracerebral MSC injection, there was no statistical difference in limb-placement score between the four ischemic groups. Eight days after MCAO, the MSC-BDNF-administered rats achieved significantly high limb-placement scores (8.43±1.52) compared to the control DMEM rats (3.71±0.49, P=0.0001) and the fibroblast-administered rats (5.00±1.10, P=0.003). Fifteen days after MCAO, the MSC-BDNF-administered rats scored 9.14±2.61, which was significantly higher than the scores seen in the DMEM group (5.00±1.73, P=0.024). In contrast, on both Day 8 and Day 15 the MCS-administered rats did not achieve higher scores than the DMEM- or fibroblast-administered control rats, .

(2) Treadmill test (FIG. 28B)

In the treadmill test, rats were placed on an accelerating treadmill (Model MK-680, Muromachi Kikai Co., Ltd., Japan) (Mokry, J. (1995). Experimental models and behavioural tests used in the study of Parkinson's disease. Physiol Res 44, 143-150.). The rats were made to run on a belt, the speed of which gradually increased by 10 m/s every 10 seconds to a maximum speed of 70 m/s, and were made to maintain the middle position on that belt. When a rat could no longer run, the trial was officially ended. The maximum speed at which each animal could run was measured. The rats were tested eight and 15 days after MCAO.

Average treadmill speeds prior to MCAO were comparable between groups. Eight days after MCAO, the rats of the MSC-BDNF group achieved significantly higher speeds (23.4±2.6 m/s) compared to the control DMEM- (9.57±5.6 m/s; P=0.001) and fibroblast-treated (11.8±6.2 m/s; P=0.017) groups. These differences were maintained even on Day 15. The MSC-BDNF, control DMEM, and control fibroblast groups showed speeds of 36.6±9.5, 12.1±9.4 (P=0.002), and 15.8±11.3 (P=0.023), respectively. MSC-treated rats showed no enhancement in recovery on Day 8 or Day 15.

(3) Reduction in infarct volume after MSC-BDNF treatment, as determined by MRI (FIGS. 29A and 29B)

MRI was conducted on all the animals two, seven, and 14 days after MCAO. The animals were anaesthetized prior to MRI. The MRI device comprised a superconductive magnet of 7 T and 18 cm in diameter, connected to a LTNITYINOVA console (Oxford Instruments, UK, and Varian, Inc., Palo Alto, CA) via an interface. The animals were fixed in the same position during imaging. Multislice T2-weighted spin echo MR images (TR 3000 msec, TE 40 msec, field of view 40x 30 mm, section thickness 2 mm, gapless) were obtained.

The disposition of the ischemic area was evaluated by calculating the percent hemisphere lesion volume (% HLV) from the T2-weighted images using imaging software (Scion Image, Version Beta 4.0.2, Scion Corporation). Ischemic tissue in each section was marked, and the infarct volume was calculated considering the thickness of the section (2 mm/section). To avoid overestimating infarct volume, a corrected infarct volume (CIV) was calculated according to the following equation, as described by Neumann-Haefelin *et al.* (Neumann-Haefelin, T., *et al.* (2000). Serial MRI after transient focal cerebral ischemia in rats: dynamics of tissue injury, blood-brain barrier damage, and edema formation. Stroke 31, 1965-1972; discussion 1972-1963.):

$$CIV = (LT-(RT-RI))x\ d$$

In this equation, LT represents the area of the left hemisphere in $mm^2$; RT represents the area of the right hemisphere in $mm^2$; RI represents the infarcted area in $mm^2$; and d represents the thickness of the section (2 mm). The relative infarct volume (% HLV) is expressed as a percentage of right hemisphere volume.

Hyper intensity areas were summed over the six central MR images in the T2-weighted images, and lesion volume was expressed as percent contralateral hemisphere lesion volume (% HLV). All groups showed a reduction in % HLV from Day 2 to Day 14. Two days after MCAO, no significant difference in % HLV was found among the MSC-BDNF (35.0±4.8%), DMEM (38.7±4.9%), fibroblast (37.9±3.8%), and MSC (37.8±2.8%) groups, but the % HLV of the MSC-BDNF group was somewhat reduced compared to the other groups.

In contrast, seven days after MCAO, the rats of the MSC-BDNF group showed a significant reduction in % HLV (25.4±2.8%) compared to the control DMEM (32.8±4.9%; P=0.002), control fibroblast- (31.6±2.2%; P=0.015), and control MSC-treated (30.8±4.3%; P=0.028) groups. After fourteen days, the rats of the MSC-BDNF group showed a significant reduction in % HLV (23.7±3.2%) compared to the DMEM control (29.6±3.6%; P=0.011). Compared to the control DMEM and fibroblast groups, the MSC-treated rats did not show any significant recovery in % HLV seven days (30.8±4.3%) or 14 days (26.2±2.9%) after MCAO.

[Example 28] *In vivo* BDNF production level (Experiment 2; FIG. 30)

In Experiment 2, growth factors were measured in the following animal groups:

Group 1 (control): Normal rats (n=3),
Group 2 (control): DMEM-injected rats (n=3),
Group 3: MSC-injected rats (n=3), and
Group 4: Rats injected with MSC-BDNF to the ischemic boundary zone 24 hours after MCAO.

The rats were sacrificed seven days after MCAO to measure BDNF concentration in the local brain tissue.
The present inventors determined the BDNF level in the local brain tissue seven days after MCAO using sandwich ELISA.
MSCs were transfected *in vitro* at different MOIs (pu/cell), and culture supernatants were collected 48 hours later for analysis. Seven days after MCAO, the rats were anaesthetized by the intraperitoneal administration of ketamine (4.4 to 8 mg/100 g) and xylazine (1.3 mg/100 g), the brain was removed and sliced while on ice into coronal sections (200 mg) from -1.0 to 1.0 mm bregma of the ischemic hemisphere , and these were stored at -80°C until use. Each tissue sample was suspended in an equal weight of a homogenate buffer (1 ml; 137 mM NaCl, 20 mM Tris, 1 % NP40, 1 mM PMSF, 10 μg/ml aprotinin, 1 μg/ml leupeptin, 0.5 mM sodium vanadate) and homogenized with a Dounce homogenizer. The homogenate was centrifuged (10,000 g) at 4°C for ten minutes, and the supernatant (5 μg/μl) was collected for analysis. The BDNF concentration of each of the samples (analyzed in triplicate) was quantified using a commercially available BDNF ELISA kit (Promega, Madison, Wisconsin).
The MSC-BDNF-transplanted rats showed significantly increased BDNF levels in the ischemic hemisphere (45.2±14.8 pg/mg protein) as compared to the DMEM- (12.5±1.9 pg/mg protein; P=0.0002) or MSC-injected rats (19.3±5.5 pg/mg protein; P=0.0006). The MSC-treated rats also showed significantly increased BDNF levels in the ischemic hemisphere as compared to the DMEM-treated rats (P=0.0124).

[Example 29] Nuclear DNA fragmentation in MSC-BDNF-treated animals (Experiments 3A and B; FIG. 31)

Experiment 3A was conducted to evaluate the intensity of DNA fragmentation in brain cells seven days after ischemia. The experimental groups herein are as described in Experiment 2. The rats were sacrificed seven days after MCAO to evaluate their brain tissue using TUNEL staining.
Seven days after MCAO, the rats were anaesthetized and transcardially perfused, initially, with phosphate-buffered saline (PBS) and then with PBS containing 4% paraformaldehyde (PFA). The brains were excised, immersed for two days in PBS containing 4% PFA, and 30 μm frozen sections (coronal coordinates bregma -1.0 to 1.0 mm) were sliced in a cryostat at -20°C. DNA fragmentation of cells in the ischemic boundary zone was detected with an *In Situ* Apoptosis Detection Kit (Takara Biomedicals, Shiga, Japan) using the terminal deoxynucleotidyl transferase (dUTP) nick-end labeling (TUNEL) technique (Gavrieli, Y., Sherman, Y. and Ben-Sasson, S. A. (1992). Identification of programmed cell death *in situ* via specific labeling of nuclear DNA fragmentation. J Cell Biol 119, 493-501.). Specifically, after protease digestion, the sections were incubated in a mixture of terminal deoxynucleotidyl transferase and fluorescein isothiocyanate-labeled dUTP (green). The sections were then counter-stained with PI (propidium iodide), which stains red. In these sections the MSCs that were transfected with AxCADsR-F/RGD were stained red. The total number of positive red cells was counted in three 1x1 $mm^2$ regions of the inner boundary zone (Hayashi, T., Abe, K. and Itoyama, Y. (1998). Reduction of ischemic damage by application of vascular endothelial growth factor in rat brain after transient ischemia. J Cereb Blood Flow Metab 18, 887-895.).
Sections 100 μm thick were prepared using a vibratome and incubated at 4°C overnight with a primary antibody diluted with PBS containing 3% BSA and 0.1% Triton X-100. The primary antibodies used in this study were anti-neuronal nuclear antigen (NeuN: mAb377; Chemicon International, Temecula, CA, USA) and anti-glial fibrillary acidic protein (GFAP: G3893, Sigma) antibodies. After rinsing in PBS, the sections were incubated at room temperature for one hour with a fluorescent secondary antibody (Alexa Fluor 594 goat anti-mouse IgG (H+L): A-11032, Molecular Probes, Inc.).
Seven days after MCAO the number of TUNEL-positive cells (green) in the ischemic boundary zones of MSC-BDNF-injected animals was significantly less than in the DMEM-injected group (275±73 vs. 55.0±41.0; P=0.013). In contrast, there were significantly more of these cells in the MSC-injected animals than in the DMEM-injected animals (173.0±64.9 vs. 55.0±41.0; P=0.20) (FIGS. 31A, B, and C).

In Experiment 3B, DNA fragmentation on Day 7 was determined in animals transplanted with MSC-DsR- (Group 2; n=3) or MSC-BDNF-DsR- (Group 3; n=3), as well as in control animals (Group 1; n=3).

A large number of DsR-positive MCS cells were detected less than 2 mm from the injection site. The MSC-BENF-treated animals showed a reduced number of TUNEL-positive transplanted MSCs in the injection site, as compared to the MSC group (FIG. 31D). In addition, compared to the MSC group, the MSC-BDNF-treated animals showed a reduced number of TUNEL-positive cells near MSCs in the injection site,.

[Example 30] MSC phenotypes (Experiment 4; FIG. 32)

Experiment 4 was conducted to determine cell morphology on Day 7. Experimental groups included DMEM-injected control rats (Group 1; n=3), MSC-EGFP-transplanted rats (Group 2; n=3), and MSC-BDNF-EGFP-transplanted rats (Group 3; n=3). Rats were sacrificed seven days after MCAO to morphologically evaluate brain tissue.

To determine whether or not MSCs in the ischemic area expressed a neuronal phenotype, morphological examinations were conducted seven days after MCAO. Some transplanted MSCs were immunopositive to the neuron marker NeuN and astrocyte marker GFAP. Some displayed fibrous projections, while others had a round shape. The transplanted MSC-BDNFs showed similar features to those of the MSCs.

[Example 31] Data analyses

The data shown in Examples 22 to 31 are presented as "means ±standard deviation (SD)". The data from the limb placement and treadmill tests were analyzed using one-way ANOVA and then Games Howell's post hoc tests. The HLV data were analyzed using one-way ANOVA and then Tukey's HSD post hoc tests. The ELISA data were compared between individual groups using Student's t-tests. TUNEL-positive cell numbers were compared between individual groups using one-way ANOVA and then Sheffe's post hoc tests. Significance was assumed if the P value was <0.05.

The following Examples 32 to 44 examine the therapeutic effects of transgenic stem cell transplants on brain tumors.

[Example 32] Establishment of cell lines

A 9L rat glioma cell line (syngenetic with Fisher 344 rats) and normal rat kidney (NRK) cells were maintained in Dulbecco's Modified Eagle's Medium (DMEM, Sigma-Aldrich Inc., St Lewis, MO, USA) supplemented with 10% heat-inactivated fetal bovine serum (FBS, Invitrogen Life Technologies Inc., Grand Island, NY, USA), 2 mM L-glutamine, 50 μg streptomycin, and 50 units/ml penicillin. To biologically label the 9L cells, pDsR2-N1 plasmid encoding humanized Discosoma red fluorescent protein (DsRed2) under the control of a CMV promoter was purchased from BD Biosciences Clontech (Palo Alto, CA, USA). Using a DNA complex prepared at a ratio of 1 μg DNA : 2.5 μl of NeuroPORTER reagent (Gene Therapy Systems, Inc., San Diego, CA, USA), pDsR2-N1 was transfected using NeuroPORTER to cells at 50% to 60% confluence. 24 hours after the transfection DsRed2-positive cells were isolated using FACScalibur (Becton Dickinson Co., Franklin Lakes, NJ, USA), and further purified by repeating selection 72 hours after the transfection. The isolated DsRed2-positive 9L cells were selected in DMEM supplemented with 10% FBS and 1 mg/ml G418 (Invitrogen Life Technologies) for 14 days to establish a stable cell line (9L-DsR).

[Example 33] MSC preparation

MSCs were prepared from bone marrow according to previously reported procedures (Tsuda *H et al.* Efficient BMP2 gene transfer and bone formation of mesenchymal stem cells by a fiber-mutant adenoviral vector. Mol Ther 2003; 7: 354-365.).

Briefly, Fischer 344 rats (nine weeks of age, male) were sacrificed by cervical dislocation, the femur and tibias were cut from the soft tissues, and the epiphyses were removed using rongeurs. The mid shaft bone marrow tissues of the femur and tibias were then flushed into normal medium (DMEM supplemented with 10% FBS, 100 unit/ml penicillin, 100 μg/ml streptomycin, 0.25 μg/ml amphotericin-B, and 2 mM L-glutamine). The bone marrow was successively aspirated into syringes using needles of gradually decreasing size (18, 20, and 22 gauge, respectively) to give a single cell suspension. The primary culture MSCs were seeded in normal medium at a density of 5x $10^7$ cells per 10-cm culture dish. Four days after initial culture, the medium was replaced with fresh normal medium to remove non-adherent cells. MSCs were maintained at 37°C and 5% $CO_2$, and consumed medium was exchanged with fresh medium every four days.

[Example 34] Adenoviral vectors and *in vivo* gene transduction

An adenoviral vector with modified fiber encoding human IL-2 (AxCAhIL2-F/RGD) has been already described (Dehari *H et al*. Enhanced antitumor effect of RGD fiber-modified adenovirus for gene therapy of oral cancer. Cancer Gene Ther 2003; 10: 75-85.). Another adenoviral vector (AxCAEGFP-F/RGD) with RGD mutated-fiber and a humanized variant of *Aequoria victoria* green fluorescent protein (enhanced GFP: EGFP) under the control of CA promoter (chicken β-actin promoter with CMV-IE enhancer) was constructed as already described (Dehari H *et al*. Enhanced antitumor effect of RGD fiber-modified adenovirus for gene therapy of oral cancer. Cancer Gene Ther 2003; 10: 75-85., Nakamura T, Sato K, Hamada H. Effective gene transfer to human melanomas via integrin-targeted adenoviral vectors. Hum Gene Ther 2002; 13: 613-626.).

An EGFP gene fragment was isolated from the pEGFP vector (BD BIOSCIENCES CLONTECH, Palo Alto, CA, USA) and inserted into the pCAcc vector (Yamauchi A *et al*. Pre-administration of angiopoietin-1 followed by VEGF induces functional and mature vascular formation in a rabbit ischemic model. J Gene Med 2003; 5: 994-1004.) (pCAEGFP). An expression cassette containing the EGFP gene was isolated by restriction enzyme digestion with ClaI, and inserted into the ClaI site of cosmid vector pL. The thus-generated cosmid vector pLEGFP, together with ClaI- and EcoT22I-digested DNA-TPC derived from Ad5dlx-F/RGD, were co-transfected into human embryonic kidney 293 cells. Plaques produced from the transfected 293 cells were isolated and evaluated using restriction enzyme digestion of the viral genome. AxCAEGFP-F/RGD, which is an adenoviral EGFP expression vector with RGD fiber, obtained from the isolated plaques, was proliferated in the 293 cells. All adenoviral vectors were proliferated in the 293 cells and purified by cesium chloride ultracentrifugation. After purification, the virus was dialyzed against phosphate-buffered saline (PBS) containing 10% glycerol, and stored at -80°C. Viral titer was determined in terms of particle units (pu) by spectrophotometry at $A_{260\ nm}$ (Dehari *H et al*. Enhanced antitumor effect of RGD fiber-modified adenovirus for gene therapy of oral cancer. Cancer Gene Ther 2003; 10: 75-85.).

*Ex vivo* adenoviral gene transduction of primary culture MSCs has been described. (Tsuda *H et al*. Efficient BMP2 gene transfer and bone formation of mesenchymal stem cells by a fiber-mutant adenoviral vector. Mol Ther 2003; 7: 354-365.).

Briefly, one day before adenoviral infection, 5x $10^5$ MSCs were inoculated on a 10-cm culture dish. The cells were infected by incubating at 37°C in 5% $CO_2$ for one hour with 5 ml of a preserved viral solution containing either 1000 pu/cell of AxCAEGFP-F/wt or AxCAhIL2-F/RGD. After infection, the cells were washed twice with PBS (pH 7.4) and supplemented with 10 ml of normal medium.

[Example 35] Characteristics of primary culture rat MSCs

The present inventors analyzed the surface antigens on rat primary culture MSCs using flow cytometry.

The phenotypes of the primary culture MSCs were analyzed using FACScalibur (Becton, Dickinson and Company). In summary, cells were washed twice with PBS containing 0.1% bovine serum albumin (BSA). After labeling with anti-rat CD73 (SH3), CD45, or CD11b/c monoclonal antibody (Pharmingen, San Diego, CA, USA), the cells were labeled with a secondary antibody: goat anti-mouse IgG antibody (Immunotech, Marseille, France) combined with fluorescein isothiocyanate. Mouse $IgG_1$-labeled cells (Immunotech) or mouse $IgG_{2a}$-labeled cells were analyzed as controls with matching isotypes.

The cultured rat MSC cells were CD73 antigen-positive (FIG. 33b). This antigen has been reported as a typical mesenchymal surface antigens on human MSCs (Kobune M *et al*. Telomerized human multipotent mesenchymal cells can differentiate into hematopoietic and cobblestone area-supporting cells. Exp Hematol 2003; 31: 715-722.).

No contamination by hematopoietic cells (CD45 or CD11/b) was detected in the MSC cultures of the present inventors (FIGS. 33c and 33e).

[Example 36] The *in vitro* capacity of MSCs for differentiation into mesenchymal cells

The present inventors similarly investigated the differentiation of rat MSCs into typical mesenchymal lineages.

The *in vitro* capacities for differentiation of the rat primary culture MSCs or genetically modified MSCs into typical mesenchymal lineages were evaluated as previously described (Tsuda H *et al*. Efficient BMP2 gene transfer and bone formation of mesenchymal stem cells by a fiber-mutant adenoviral vector. Mol Ther 2003; 7: 354-365., Kobune M *et al*. Telomerized human multipotent mesenchymal cells can differentiate into hematopoietic and cobblestone area-supporting cells. Exp Hematol 2003; 31: 715-722.).

In summary, MSC cells were treated with an osteogenic differentiation medium supplemented with 80 μg/ml vitamin C phosphate (Wako Pure Chemical Industries, Ltd., Osaka, Japan), 10 mM sodium β-glycerophosphate (CALBIOCHEM, San Diego, CA, USA), and $10^{-7}$ M dexamethasone (Sigma-Aldrich Inc.), or an adipogenic differentiation medium supplemented with 0.5 μM hydrocortisone, 500 μM isobutylmethylxanthine, and 60 μM indomethacin. The

differentiation medium was exchanged every three days until Day 21.

To confirm osteogenic differentiation, cells were fixed with 10% formalin for ten minutes and stained with 5% silver nitrate (Sigma-Aldrich) for 15 minutes to detect deposition of minerals (von Kossa staining).

To detect adipogenic differentiation, the cells were fixed with 10% formalin for 15 minutes and stained with a fresh Oil Red O solution (a 3:2 mixture of 0.5% isopropanol stock solution of Oil Red O and distilled water) to detect lipid droplet formation in cell cultures.

The present inventors' cultured rat MSCs were able to differentiate into osteocyte lineage (FIG. 33f) and adipocyte lineage (FIG. 33h). The capacities of primary culture MSCs for differentiation in to osteogenic and adipogenic lineages were not affected by LI-2 gene modification using the adenoviral vector (FIGS. 33g and 33i).

[Example 37] Effects of MSCs on *in vitro* proliferation of 9L cells

It is unclear whether or not *in vivo* administration of MSCs to brain tumors affects tumor growth. However, MSCs are known to produce cytokines such as fibroblast growth factor (GFG), and other tumor growth factors (TGFs) capable of supporting tumor growth (Tille JC, Pepper MS. Mesenchymal cells potentiate vascular endothelial growth factor-induced angiogenesis *in vitro*. Exp Cell Res 2002; 280: 179-191.). The present inventors initially evaluated the effects of MSC co-culture on the growth of 9L glioma cells *in vitro*.

The present inventors cultured Ds-Red2 (humanized Discosoma red fluorescent protein)-labeled 9L cells (9L-DsR) (5x $10^4$ cell/well) alone or with MSC (5x $10^3$ cell/well) or with normal rat kidney (NRK) cells (5x $10^3$ cell/well) in a 6-well plate for 72 hours. The cells were then trypsinized and counted. The number of 9L-DsR cells was determined using a flow cytometer (FACScalibur).

As is shown in FIG. 34a, the proliferation of 9L cells co-cultured with MSCs (24.5$\pm$1.9% inhibition) was significantly inhibited compared to those co-cultured with NRK cells (17.4$\pm$1.9% inhibition, p<0.01).

To determine the effect of soluble factors released from MSCs on the proliferation of 9L cells, the present inventors used a two-chamber culture system.

MSC or NRK cells were inoculated to DMEM containing 10% FBS in a Transwell Insert (pore size 0.4 $\mu$m, Costar Corporation, Cambridge, MA, USA) at a density of 1x $10^5$ cell/Transwell. 9L cells were inoculated to DMEM containing 10% FBS in a well at a density of 5x $10^3$ cell/well. The co-cultures were incubated for 72 hours and the cells were directly counted to determine proliferation in the co-culture system. All data are expressed as percent inhibition, calculated according to the following equation:

Percent Growth Inhibition=[1-(Cell number of 9L-DsR co-cultured with MSC or NRK cells/Cell number of 9L-DsR cells cultured alone)]x 100.

As shown in FIG. 34b, significant growth suppression of 9L cells was also effected by MSCs but was not affected by the NRK cells cultivated in the different chamber (9.8$\pm$3.1% and 1.8$\pm$1.2%, respectively, P<0.01).

These results show that the MSCs themselves have a direct antitumor effect against 9L glioma cells *in vitro,* which is mediated by a soluble factor.

[Example 38] *In vitro* migration capability of MSCs

The present inventors evaluated the migratory nature of MSCs towards glioma cells *in vitro.*

A cell migration assay was conducted using a culture dish with two chambers: a Transwell (Costar Corporation). Cells were metabolically labeled with $^{125}$I-deoxyuridine ($^{125}$U-IUDR, Amersham Biosciences Corp., Piscataway, NJ, USA).

In summary, 1x $10^5$ cell/ml cells were cultured for 24 hours in a medium containing 0.1 $\mu$Ci/ml $^{125}$I-IUDR. Next, the cells were washed with DMEM three times and re-suspended in the same medium. $^{125}$I-IUDR-labeled cells (5x $10^4$ cells) were placed in an upper chamber 8$\mu$ m in pore size, and the 9L cells were placed in a lower chamber. The Transwell was left stand at 37°C in 5% $CO_2$ for 24 hours, and the cells in the lower chamber were lysed with 1 N NaOH. The radioactivity of the cellular lysate was assessed using a gamma counter. Results of cell migration assay are expressed as percentages (count in the lower chamber as a % of the total cell count).

Neither MSC nor NRK cells spontaneously migrated, but adding 9L cells to the lower chamber stimulated spontaneous migration (FIG. 34c). Migration activity increased dose-dependently with an increasing number of 9L cells. The migration capacity of MSCs was found to be significantly higher than that of NRK cells (p<0.01).

[Example 39] Migration and tumor-tropism of transplanted MSCs

Having established the *in vitro* migration capability of MSCs, the present inventors investigated whether or not transplanted MSCs migrate *in vivo* through a normal brain parenchyma toward intracranial gliomas.

To evaluate the intracranial distribution of MSCs, 4x $10^4$ 9L-DsR glioma cells were intracranially inoculated to the right basal glioma, and three days later 4x $10^5$ EGFP (enhanced green fluorescent protein)-labeled MSC (MSC-EGFP) cells were directly injected into the glioma or into the contralateral hemisphere. Fourteen days after tumor inoculation, the rat brain under deep anesthesia was perfused with PBS and then with 4% paraformaldehyde. The excised brain was fixed with 4% paraformaldehyde overnight and equilibrated with PBS containing 30% sucrose for 48 hours. The fixed brain was embedded in OTC compound (Miles, Inc., Elkhart, IN, USA), snap frozen in liquid nitrogen, and stored at -70°C. The tissue was cryo-sectioned to 20 μm thick and stained with hematoxylin and eosin (H-E), or immunohistochemically stained with an anti-GFP monoclonal antibody (BD Sciences Clontech). The sections stained with the first antibody were visualized using VECTASTATIN Kit (Vector Laboratories, Burlingame, CA, USA). Imaging was conducted with a Zeiss-Pascal microscope (Carl Zeiss, Inc., Thornwood, NY, USA).

As is shown in FIGS. 35a and 35c, a large glioma mass intensively stained with hematoxylin was found in all rats inoculated with the 9L cells. The glioma mass occupied the right hemisphere and caused the midline to be shifted toward the left hemisphere. Most of the gene-labeled MSCs were observed in the boundary zone between the tumor and the normal parenchyma, but after intratumoral injection some of them relatively evenly infiltrated into the tumor bed (FIG. 35b). MSCs did not migrate into the distal brain parenchyma or to the contralateral hemisphere.

Confocal laser microscopy revealed accumulation of EGFP-positive MSCs. Most of them maintained their spindle-like shape at the edge of the DsRed-positive tumor (FIG. 35e). The MSCs existed in concordance with glioma cells, which spread from the main tumor (FIG. 35g). In contrast, MSCs inoculated into the contralateral hemisphere migrated away from the initial injection site along the corpus callosum towards the glioma cells (FIG. 35d). Most of these MSCs remained in the corpus callosum and at the edge of the adjacent tumor (FIG. 35h). MSCs also infiltrated the tumor. Having confirmed the excellent migration capacity and glioma-tropism of MSCs after intracranial transplantation, therapeutic genetically modified cells for treating experimental glioma were therefore prepared in subsequent steps.

[Example 40] Human IL-2 production by genetically modified MSCs

The human IL-2 (hIL-2) was selected as a therapeutic gene since the antitumor effects of IL-2 on 9L glioma cells has been sufficiently established in rat models (Rhines LD *et al*. Local immunotherapy with interleukin-2 delivered from biodegradable polymer microspheres combined with interstitial chemotherapy: a novel treatment for experimental malignant glioma. Neurosurgery 2003; 52: 872-879; discussion 879-880., Iwadate Y *et al*. Induction of immunity in peripheral tissues combined with intracerebral transplantation of interleukin-2-producing cells eliminates established brain tumors. Cancer Res 2001; 61: 8769-8774.).

Human IL-2-transfected MSCs (MSC-IL2s) were prepared by infection with a modified adenoviral vector, as previously described (Tsuda *H et al*. Efficient BMP2 gene transfer and bone formation of mesenchymal stem cells by a fiber-mutant adenoviral vector. Mol Ther 2003; 7: 354-365.). The rat primary culture MSCs have low expression levels of adenoviral receptor and Coxsackie-adenoviral receptor (CAR), and are relatively resistant to wild type adenoviral infection. A fiber-mutant vector was therefore used.

To measure human interleukin-2 (IL-2) production by MSCs transfected with the human IL-2 gene, MSCs were inoculated to a 24-well plate in triplicate at a density of $10^4$ cell/well, twelve hours prior to adenoviral infection. Next, the cells were infected with AxCAhIL2-F/RGD and incubated for 72 hours. The concentration of human IL-2 in the culture supernatant was measured using ELISA (IL-2 Immunoassay Kit; R&D Systems, Inc., Minneapolis, MN, USA).

A high level of hIL-2 was detected in the supernatant of MSCs infected with a relatively low concentration of AxCAhIL2-F/RGD ($8.6 \pm 0.5$ and $24.0 \pm 1.7$ ng/ml/$10^4$ cell/72 h at 300 and 1000 particle units/cell, respectively). This agrees with the present inventor's previous findings. This high-level IL-2 production further increased dose-dependently with increasing adenoviral concentration.

[Example 41] Prolonged survival of glioma-bearing rats transplanted with IL-2 gene-transfected MSCs

The present inventors investigated whether or not MSC-IL2s provide *in vivo* therapeutic benefits.

Male Fisher 344 rats (seven to eight weeks of age, 200 to 240 g) were purchased from Japan SLC, Inc. (Hamamatsu, Japan). The animals were anaesthetized and placed in a stereotaxic apparatus (Narishige Scientific Instrument Lab., Tokyo, Japan). A burr hole was made at an appropriate location (1 mm posterior to bregma and 3 mm right to midline). A 26-gauge needle was inserted at a position 4 mm ventral from the dura, and 5 μl of a PBS suspension of 9L tumor cells was inoculated thereto using a 10-μl microsyringe (Hamilton Company, Reno, NV, USA). Then, 4x $10^4$ 9L cells were mixed with 5 μl of a PBS suspension of 4x $10^5$ MSCs or IL-2-transfected MSCs (MSC-IL2s) (infected with 1000 pu/cell AxCAhIL2-F/RGD). The resulting cell suspensions were intracranially injected as described above (FIG. 35a). Injection of the 9L cells either with unmodified MSCs or EGFP-modified MSCs (MSC-EGFPs) was also evaluated in the same manner.

Rats injected with both the 9L glioma cells and MSC-IL2s showed a significantly prolonged survival (26.3±2.2 days, P=0.0003 vs. 9L alone, P=0.0008 vs. MSC, P=0.0007 vs. MSC-EGFP) compared to the control rats, which were injected with 9L alone or with 9L cells together with unmodified MSCs or MSC-EGFPs (17.1±1.1, 22.0±0.8, 21.3±1.5 days). The rat groups injected with 9L cells together with unmodified MSCs (22.0±0.8 days, P=0.0003) or MSC-EGFPs (21.3±1.5 days, P=0.0003) survived for significantly longer than the controls, but no significant difference was found between survival of the MSC group and that of the MSC-EGFP group (P=0.5881).

IL-2 gene modification of the MSCs conferred additional therapeutic advantages to the survival of rats injected with both MSCs and 9L glioma cells, but genetic modification itself did not affect their survival.

The therapeutic benefits of MSC-IL2s were also confirmed in a therapeutic model of glioma-bearing rats. The rats were transplanted with 4x $10^4$ 9L glioma cells. On Day 3 after tumor inoculation, 5 μl of a PBS suspension containing 4x $10^5$ MSCs or MSC-IL2s was transplanted into the tumor (FIG. 36b).

The intratumoral inoculation of MSC-IL2s significantly prolonged survival of 9L glioma-bearing rats (27.7±1.1 days, P=0.0002 vs. 9L alone) as compared to the control (17.1±1.1 days). The average survival time of the MSC-EGFP-injected glioma-bearing rats (23.2±0.8 days) was significantly less than that of the MSC-IL2-injected rats (P=0.0024), but significantly more than the untreated control (P=0.0006).

[Example 42] Effects of genetically modified MSCs on *in vivo* tumor growth evaluated by MRI

The present inventors evaluated whether or not the prolonged survival observed after injecting MSC-IL2s or MSCs was related to tumor growth inhibition. The present inventors conducted magnetic resonance imaging (MRI) on all animals every seven days to estimate intracerebral tumor volume.

The animals were anaesthetized by an intraperitoneal injection of ketamine (2.7 to 3 mg/100-g) and xylazine (0.36 to 0.4 mg/100-g). Next, 0.2 ml of Gd-DTPA (0.8 to 1.0 mg/kg, Magnevist, Schering Japan, Tokyo, Japan) was injected to the animals, and coronal T1-weighted spin echo images (TR 500 msec, TE 10 msec, field of view 50x 50 mm, slice thickness 1.5 mm, gapless) were obtained using a superconductive magnet of 7 T and 18 cm in diameter, connected via an interface to UNITYINOVA console (Oxford Instruments KK, Tokyo, Japan). The tumor volume ($mm^3$) was calculated as a sum of the Gd-DTPA enhanced portion of each MRI imaged area, times the image thickness. The estimated tumor volume based on MRI has a linear correlation with the actual tumor weight obtained immediately after the imaging test (Namba H *et al*. Evaluation of the bystander effect in experimental brain tumors bearing herpes simplex virus-thymidine kinase gene by serial magnetic resonance imaging. Hum Gene Ther 1996; 7: 1847-1852.).

In T1-weighted imaging, 9L gliomas were clearly recognized as enhanced regions in coronal cross sections (FIG. 37). As shown in Table 1 and FIG. 37, 9L gliomas showed progressive growth in the brains of untreated rats, and reached a fatal volume on Day 14 after tumor inoculation. Table 1 shows the 9L glioma volumes ($mm^3$) measured by MRI.

Table 1

| | Day 7[b] | Day 14 | Day 21 | Day 28 |
|---|---|---|---|---|
| 9L alone (n=4) | 17.6±5.8 | 150.0±10.6 | ND[c] | ND |
| MSCs(n=4) | 5.4±1.1* | 17.6±5.6* | 151.3±4.8 | ND |
| MSC-IL2s (n=4) | 3.3±0.9* | 5.3±0.9* | 16.1±0.9** | 143.7±7.7 |

In Table 1, the symbol "b" represents the number of days after tumor inoculation; the symbol "c" indicates that the test was not done; the symbol "*" represents p<0.01 vs. 9L alone; and the symbol "**" represents p<0.01 vs. MSCs.

In contrast, the brain tumor volume of MSC-IL2- or MSC-treated animals was significantly smaller (P<0.01 compared to the untreated control on Day 14 after tumor inoculation). On Day 14, the unmodified MSC-treated group and the MSC-IL2-treated group showed no significant difference in tumor volume. However, the IL-2 gene modification showed a clear therapeutic effect 21 days after tumor inoculation according to MRI. At this time, the gliomas in unmodified MSC-treated animals had virtually reached fatal volume, but the tumors remained small when the animals were treated with MSC-IL2s. These findings on changes in tumor volume agree with the survival duration in different treatment groups.

[Example 43] Induction of lymphocyte invasion into gliomas by MSC-IL2 transplant

The present inventors investigated whether transplantation of MSC-IL2s to 9L gliomas induces *in vivo* immunoreaction.

To detect the infiltration of CD4 or CD8-positive cells into gliomas after MSC-IL2 treatment, 4x $10^4$ 9L-DsR cells were transplanted, and 4x $10^5$ cells of MSC-EGFPs or MSC-IL2s were injected into the tumor three days after tumor inoculation. The rats were sacrificed seven days after tumor inoculation, and the excised brains were embedded in paraffin. Brain preparations 6 μm thick were immunohistochemically stained with an anti-rat CD4 (Clone W3/25, Serotec Inc., Oxford, UK) or an anti-rat CD8 (Clone OX-8, Serotec Inc.) monoclonal antibody, and visualized using a Vectastain ABC Kit (Vector Laboratories Ltd.).

Histological analyses of MSC-treated 9L glioma using HE staining revealed that the IL2 gene-modified MSC-treated 9L gliomas showed a large amount of mononuclear leukocyte infiltration (FIGS. 38c and d). In contrast, unmodified MSC-transplanted gliomas showed minimal inflammatory cell infiltration (FIGS. 38a and b). Samples of the unmodified MSC-transplanted tumors showed virtually no infiltration of CD4 and CD8 cells (FIGS. 38e and g). In clear contrast to this, tumors inoculated with IL-2 genetically modified MSCs showed infiltration of CD4- and CD8-positive lymphocytes (FIGS. 38f and h).

[Example 44] Statistical analyses

Statistical analysis of the cell proliferation assays and migration assays in Examples 32 to 43 were performed using Student's t-tests. Scheffe's tests were conducted for tumor volume assessments on Day 7 and Day 14, and Student's t-tests were performed on Day 21. P values less than 0.05 in the Student's t-tests and Scheffe's tests were considered significant. Statistical analyses of survival were conducted using log-rank tests.

[Example 45] MSCs introduced with cytokine genes other than BDNF (1) Cytokine productivity

Genes other than the BDNF (brain-derived neurotrophic factor) gene, such as GDNF (glial cell line-derived neurotrophic factor), CNTF (cliary neurotrophic factor), or NT3 (neurotrophin-3) gene, were introduced into MSCs, and production of BDNF, GDNF, CNTF and NT3 by cultivated cells was examined.

The results are shown in FIG. 39. MSCs transfected with AxCAhBDNF-F/RGD (MSC-BDNFs) at MOIs of 100, 300, 1000, or 3000 pu/cell secreted BDNF at rates of $0.230\pm0.110$, $0.434\pm0.122$, $0.931\pm0.101$, and $1.860\pm0.41$ ng/$10^5$ cell/48-hr, respectively. The untransfected MSCs also produced BDNF ($0.0407\pm0.0059$ ng/$10^5$ cell/48-hr).

For GDNF, those MSCs transfected with AxCAhGDNF-F/RGD (MSC-GDNFs) at MOIs of 300, 1000, or 3000 pu/cell secreted GDNF at rates of $1.05\pm0.20$, $2.26\pm0.41$, and $4.15\pm0.54$ ng/$10^5$ cell/48-hr, respectively. Untransfected MSCs also produced GDNF protein ($0.044\pm0.034$ ng/$10^5$ cell/48-hr).

For CNTF, MSCs transfected with AxCAhCNT-F/RGD (MSC-CNTFs) at MOIs of 300, 1000, or 300 pu/cell secreted CNTF at rates of $0.136\pm0.028$, $0.854\pm0.145$, and $3.58\pm0.43$ ng/$10^5$ cell/48-hr, respectively. Untransfected MSCs also produced CNTF protein ($0.0520\pm0.0150$ ng/$10^5$ cell/48-hr).

For NT3, MSCs transfected with AxCAhNT3-F/RGD (MSC-NT3s) at MOIs of 300, 1000, or 3000 pu/cell secreted NT3 at rates of $2.67\pm0.09$, $4.24\pm0.16$, and $6.88\pm0.07$ ng/$10^5$ cell/48-hr, respectively. Untransfected MSCs also produced NT3 protein ($0.12\pm0.001$ ng/$10^5$ cell/48-hr).

(2) Evaluation of neurological disorders induced by cerebral ischemia

MSC cells introduced with the GDNF, CNTF, or NT3 gene were transplanted to cerebral infarction regions as in the above Examples, and limb placement tests were conducted. The limb placement tests were performed according to the procedure in Example 27 (1). Limb placement disorder was evaluated according to the following parameters: 0: severe neurological disorder, 16: no neurological disorder. Limb placement tests were conducted one eight, and 15 days after MCAO.

The results are shown in FIG. 40. The four ischemic groups showed no statistical difference in limb placement score one day after MCAO (which was prior to intracranial injection of MSCs). Eight days after MCAO, the limb placement scores of rats administered with MSC-BDNF and MSC-GDNF were significantly greater than those of the DMEM rats (each $P<0.05$). Fifteen days after MCAO, the rats administered with MSC-BDNF and MSC-GDNF also showed significantly higher scores than those in the DMEM group (each $P<0.05$).

In contrast, on both Day 8 and Day 15 the rats administered with MSC-CNTF and MSC-NT3 did not score higher than the DMEM-administered control rats.

(3) Reduction in infarct volume after MSC-BDNF and MSC-GDNF treatment as determined by MRI

MRI was conducted on all animals two, seven, and 14 days after MCAO. The procedures and evaluations are as in Example 27 (3).

Compared to the control DMEM group rats, MSC-BDNF group and MSC-GDNF group rats showed significant reductions in HLV seven days after MCAO (each $P<0.05$). Likewise, 14 days after, the MSC-BDNF group and MSC-GDNF group rats showed significant reductions in HLV compared to the control DMEM group rats. Both seven and 14 days after, rats administered with MSC-CNTF or MSC-NT3 showed no significant recovery in HLV, as compared to the control DMEM group and the MSC-EGFP group (each P<0.05). The results are shown in FIG. 41.

FIG. 42 shows representative T2-weighted (T2W) images of rats administered with DMEM, MSC-BDNF, MSC-GDNF, MSC-CNTF, or MSC-NT3, where the images were obtained two and seven days after MCAO. The MSC-BDNF group and MSC-GDNF group showed a reduction in ischemic injury volume on Day 7, as compared to other groups.

[Example 46] Intravenous administration of MSC-BDNF cells

MSC cells ($10^7$ cells) introduced with the BDNF gene were prepared according to the above Examples. In the rats with severe cerebral infarction (permanent middle cerebral artery occlusion model), described in Example 16, cerebral infarction was produced and the above cells were administered into the left cava twelve hours later.

MRI was used to chronologically examine the therapeutic effects on living animals. The cerebral infarction lesions of the untreated group (control), MSC-administered group, and MSC-BDNF-administered group were observed 24 hours, 72 hours, and seven days after MCAO (FIG. 43).

In addition, the cerebral infarct volumes of the untreated group (control), MSC-administered group, and MSC-BDNF-administered group were calculated and examined six hours, 24 hours, 72 hours, and seven days after MCAO (FIG. 44).

A treadmill test was conducted on the untreated group (control), MSC-administered group, and MSC-BDNF-administered group 24 hours, 72 hours, and seven days after MCAO to examine motion recovery (FIG. 45).

All data demonstrate that MSCs introduced with the BDNF gene show higher therapeutic effects than MSCs alone.

[Example 47] Intravenous administration of MSC-PLGF cells

Instead of the BDNF gene, PLGF (placental growth factor) was introduced into MSCs. Three hours after producing a cerebral infarction in rats with severe cerebral infarction (permanent middle cerebral artery occlusion model), described in Example 16, MSC-PLGF cells ($10^7$ cells) were administered into the left cava of the rats.

The cerebral infarction lesions in the untreated group (control) and in the MSC-PLGF-administered group were observed using MRI three hours, 24 hours, three days, and seven days after MCAO (FIG. 46). The results were compared in DW2 (b=1000) images and $T_2$WI images.

The volumes of areas showing abnormal signals which arose after MCAO were sequentially quantified using MRI analysis. The results show that reduction began 24 hours after MCAO in the DWI images, and three days after MCAO in the $T_2$WI images (FIG. 47).

To compare cerebral infarct volumes, the brain tissues of the untreated group (control) and MSC-PLGF-administered group were stained with TTC seven days after MCAO (FIG. 48).

All data demonstrate that MSCs introduced with the PLGF gene show higher therapeutic effects.

[Example 48] Angiogenetic effects of injecting angiopoietin gene into a cerebral infarction model

The angiopoietin gene was directly injected into the cerebral infarction lesions of a rat cerebral infarction model (transient middle cerebral artery occlusion model: 45 miutes).

An adenovirus was used as a vector for introducing the angiopoietin gene.

Capillary vessels were visualized using FITC dextran or Evans Blue to evaluate angiogenesis.

Images of the blood vascular system of a normal rat visualized with Evans Blue and FITC dextran are shown in FIG. 49.

FITC was used to visually compare angiogenesis induction in MCAO-model rats with or without gene injection (FIG. 50). The ipsilateral / contralateral ratio was also quantified (FIG. 51).

Evans Blue was also used to visually compare angiogenesis induction in MCAO-model rats with or without gene injection (FIG. 52).

As a result, remarkable angiogenesis was observed. Cerebral infarction is a disease in which blood vessels are occluded. Thus incorporating angiogenesis is expected to exhibit remarkable therapeutic effects.

[Example 49] Local administration of MSCs in chronic stages after cerebral infarction

MSCs were locally administered to rats with severe cerebral infarction (permanent middle cerebral artery occlusion model), described in Example 16, in chronic stages after cerebral infarction, and the therapeutic effect of this was studied. Specifically, two weeks after MCAO, 1x $10^4$ MSCs were transplanted into the cerebral infarction region. Treadmill tests were conducted one day, 14 days, 28 days, and 42 days after MSC administration, and motor function recovery was compared with an MSC-untreated group (the control). As a result, improvements in motor function could be seen. The results are shown in FIG. 53.

Therapeutic effects were fairly low compared to transplantation in acute stages; however, some therapeutic effects were still observed. It is preferable to conduct treatment in an acute stage, since treatment in an acute stage shows greater therapeutic effect. However, in actual clinics the requirements from patients already with cerebral infarction are large, and thus the treatment is also thought to be effective for patients in chronic stages.

Accordingly, the agents of the present invention are preferably used for patients with cranial nerve diseases in an acute stage, but they are not restricted to acute stages, and are also effective for patients in chronic stages, for example.

## Industrial Applicability

The present inventors found that regenerative medicines with the excellent features outlined below can be performed using mesenchymal cells (mesenchymal stem cells), specifically bone marrow cells, cord blood cells, or peripheral blood cells. Specifically, regenerative treatments using bone marrow cells, cord blood cells, or peripheral blood cells, using simple *in vivo* administration (e.g., intravenous administration) of patient-derived bone marrow cells by injection or drip infusion, enable the regeneration of sites damaged by nervous system injuries, and therapies for disorders. Such nervous system injuries cannot in fact be treated by conventional techniques. The efficacy of the present treatments on cerebral infarction has been rigorously verified. Additionally, these treatments are considered effective for all neurological diseases, such as injuries of the nervous system due to cerebral infarction, intracerebral bleeding, spinal cord injury, myocardial infarction, cerebral stroke including subarachnoidal hemorrhage, central and peripheral demyelination diseases, central and peripheral degenerative diseases, brain tumor, higher-function disorders including dementia, mental disorders, epilepsia, traumatic neurological diseases including head injuries, cerebral contusion, spinal cord injuries, inflammatory diseases, and brain cell-damaging infective diseases including Creutzfeldt-Jakob disease. The treatments can be conducted in both specialized therapy facilities and in general therapy sites (such as general hospitals, during ambulance transportation, or at the site of incidents). These are revolutionary treatments, since they enable therapies of disorders that cannot be treated by conventional treatments, and further, these treatments can be achieved using a simple procedure, such as intravenous administration. In addition, since neurological injuries cause severe disorders in patients, patients can benefit tremendously from therapies for these disorders, which is of tremendous social significance.

The medical action mechanisms of the regenerative medicines of the present invention are as follows: Transplanted bone marrow cells or mesenchymal stem cells migrate to and fixate in an affected area (an *in vivo* injury site), recovering the functions of the affected area by secreting appropriate substances, accelerating inherent autotherapy, or differentiating into appropriate cells. Accordingly, the regenerative medicines of the present invention can exert effects on all types of diseases and events accompanied by neurological injury.

## Claims

1. A cranial nerve disease therapeutic agent *for in vivo* administration, comprising a mesenchymal cell as an active ingredient.

2. The agent of claim 1, wherein the cranial nerve disease is cerebral infarction.

3. An agent for *in vivo* administration, exhibiting neuroprotection and comprising a mesenchymal cell as an active ingredient.

4. An agent *for in vivo* administration, exhibiting cranial nerve regeneration and comprising a mesenchymal cell as an active ingredient.

5. The agent of any one of claims 1 to 4, wherein the *in vivo* administration is intravenous.

6. The agent of any one of claims 1 to 5, wherein the mesenchymal cell is:

   (a) a mesenchymal cell introduced with a BDNF gene, PLGF gene, GDNF gene, or IL-2 gene; or
   (b) an immortalized mesenchymal cell introduced with an hTERT gene.

7. The agent of any one of claims 1 to 6, wherein the mesenchymal cell is a mesenchymal stem cell.

8. The agent of any one of claims 1 to 6, wherein the mesenchymal cell is a bone marrow cell, a cord blood cell, or a peripheral blood cell.

9. A method for treating a cranial nerve disease comprising the *in vivo* administration to a patient of a therapeutically effective amount of the agent of any one of claims 1 to 8.

10. The method of claim 9, wherein the bone marrow cell is an autologous cell of the patient.

11. The method of claim 9 or 10, wherein the cranial nerve disease is cerebral infarction.

12. The method of any one of claims 9 to 11, wherein the *in vivo* administration is intravenous administration.

13. The method of any one of claims 9 to 12, wherein the mesenchymal cell is a bone marrow cell, a cord blood cell, or a peripheral blood cell.

FIG. 1

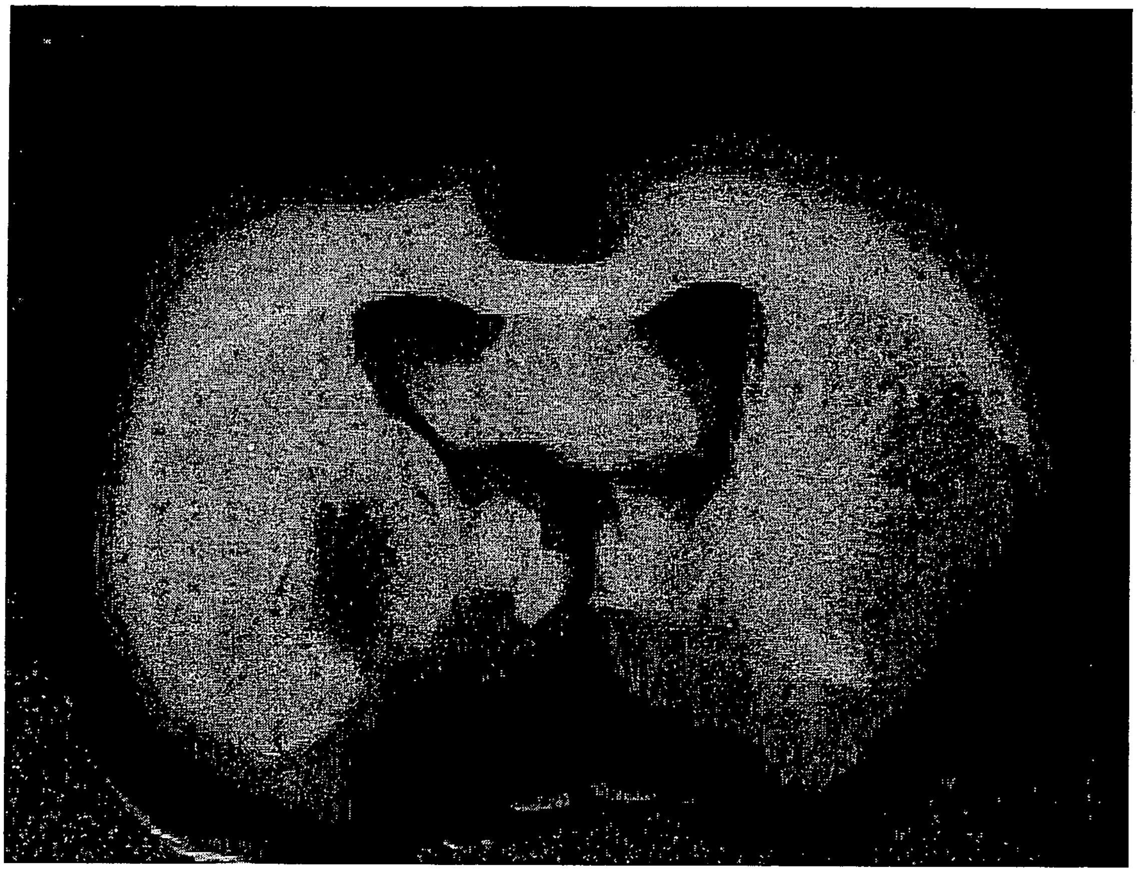

FIG. 2

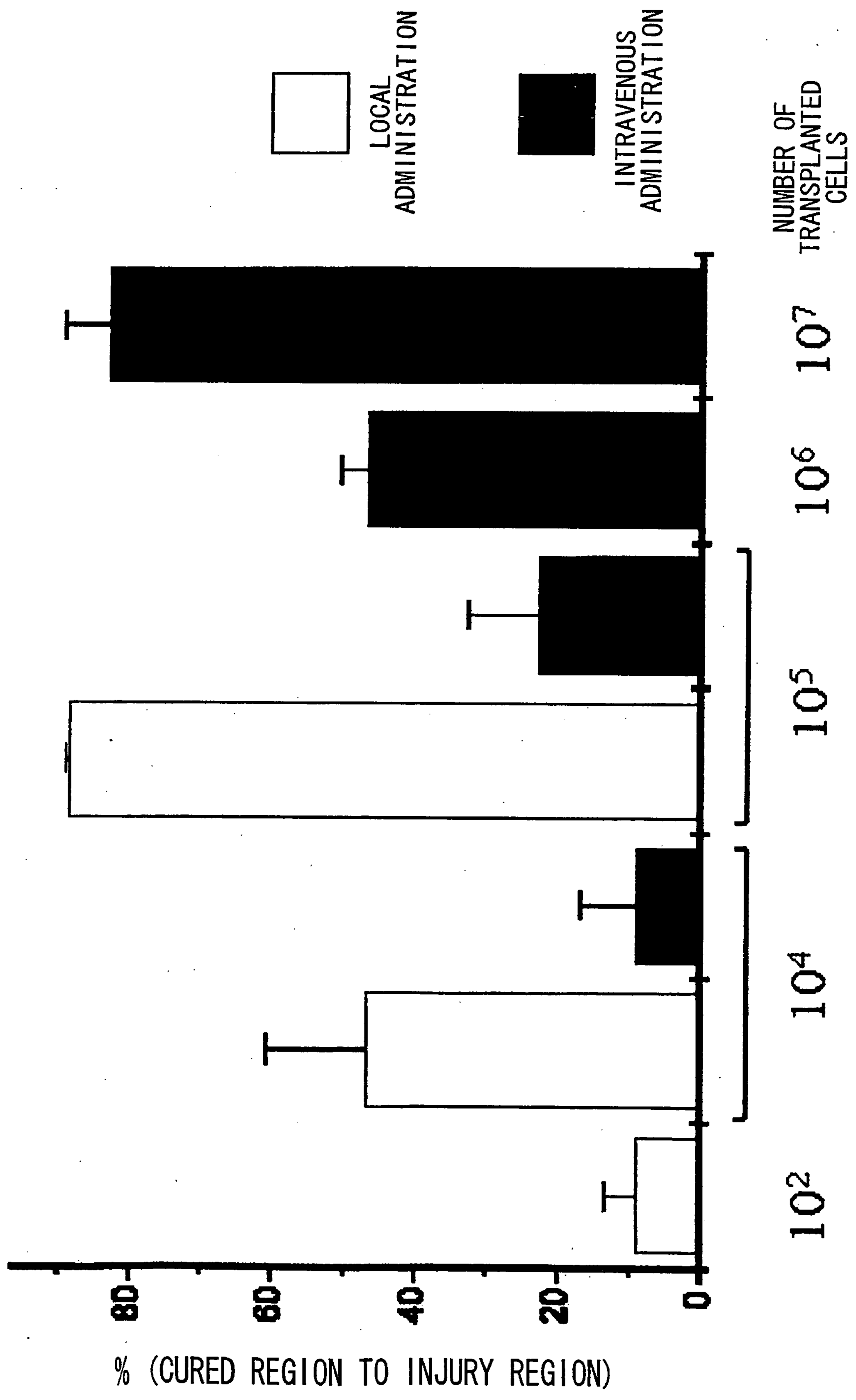
LOCAL ADMINISTRATION
INTRAVENOUS ADMINISTRATION
NUMBER OF TRANSPLANTED CELLS
$10^2$
$10^4$
$10^5$
$10^6$
$10^7$
0
20
40
60
80
% (CURED REGION TO INJURY REGION)

FIG. 3

A B C D E F

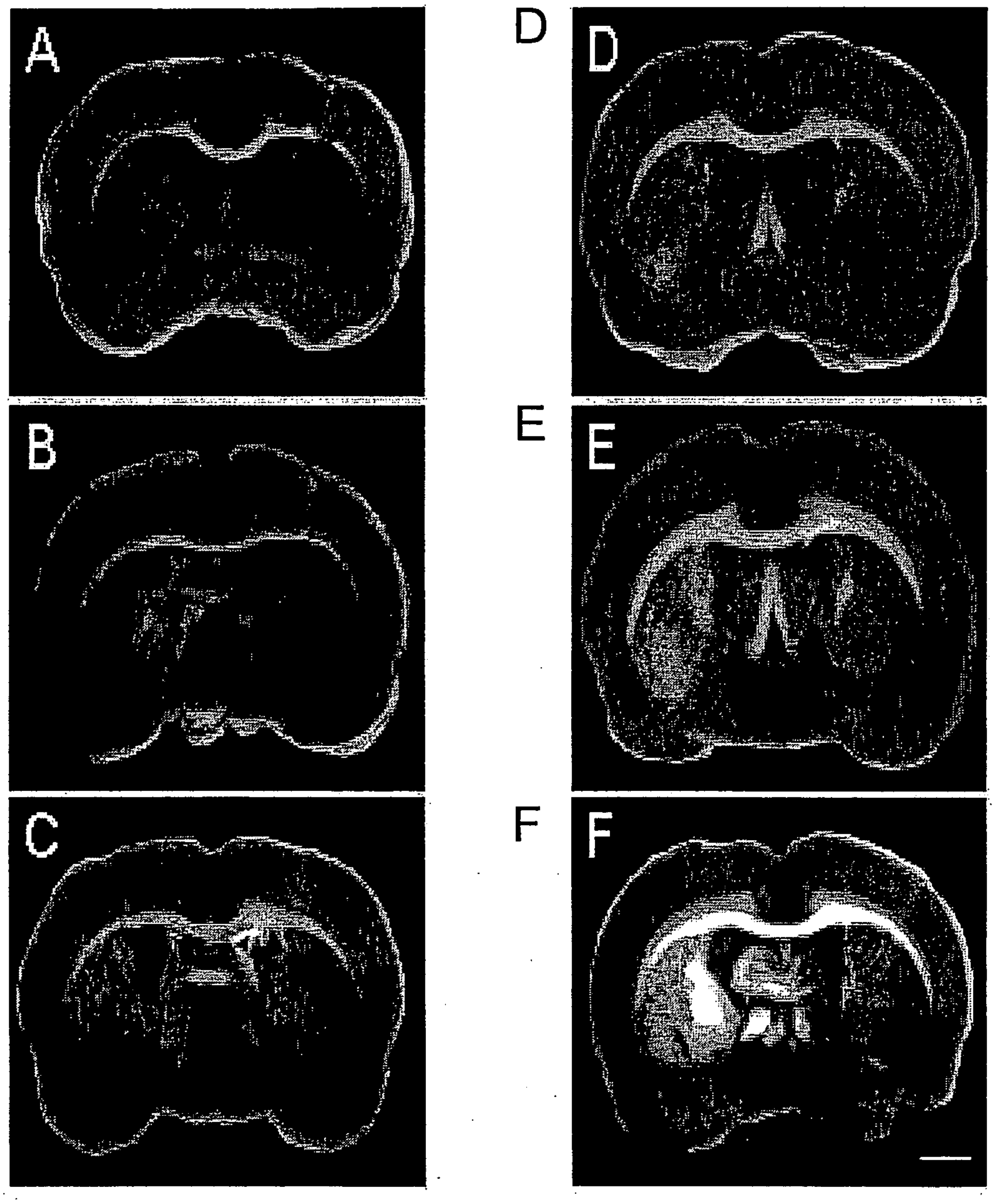

FIG. 4

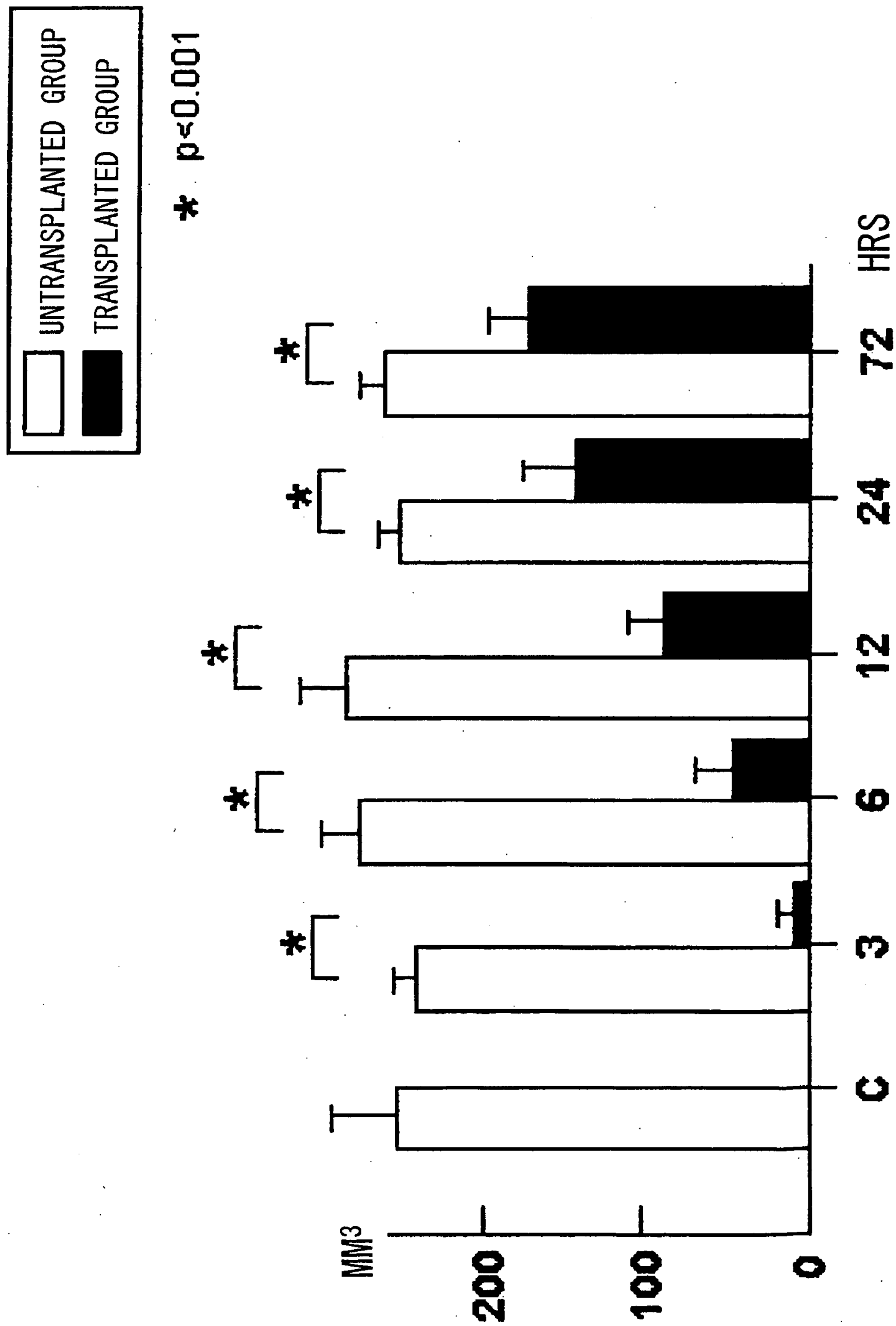
UNTRANSPLANTED GROUP
TRANSPLANTED GROUP
* p<0.001
MM3
200
100
0
C
3
6
12
24
72
HRS

FIG. 5

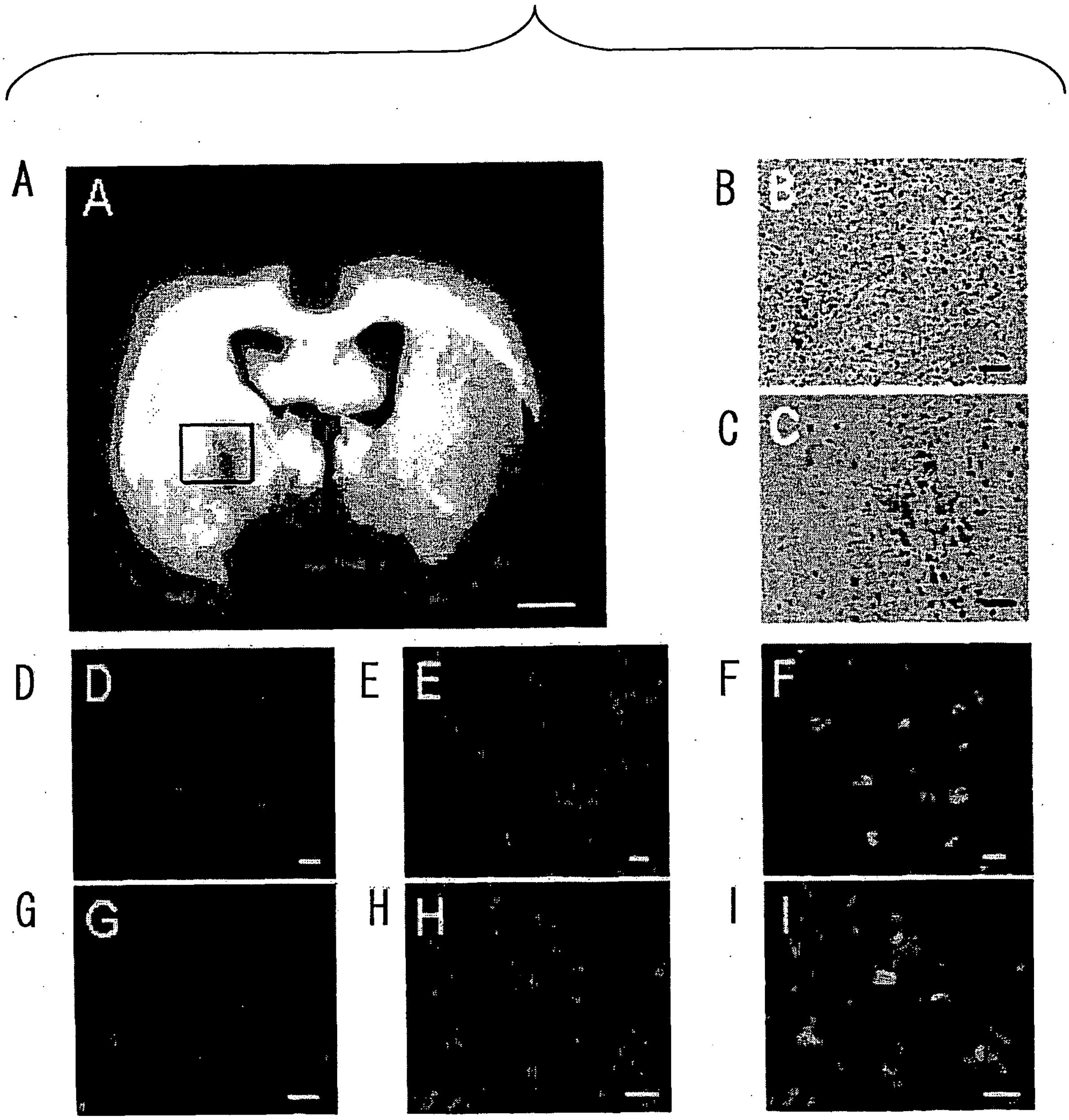

FIG. 6

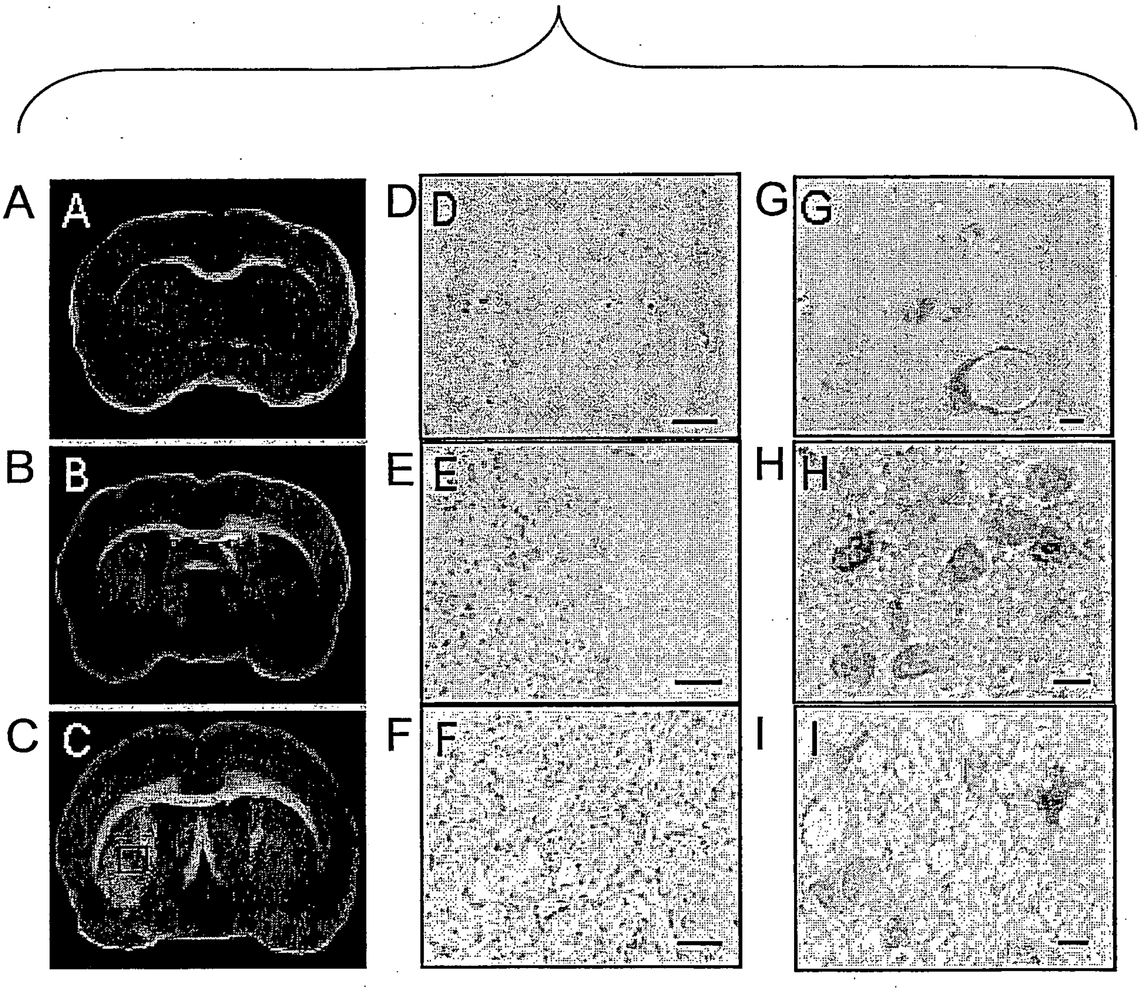

FIG. 7

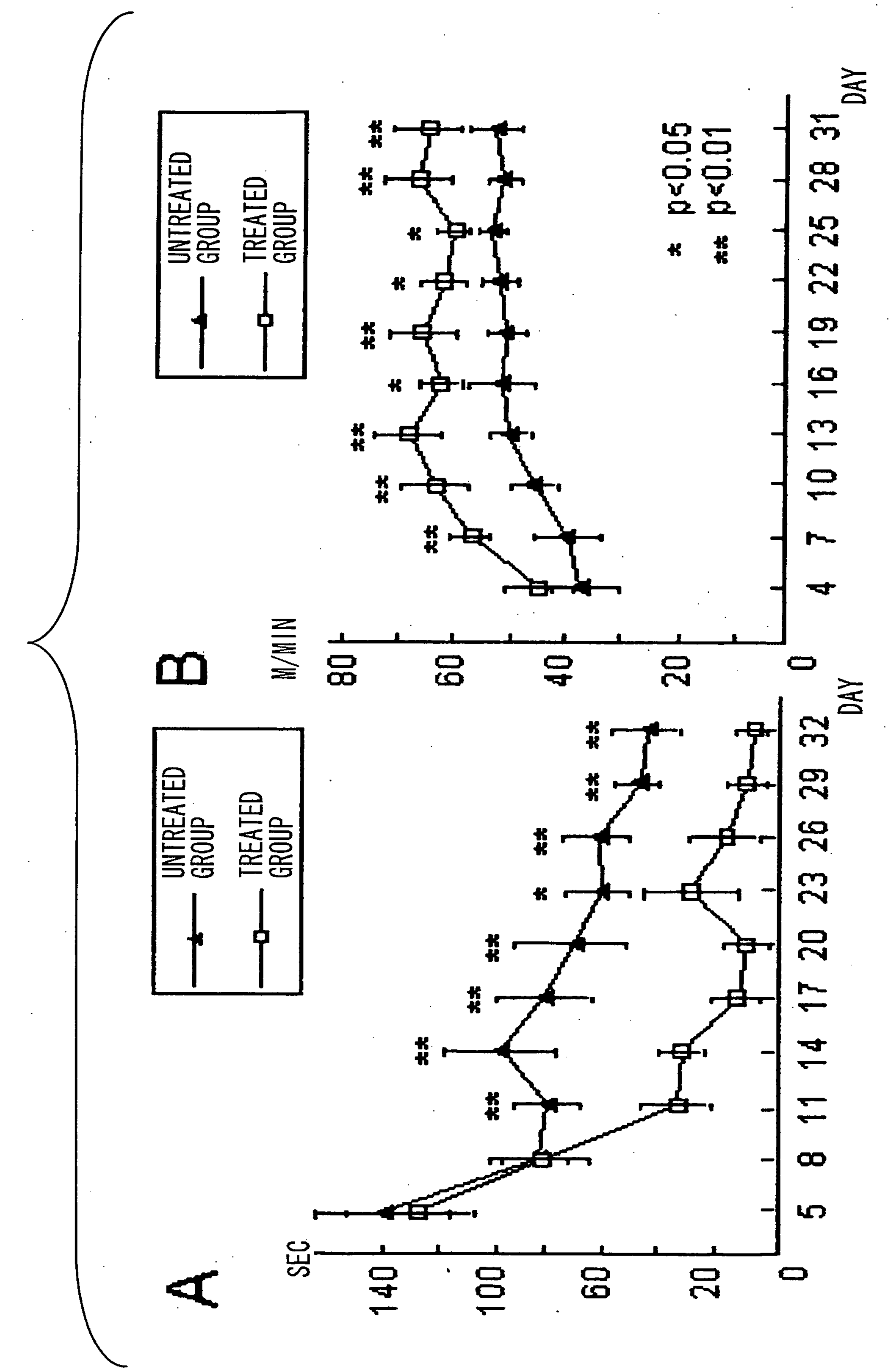
A
SEC
140
100
60
20
0
5 8 11 14 17 20 23 26 29 32
DAY
UNTREATED GROUP
TREATED GROUP
B
M/MIN
80
60
40
20
0
4 7 10 13 16 19 22 25 28 31
DAY
UNTREATED GROUP
TREATED GROUP
* p<0.05
** p<0.01

FIG. 8

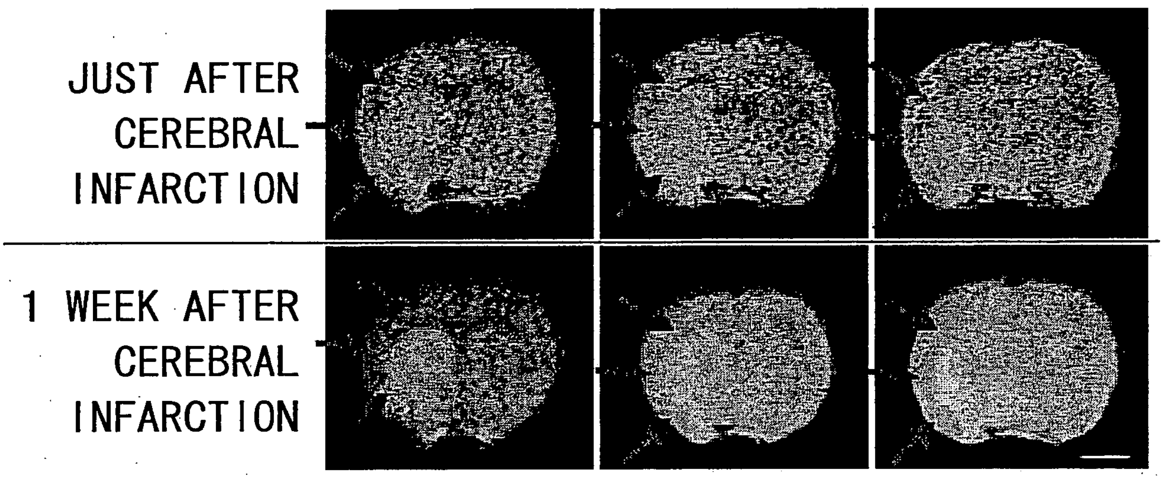
JUST AFTER CEREBRAL INFARCTION
1 WEEK AFTER CEREBRAL INFARCTION

FIG. 9

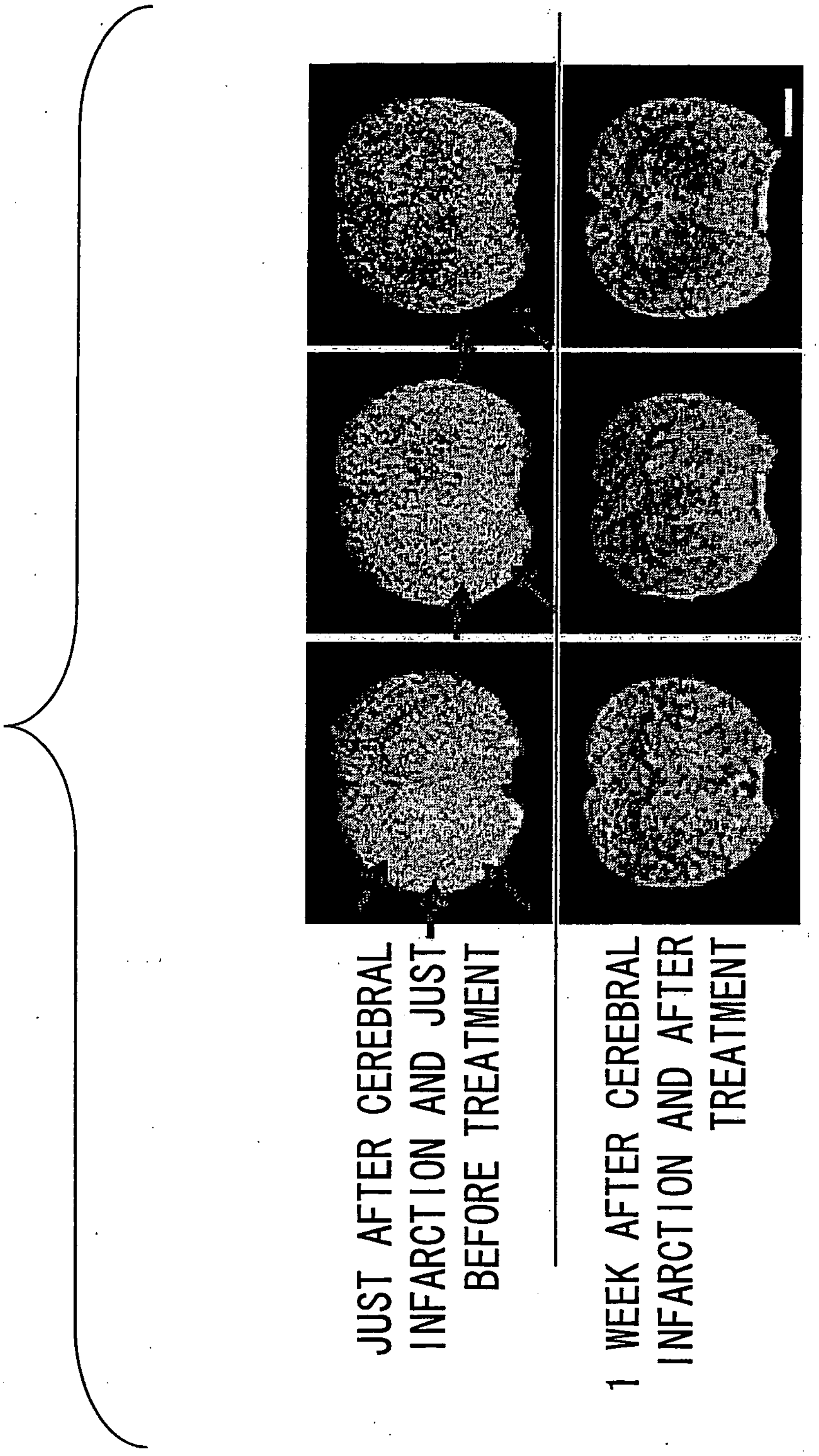
JUST AFTER CEREBRAL INFARCTION AND JUST BEFORE TREATMENT
1 WEEK AFTER CEREBRAL INFARCTION AND AFTER TREATMENT

# FIG. 10

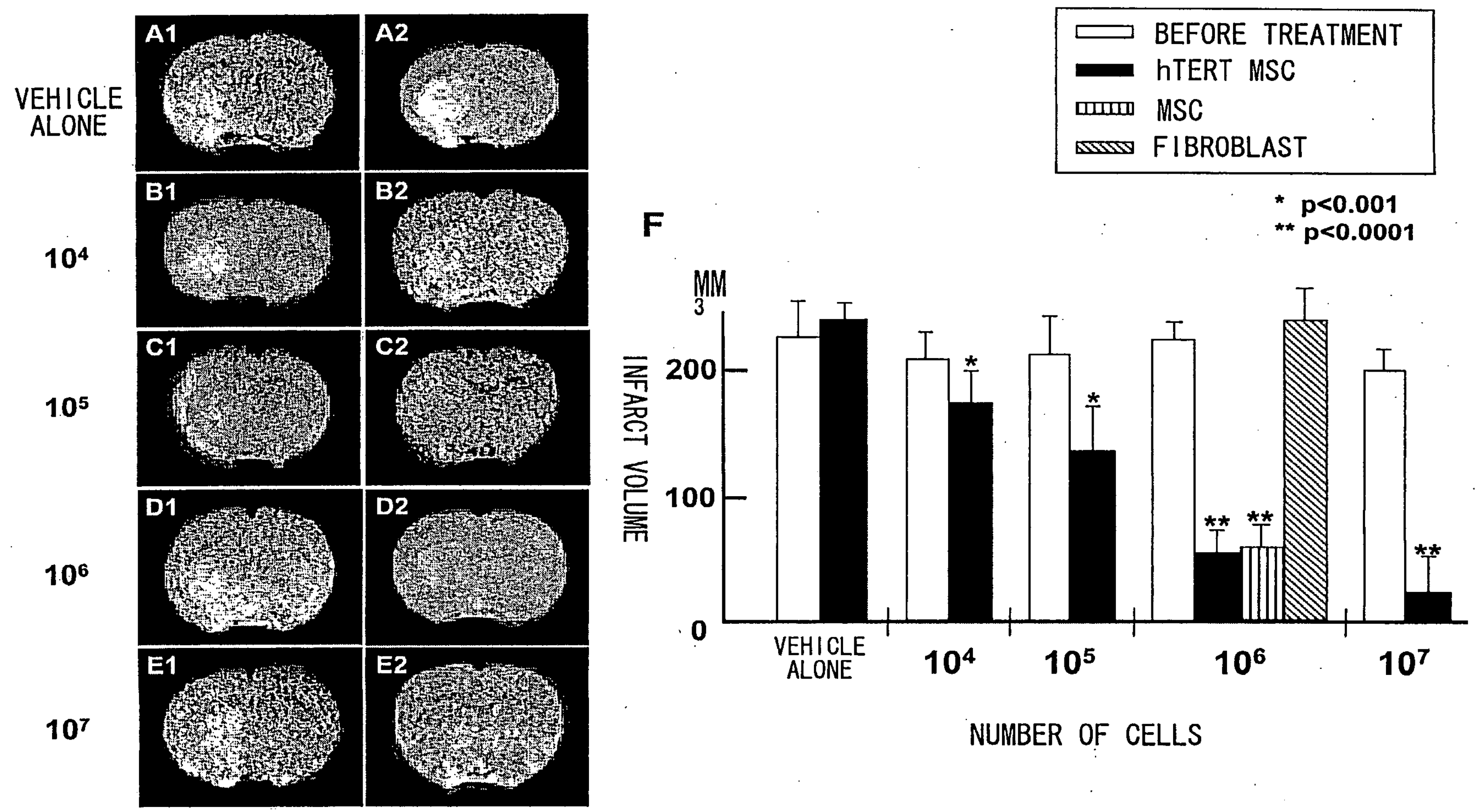
VEHICLE ALONE
$10^4$
$10^5$
$10^6$
$10^7$
A1
A2
B1
B2
C1
C2
D1
D2
E1
E2
F
BEFORE TREATMENT
hTERT MSC
MSC
FIBROBLAST
* p<0.001
** p<0.0001
MM 3
INFARCT VOLUME
200
100
0
VEHICLE ALONE
$10^4$
$10^5$
$10^6$
$10^7$
NUMBER OF CELLS

FIG. 11

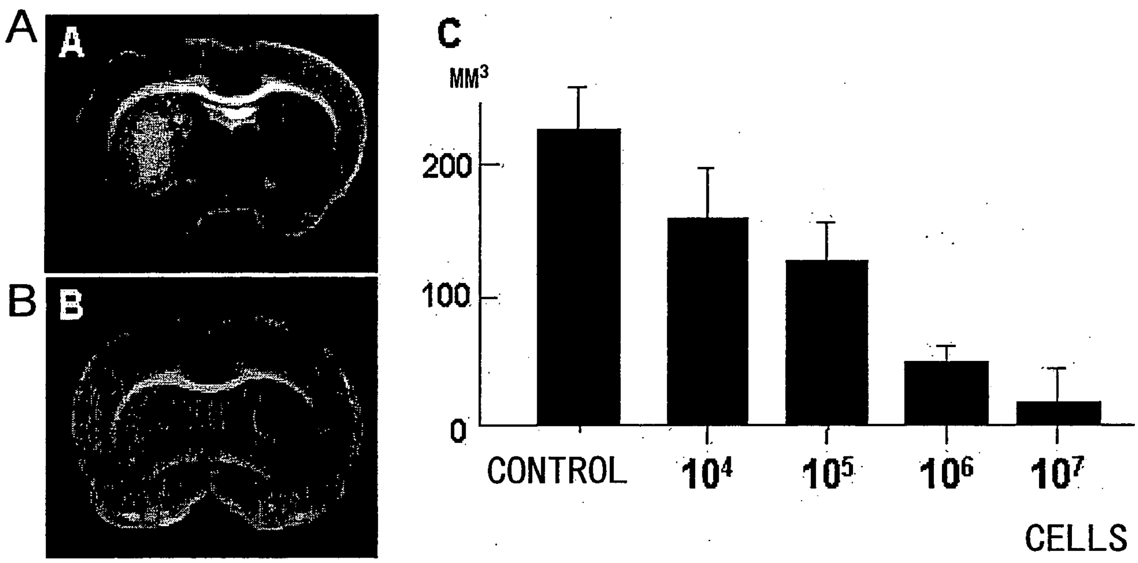

FIG. 12

A C E

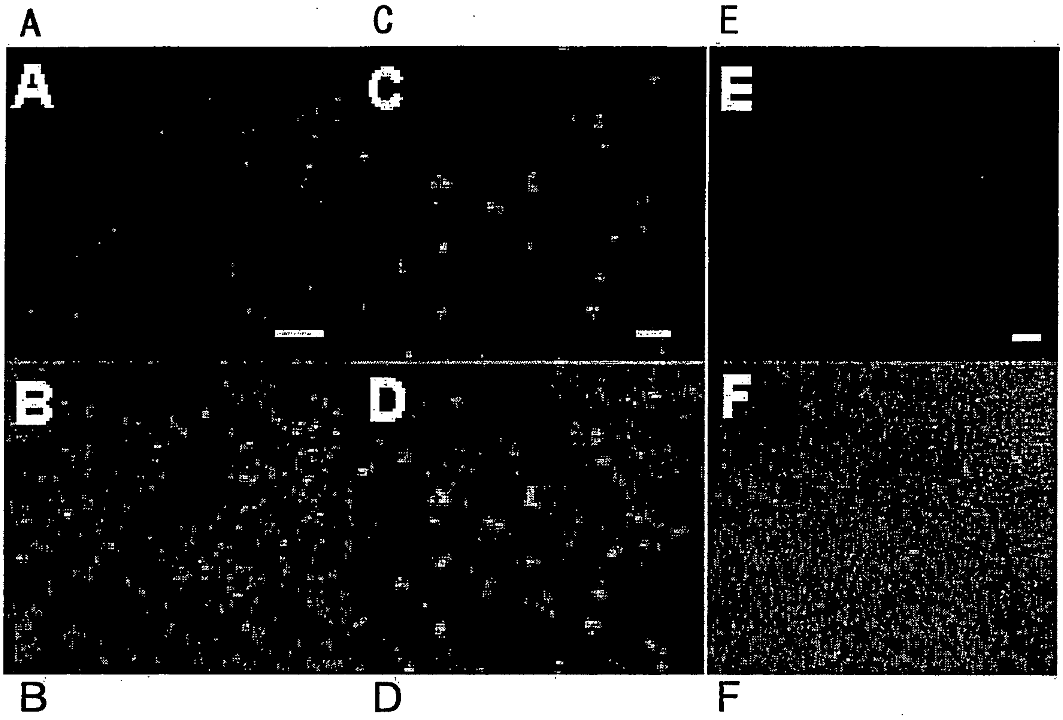


B D F

G (LacZ) I (NeuN) K (MERGED)

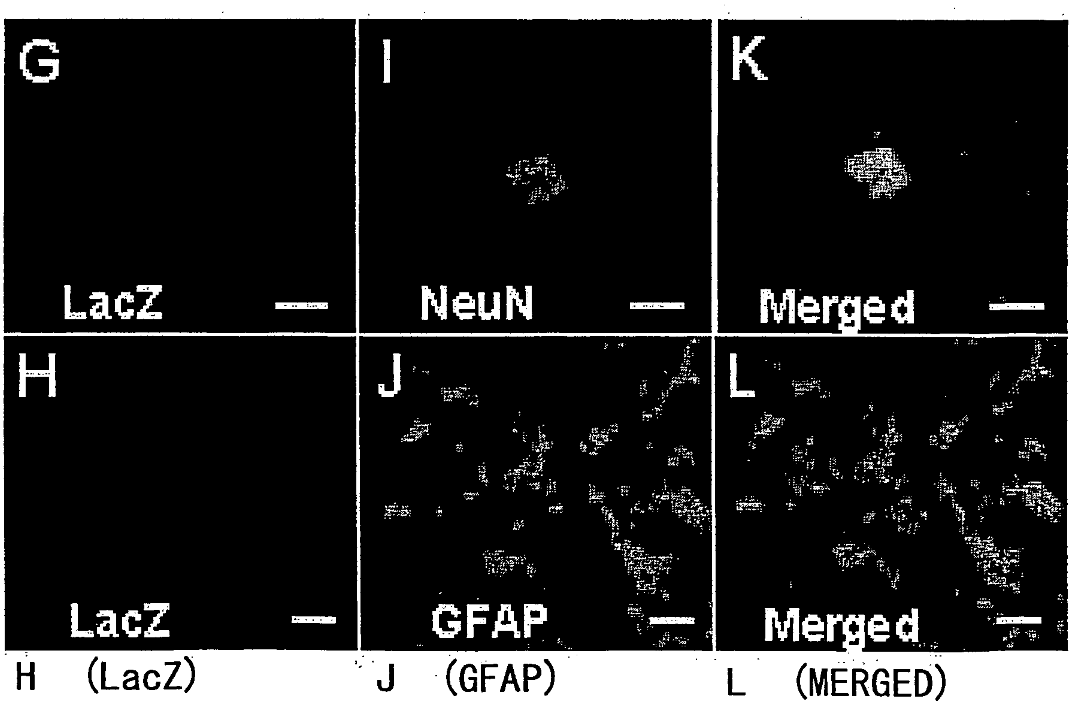


H (LacZ) J (GFAP) L (MERGED)

FIG. 13

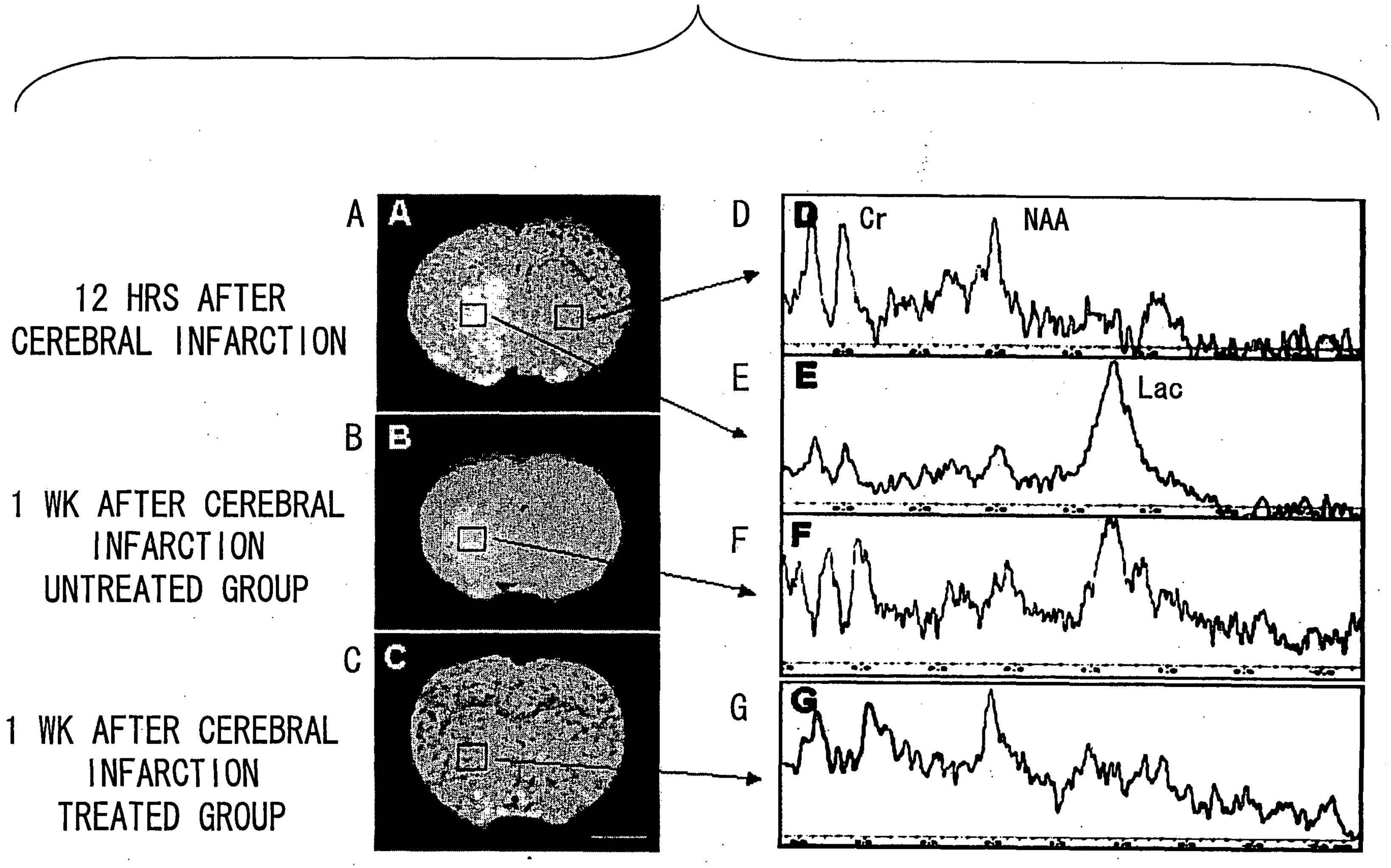
A
12 HRS AFTER CEREBRAL INFARCTION
B
1 WK AFTER CEREBRAL INFARCTION UNTREATED GROUP
C
1 WK AFTER CEREBRAL INFARCTION TREATED GROUP
A
B
C
D
E
F
G
Cr
NAA
Lac

FIG. 14

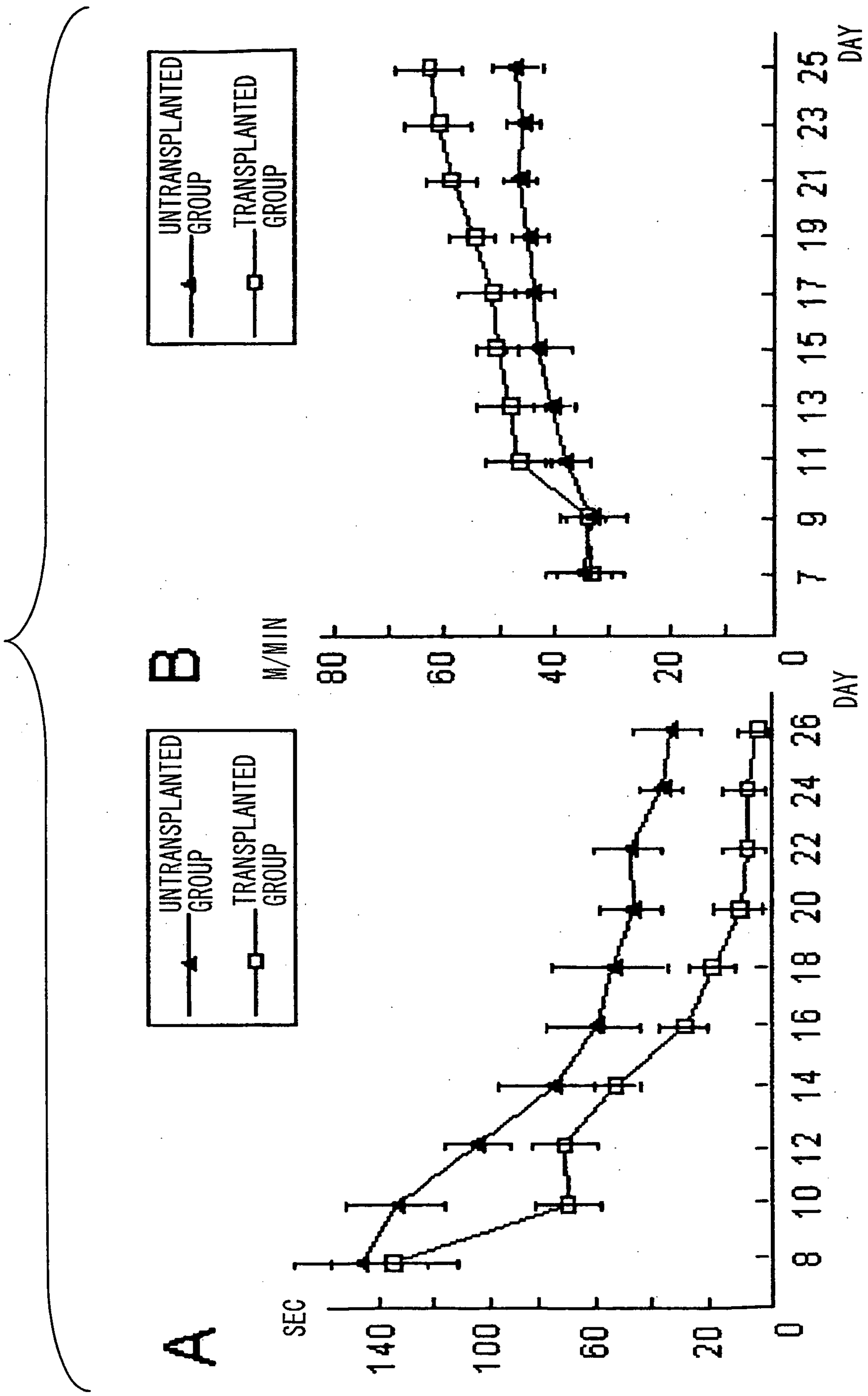
A
SEC
140
100
60
20
0
8 10 12 14 16 18 20 22 24 26
DAY
UNTRANSPLANTED GROUP
TRANSPLANTED GROUP
B
M/MIN
80
60
40
20
0
7 9 11 13 15 17 19 21 23 25
DAY
UNTRANSPLANTED GROUP
TRANSPLANTED GROUP

FIG. 15

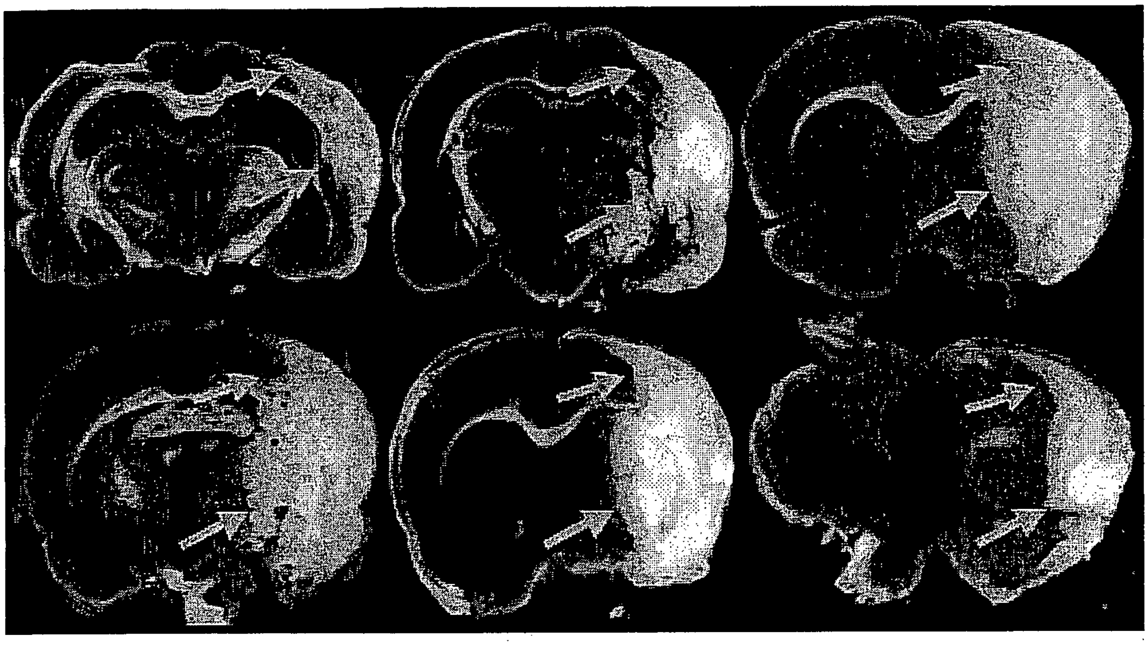

FIG. 16

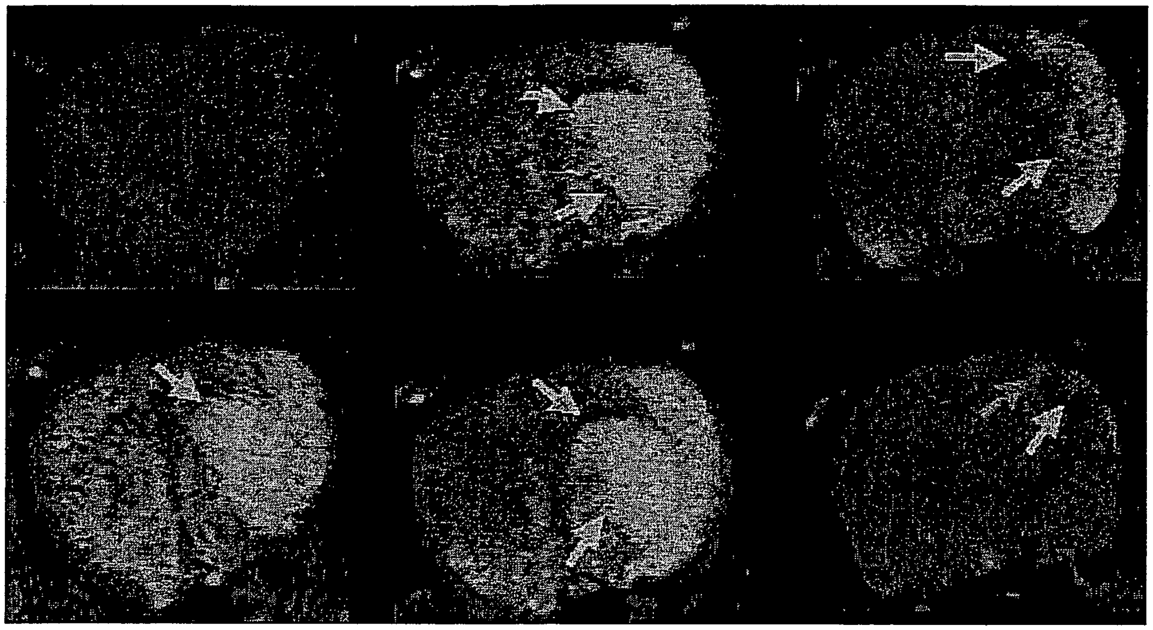

FIG. 17

12 HRS LATER | 3 DAYS LATER | 7 DAYS LATER

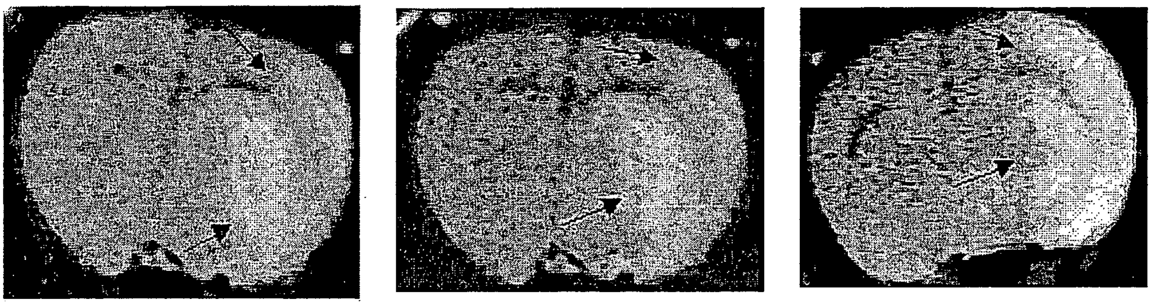

FIG. 18

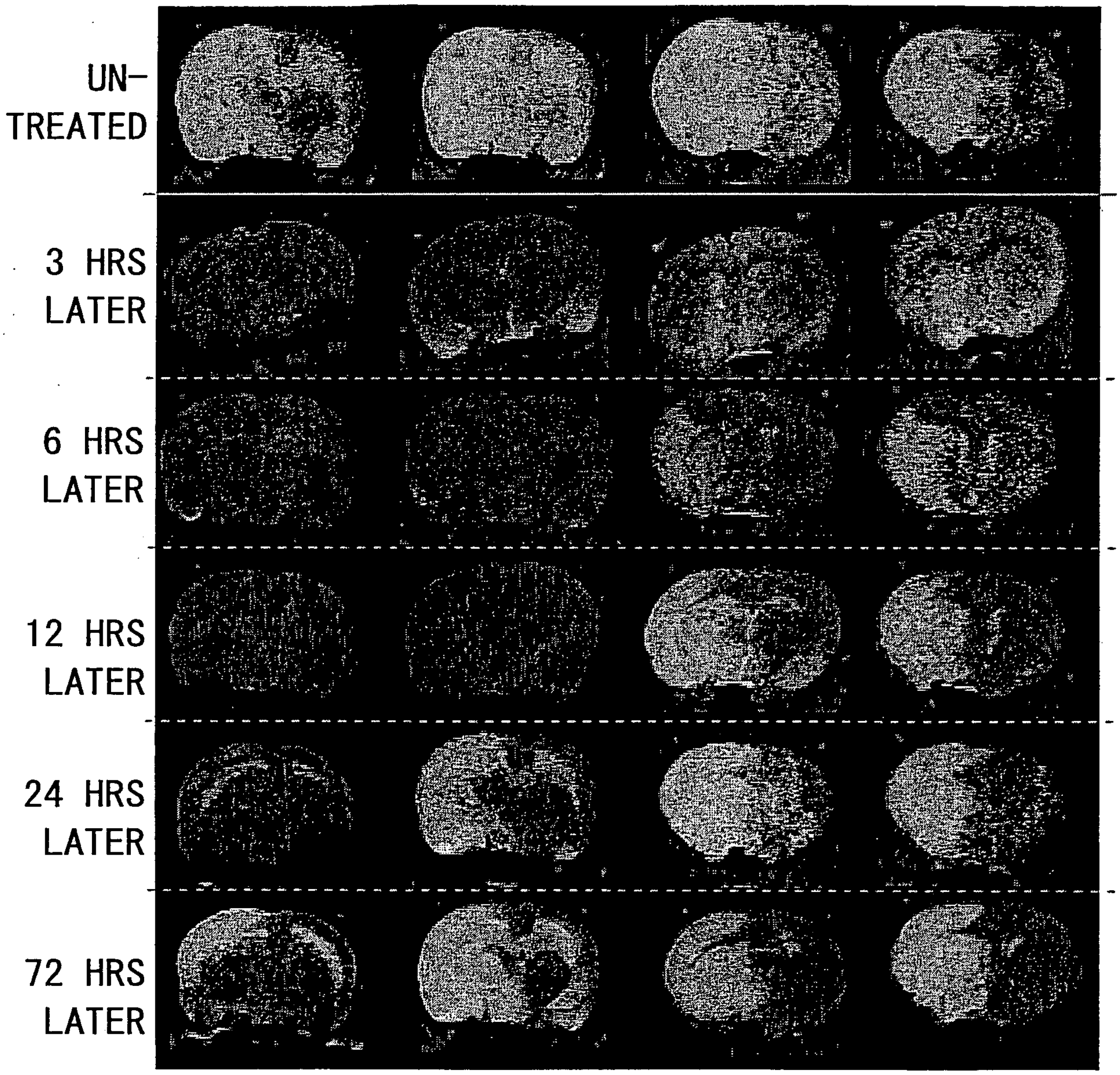
UN-
TREATED
3 HRS
LATER
6 HRS
LATER
12 HRS
LATER
24 HRS
LATER
72 HRS
LATER

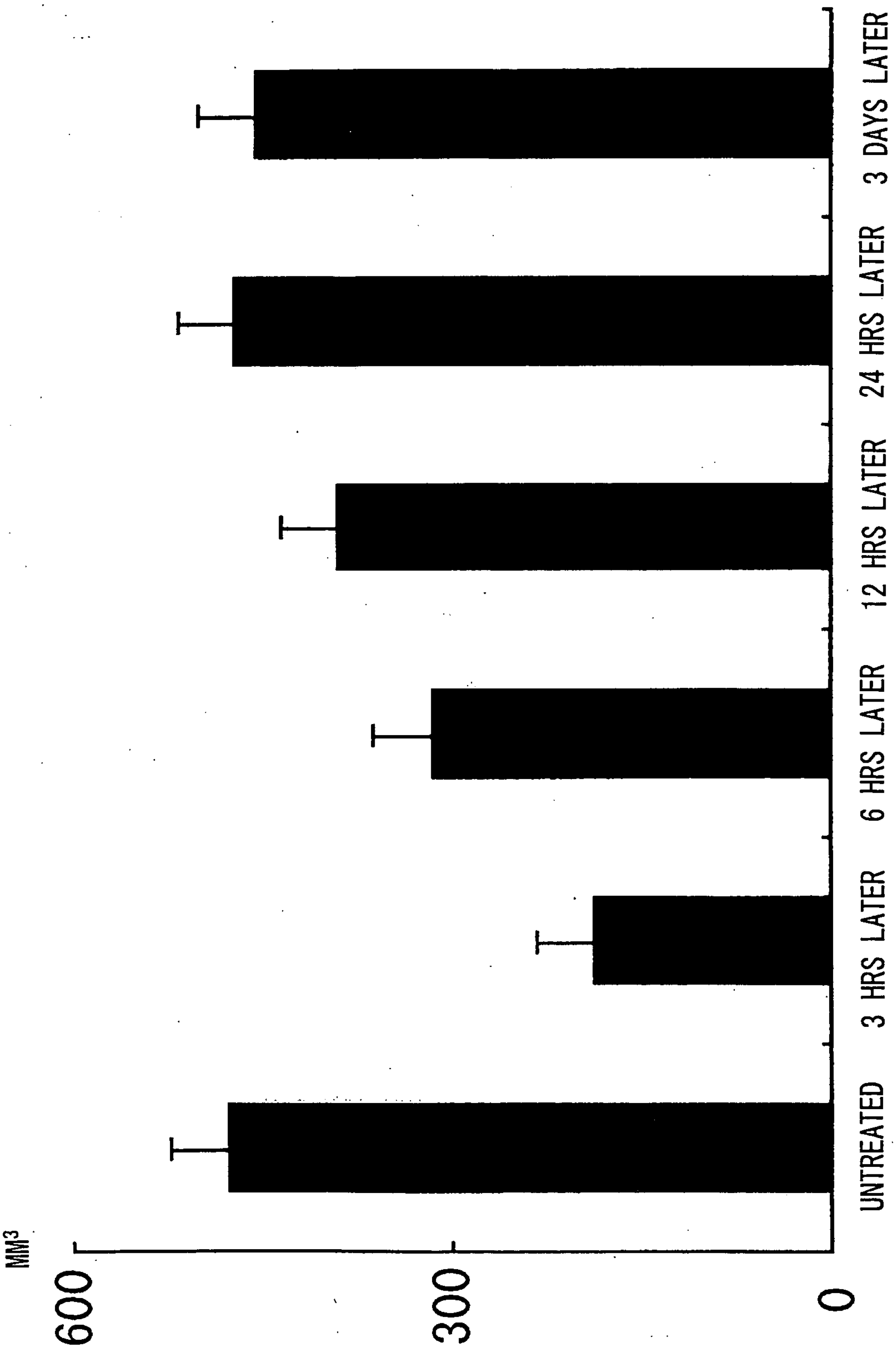
MM$^3$
600
300
0
UNTREATED
3 HRS LATER
6 HRS LATER
12 HRS LATER
24 HRS LATER
3 DAYS LATER

FIG. 19

FIG. 20

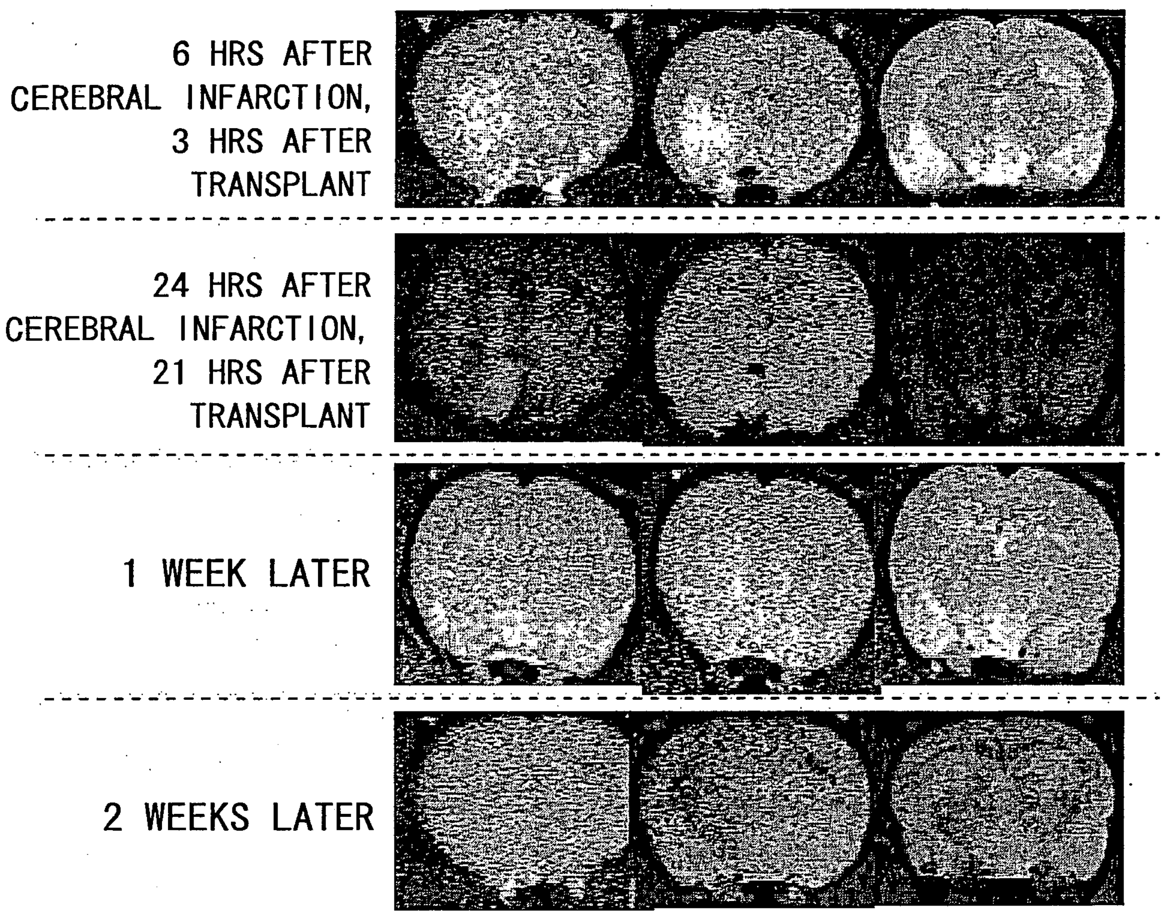
6 HRS AFTER CEREBRAL INFARCTION, 3 HRS AFTER TRANSPLANT
24 HRS AFTER CEREBRAL INFARCTION, 21 HRS AFTER TRANSPLANT
1 WEEK LATER
2 WEEKS LATER

FIG. 21

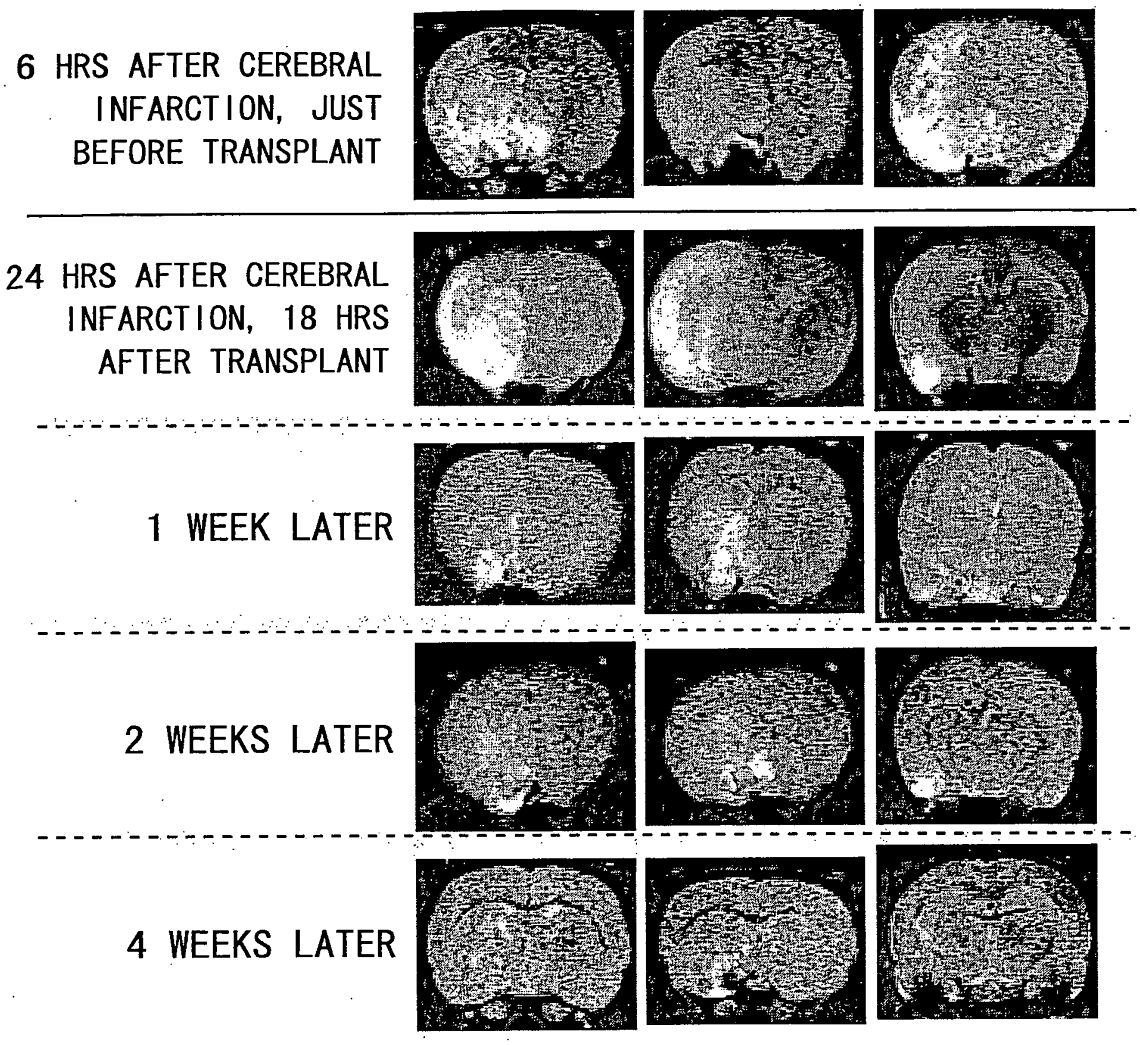
6 HRS AFTER CEREBRAL INFARCTION, JUST BEFORE TRANSPLANT
24 HRS AFTER CEREBRAL INFARCTION, 18 HRS AFTER TRANSPLANT
1 WEEK LATER
2 WEEKS LATER
4 WEEKS LATER

FIG. 22

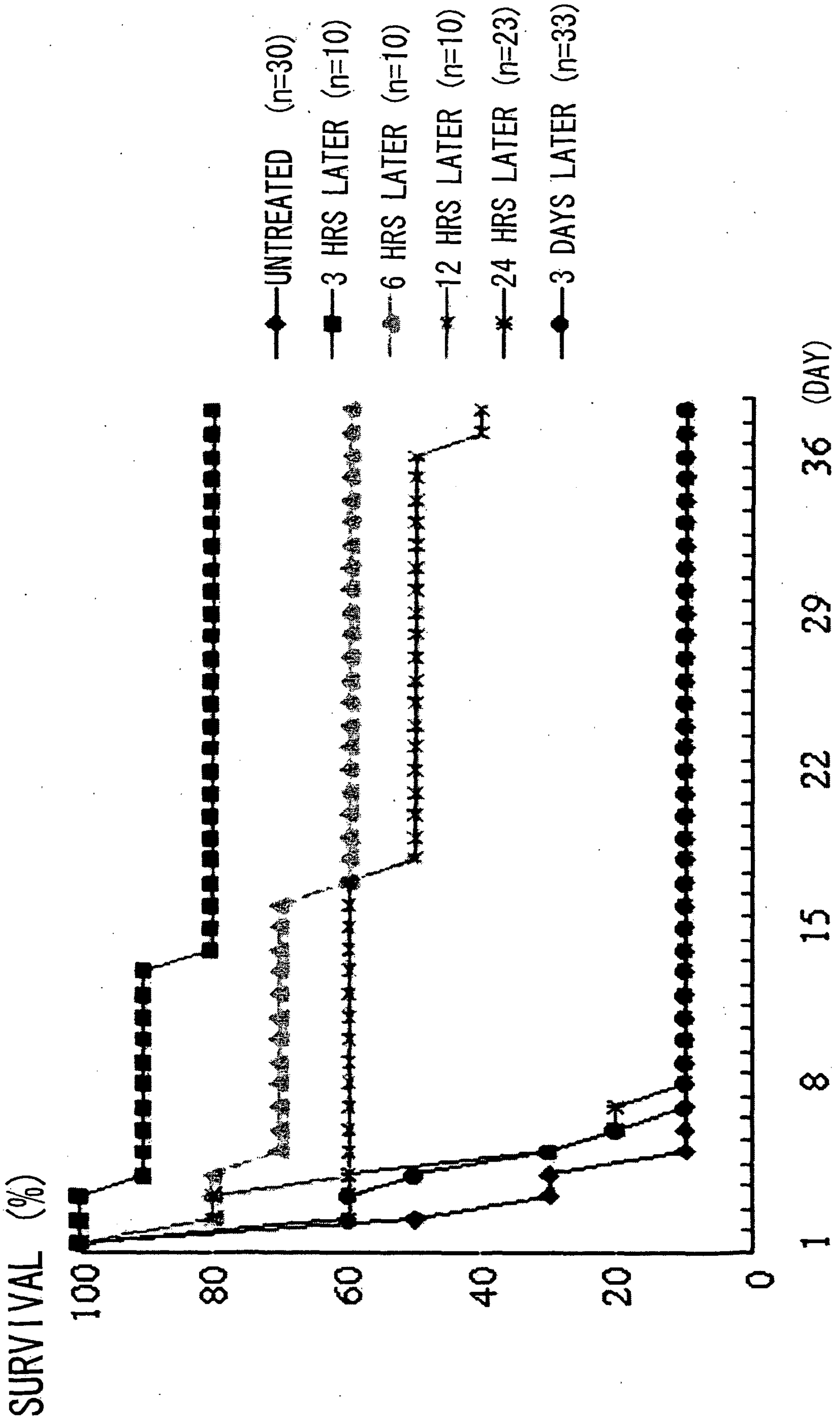
SURVIVAL (%)
100
80
60
40
20
0
1
8
15
22
29
36
(DAY)
UNTREATED (n=30)
3 HRS LATER (n=10)
6 HRS LATER (n=10)
12 HRS LATER (n=10)
24 HRS LATER (n=23)
3 DAYS LATER (n=33)

FIG. 23

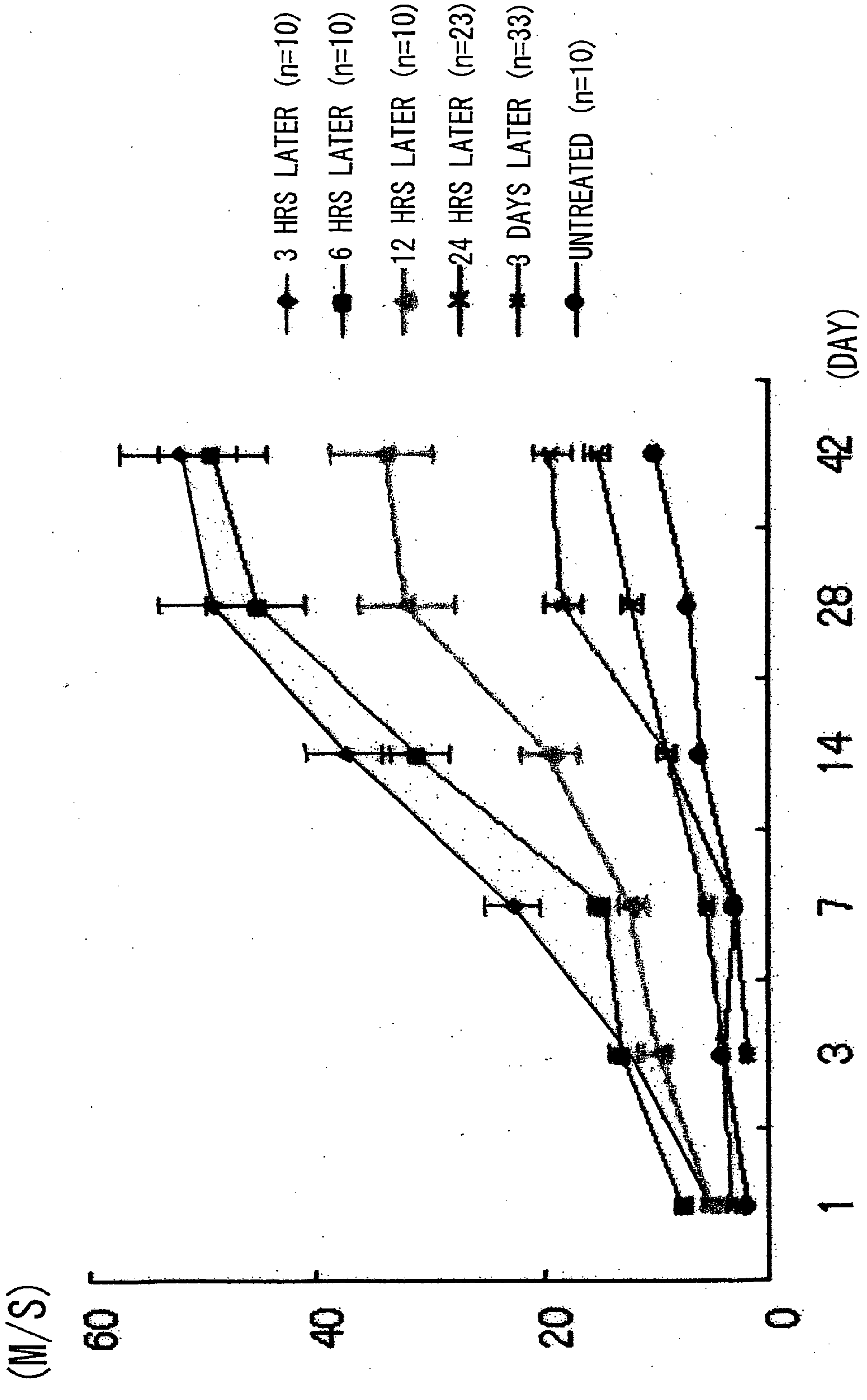
(M/S)
60
40
20
0
1
3
7
14
28
42
(DAY)
3 HRS LATER (n=10)
6 HRS LATER (n=10)
12 HRS LATER (n=10)
24 HRS LATER (n=23)
3 DAYS LATER (n=33)
UNTREATED (n=10)

# FIG. 24

MSC CELLS

CONTROL G-CSF DAY 3 S.C.

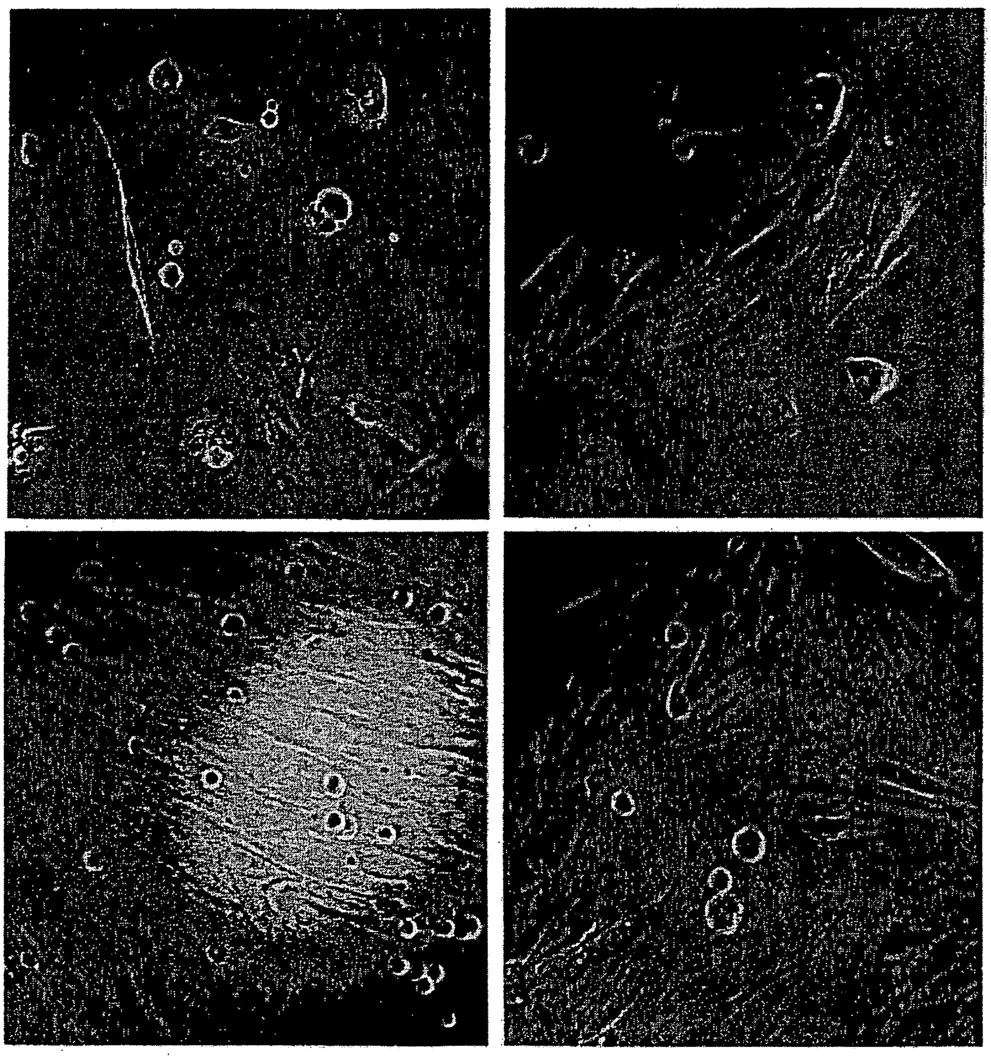

G-CSF+SCF DAY 7 S.C. CORD BLOOD

FIG. 25

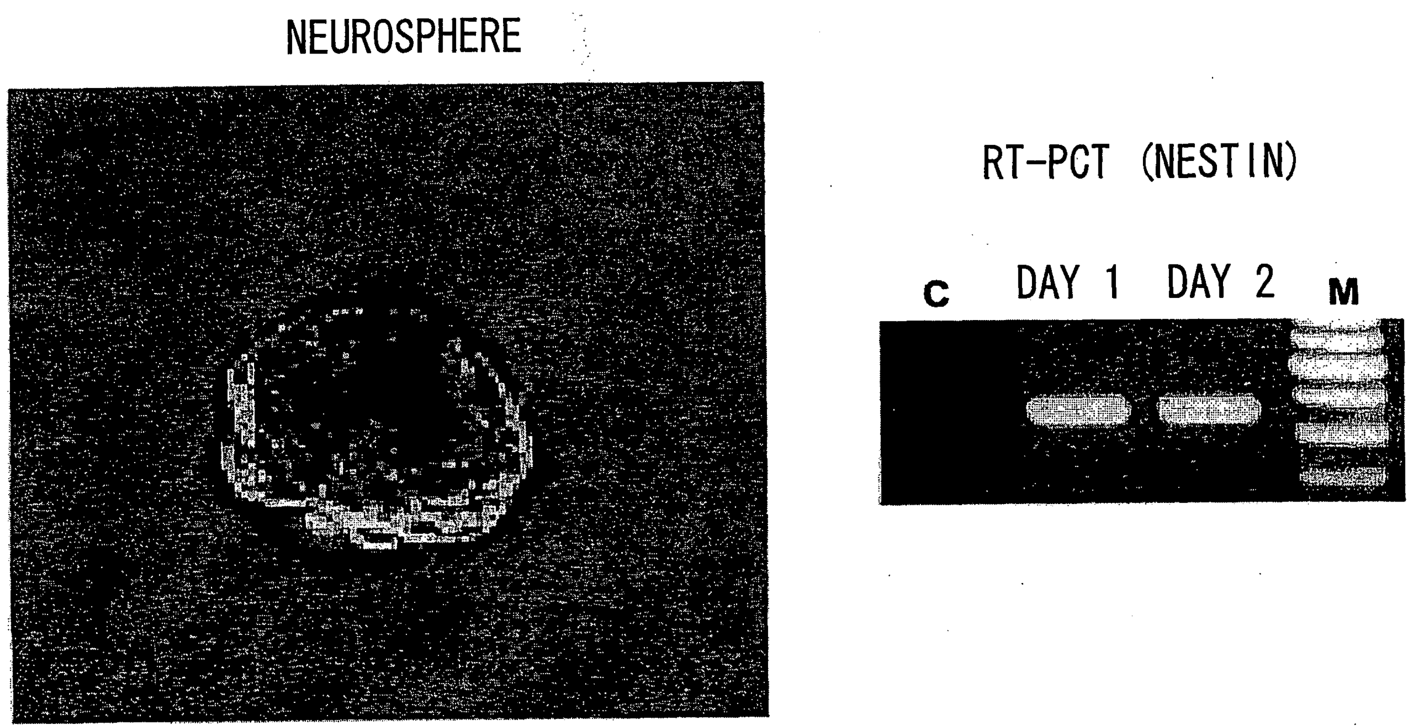

FIG. 26

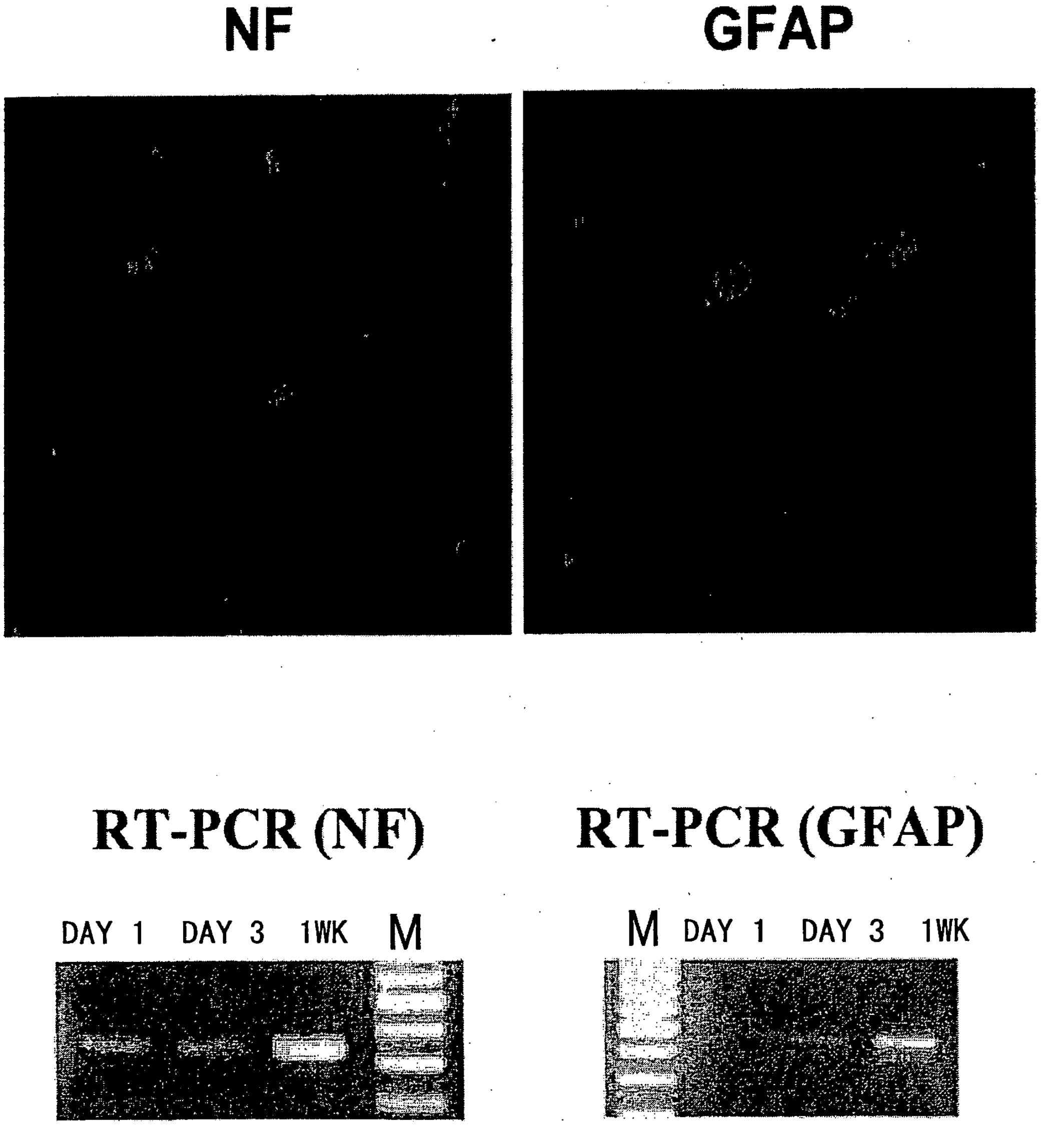

FIG. 27

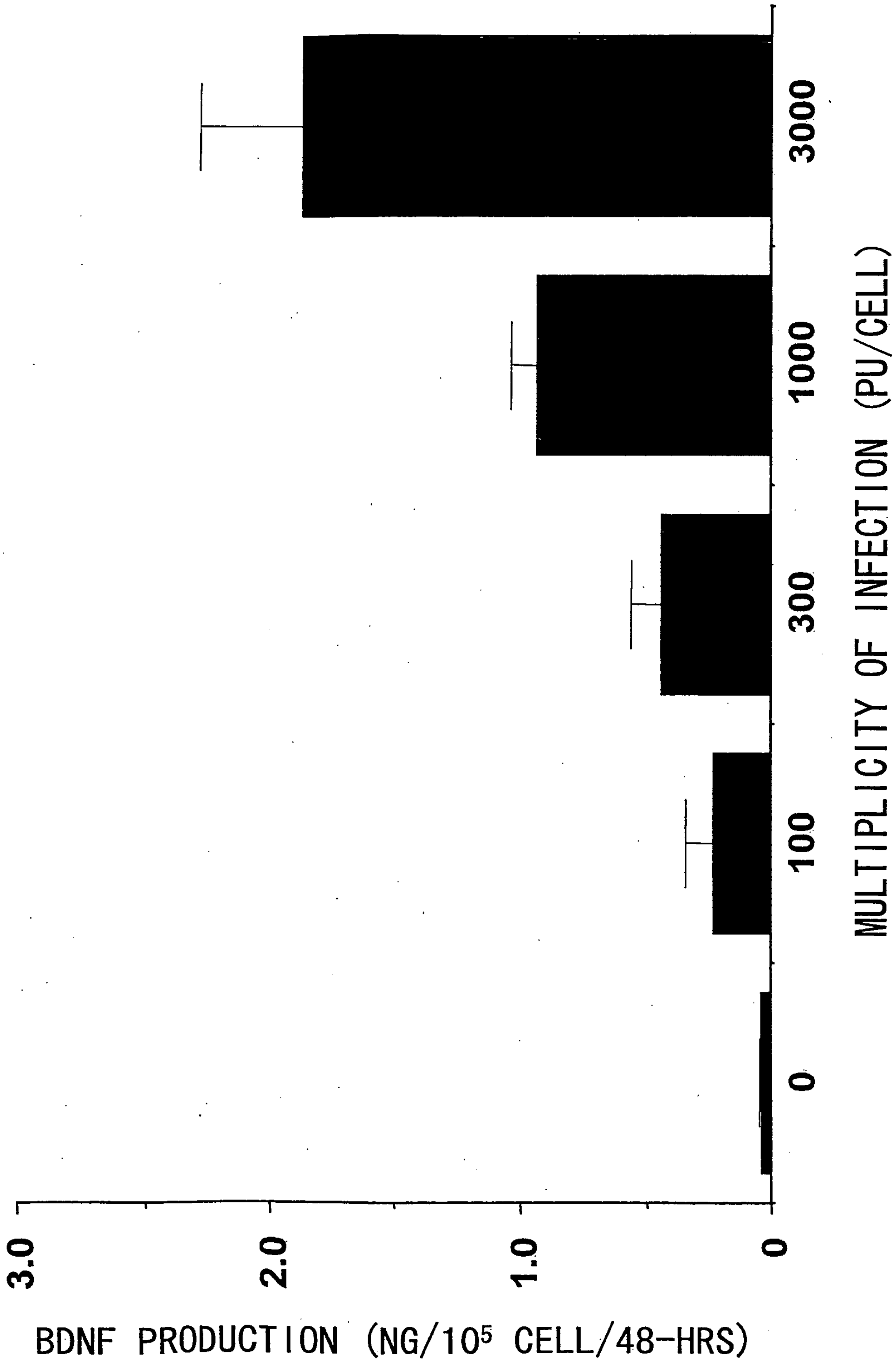
3000
1000
300
100
0
MULTIPLICITY OF INFECTION (PU/CELL)
3.0
2.0
1.0
0
BDNF PRODUCTION (NG/10⁵ CELL/48-HRS)

FIG. 28

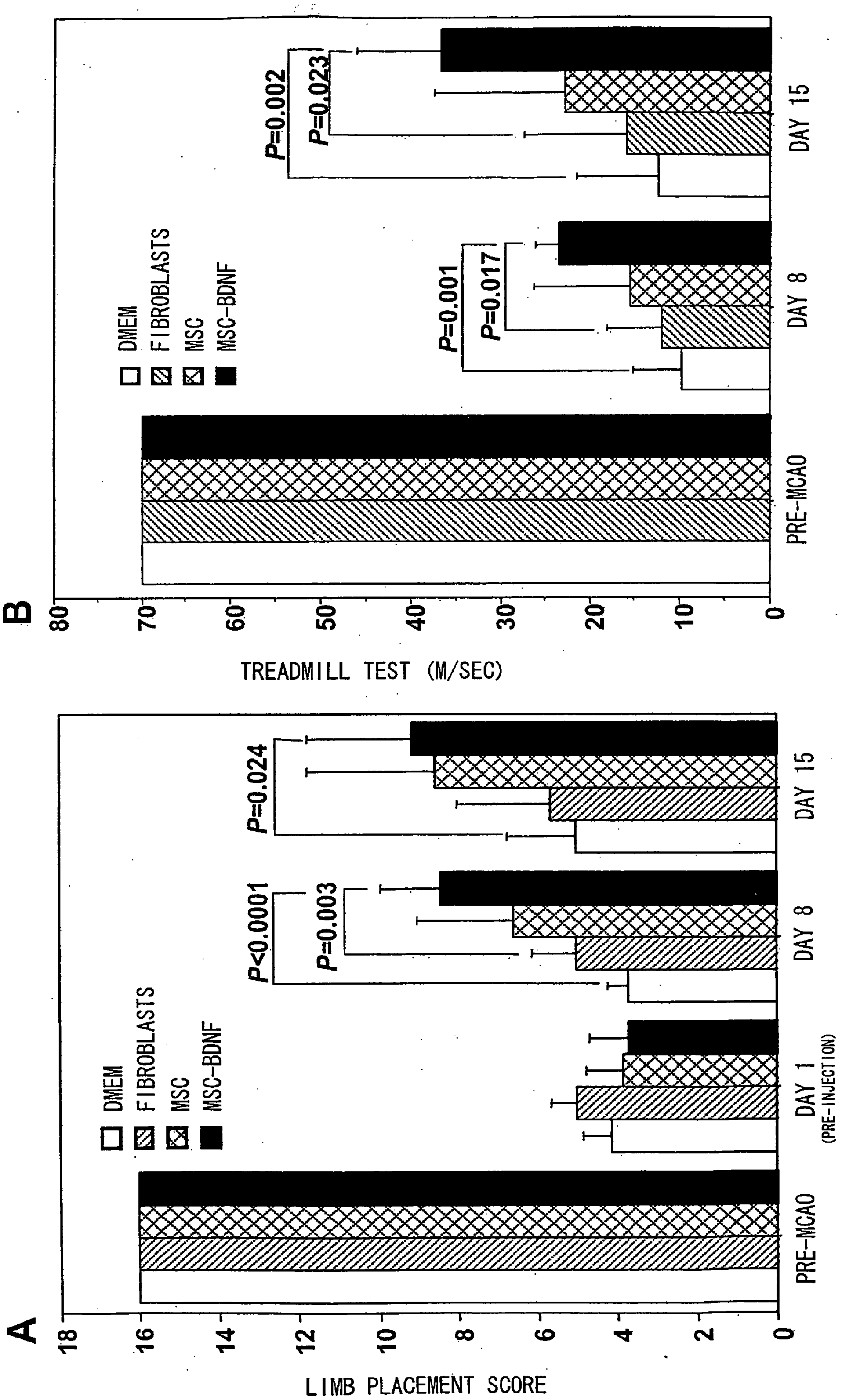
A
LIMB PLACEMENT SCORE
0
2
4
6
8
10
12
14
16
18
DMEM
FIBROBLASTS
MSC
MSC-BDNF
PRE-MCAO
DAY 1
(PRE-INJECTION)
DAY 8
DAY 15
P<0.0001
P=0.003
P=0.024
B
TREADMILL TEST (M/SEC)
0
10
20
30
40
50
60
70
80
DMEM
FIBROBLASTS
MSC
MSC-BDNF
PRE-MCAO
DAY 8
DAY 15
P=0.001
P=0.017
P=0.002
P=0.023

FIG. 29A

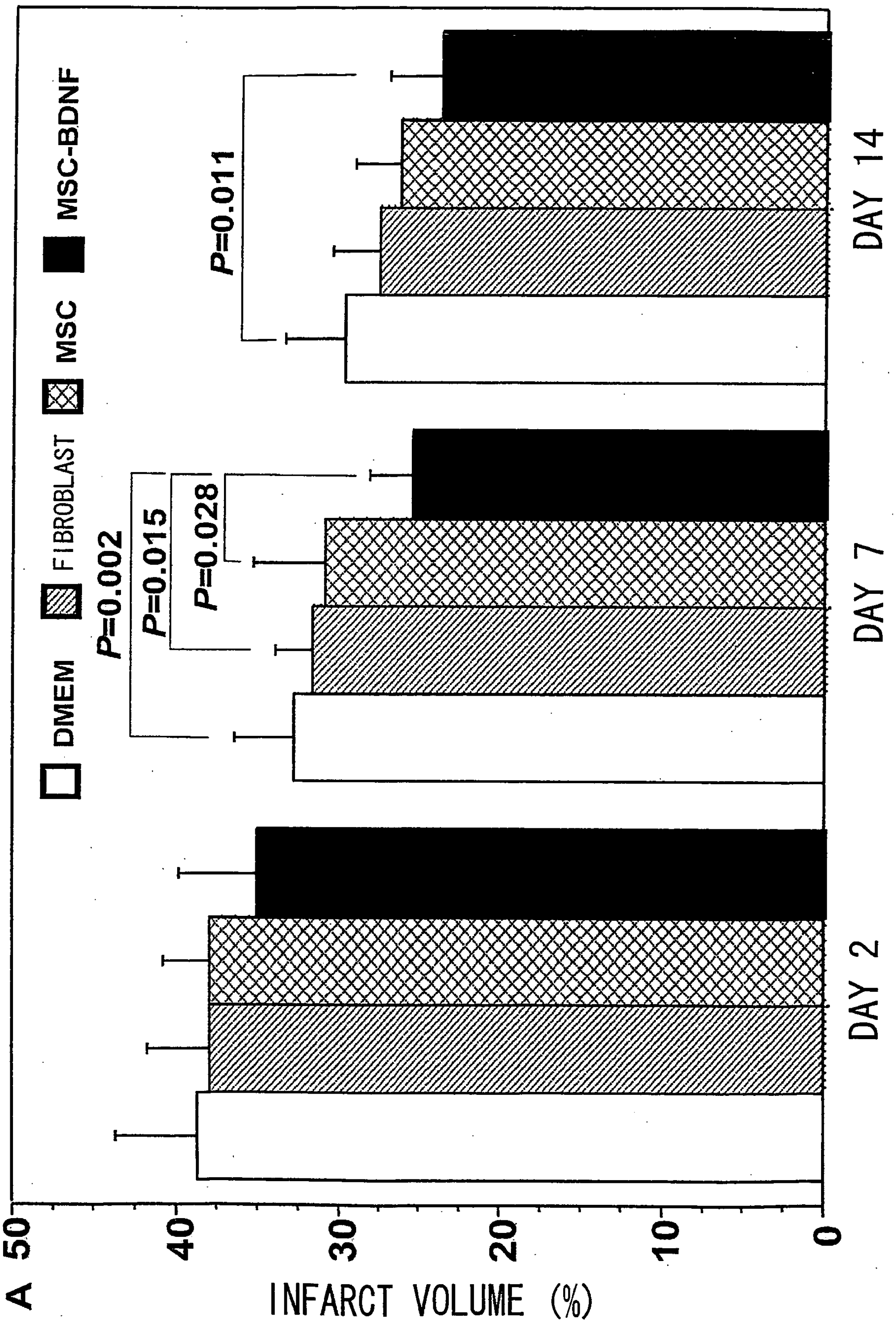
A
INFARCT VOLUME (%)
0
10
20
30
40
50
DMEM
FIBROBLAST
MSC
MSC-BDNF
DAY 2
DAY 7
DAY 14
P=0.002
P=0.015
P=0.028
P=0.011

FIG. 29B

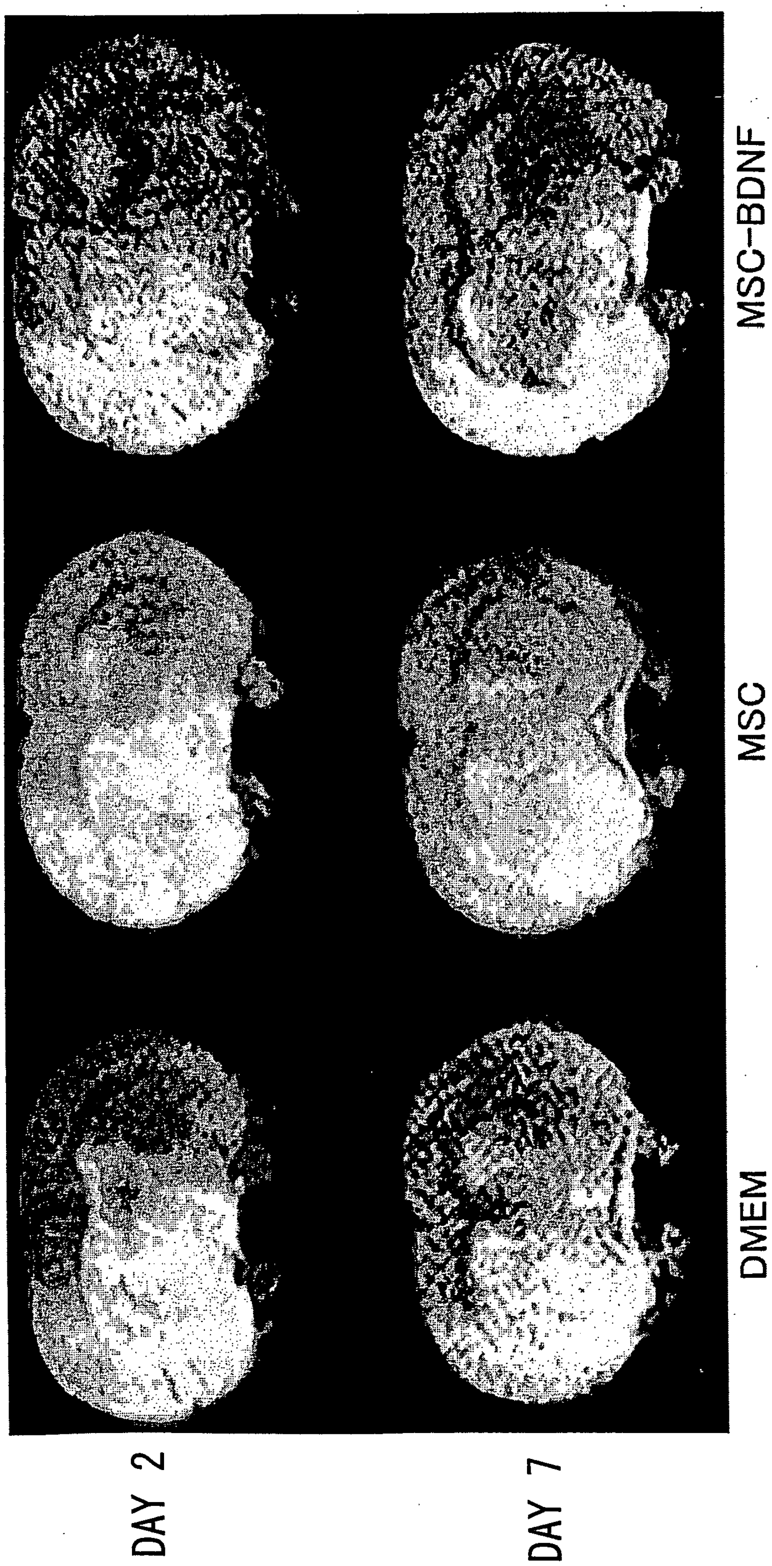

FIG. 30

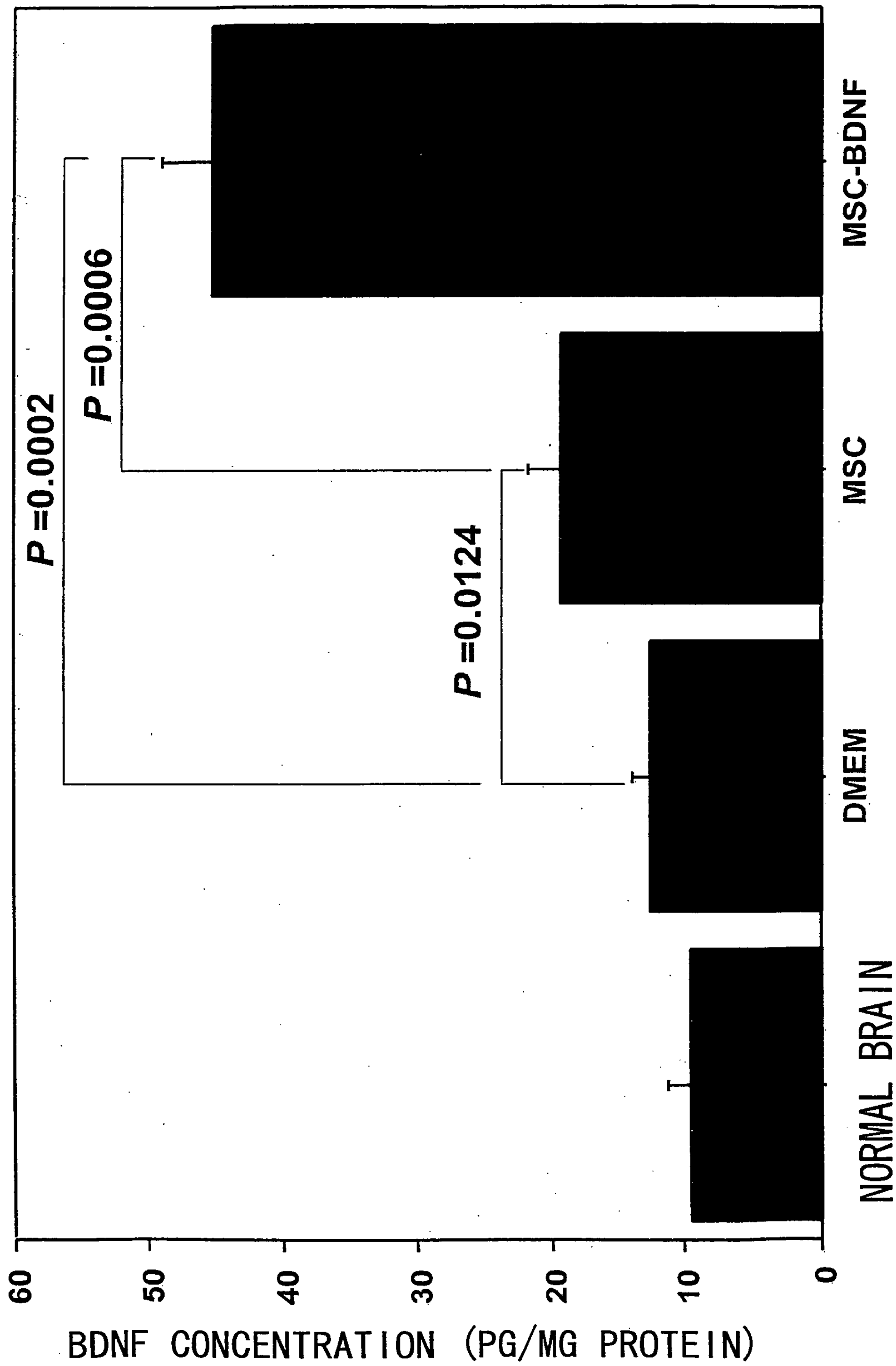
P =0.0002
P =0.0006
P =0.0124
NORMAL BRAIN
DMEM
MSC
MSC-BDNF
0
10
20
30
40
50
60
BDNF CONCENTRATION (PG/MG PROTEIN)

FIG. 31AB

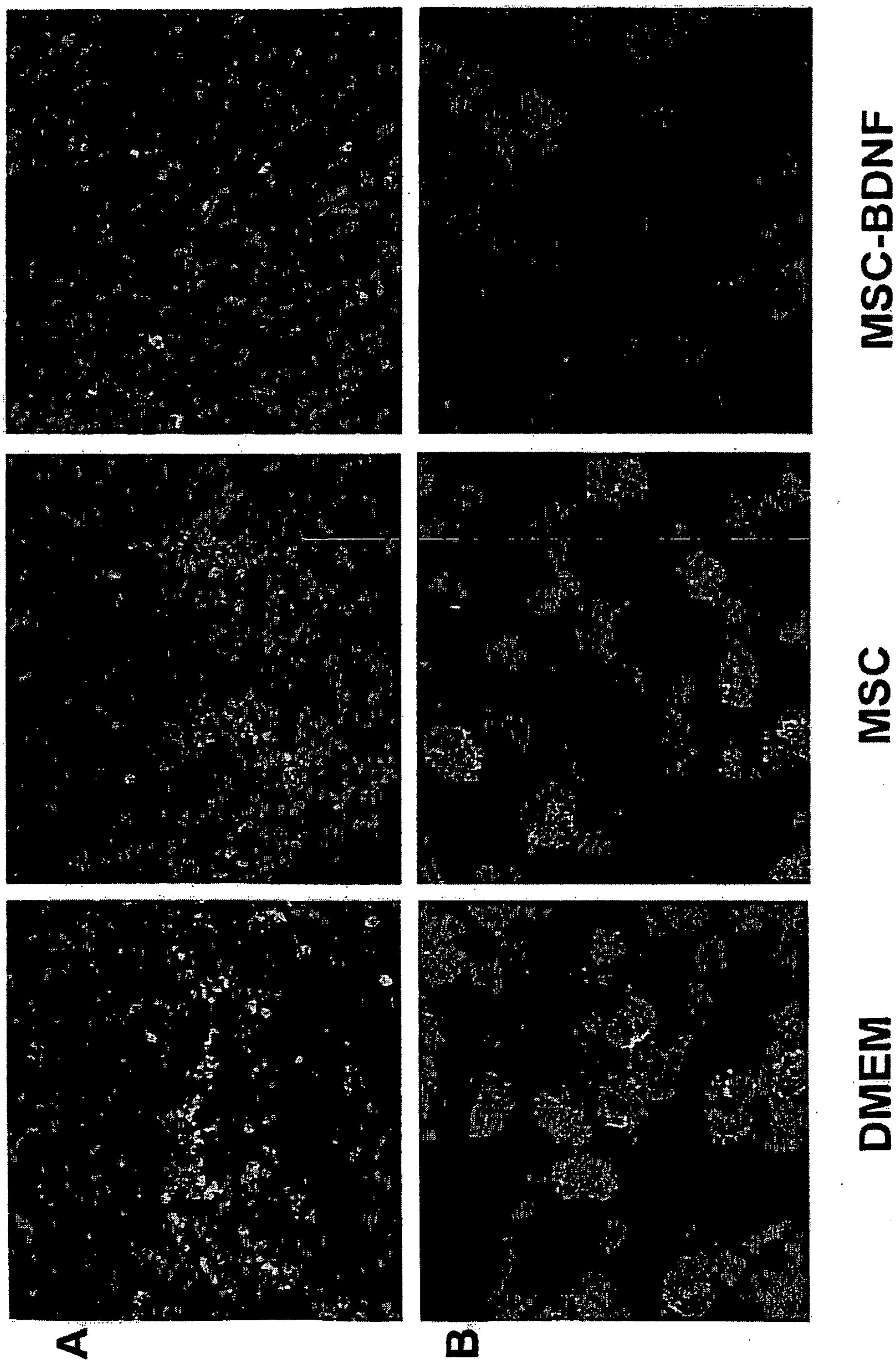

FIG. 31C

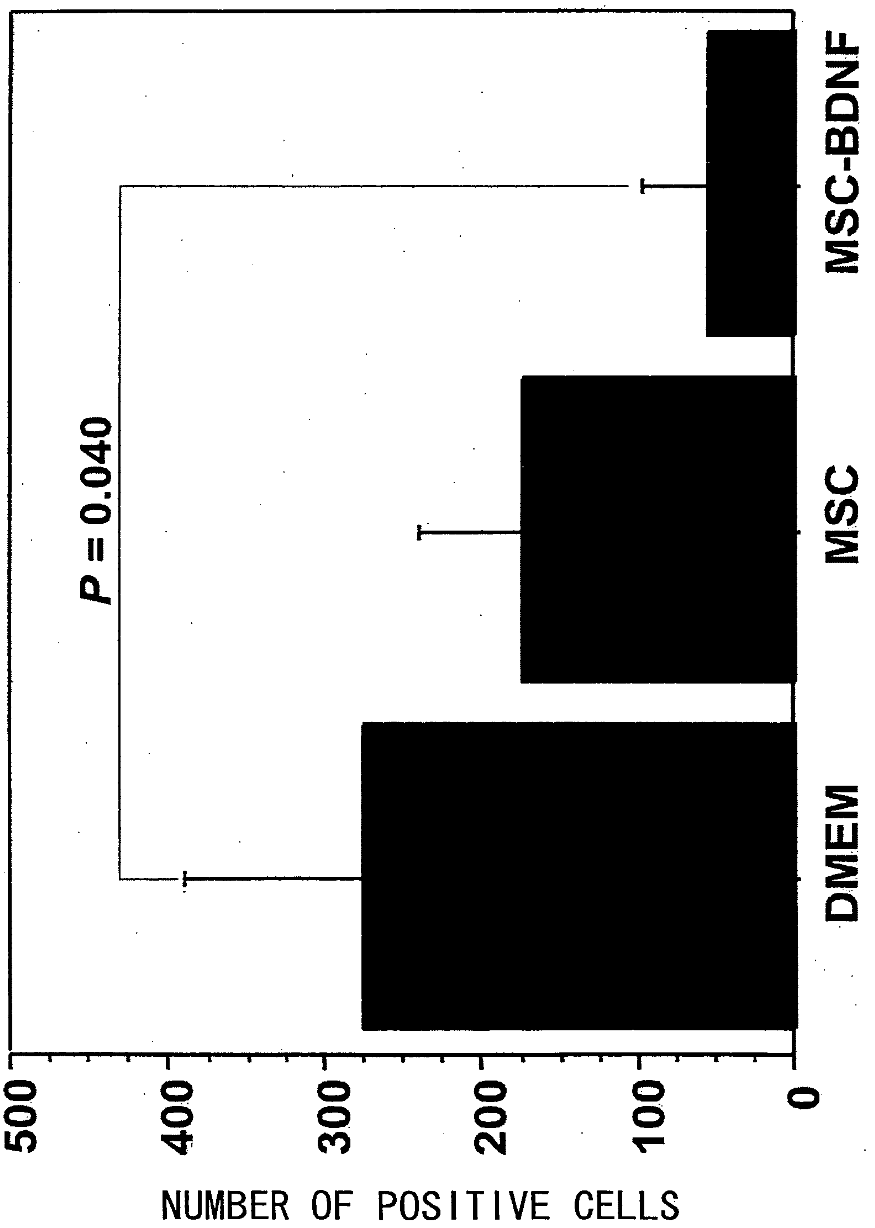
P = 0.040
DMEM
MSC
MSC-BDNF
0
100
200
300
400
500
NUMBER OF POSITIVE CELLS

FIG. 31D

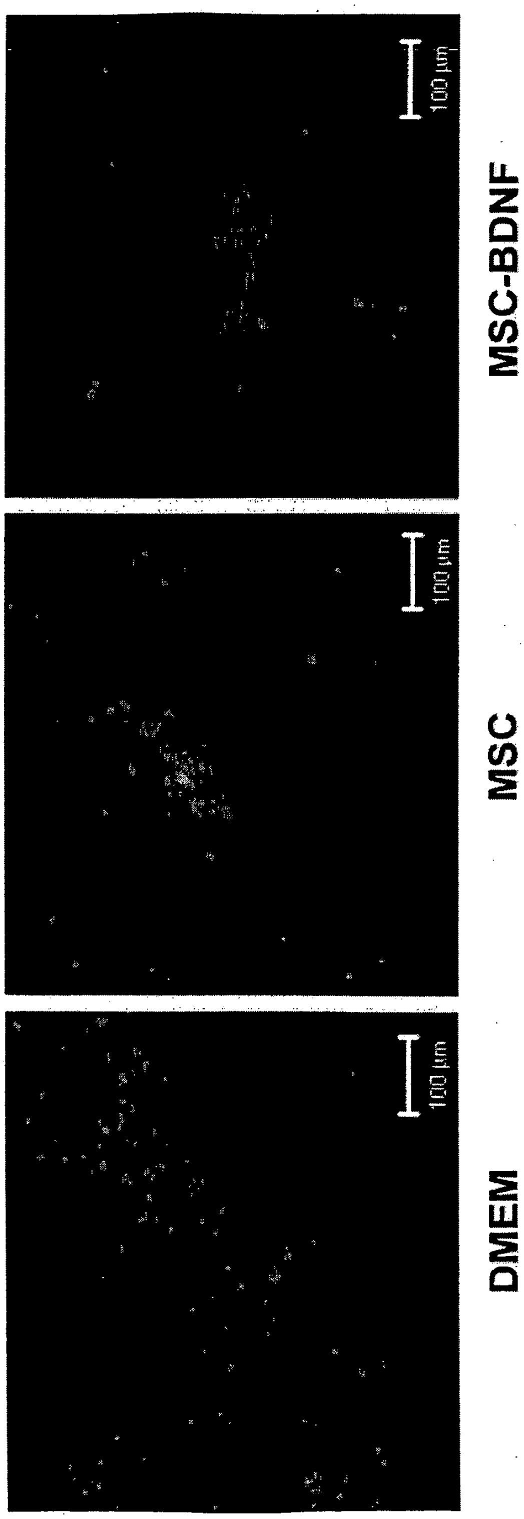

FIG. 32

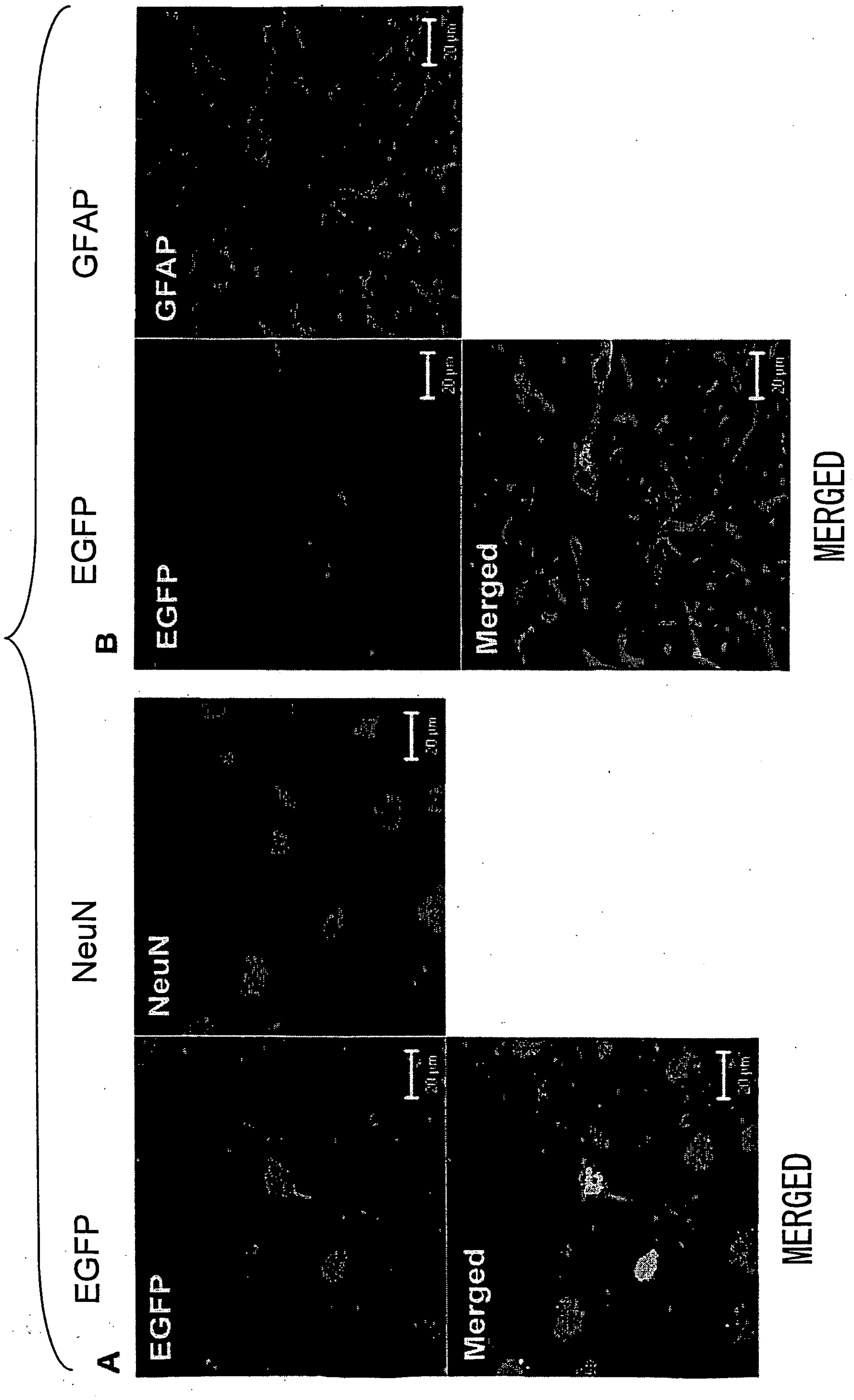
A
EGFP
NeuN
EGFP
NeuN
20 μm
Merged
MERGED
B
EGFP
GFAP
EGFP
GFAP
Merged
MERGED

# FIG. 33

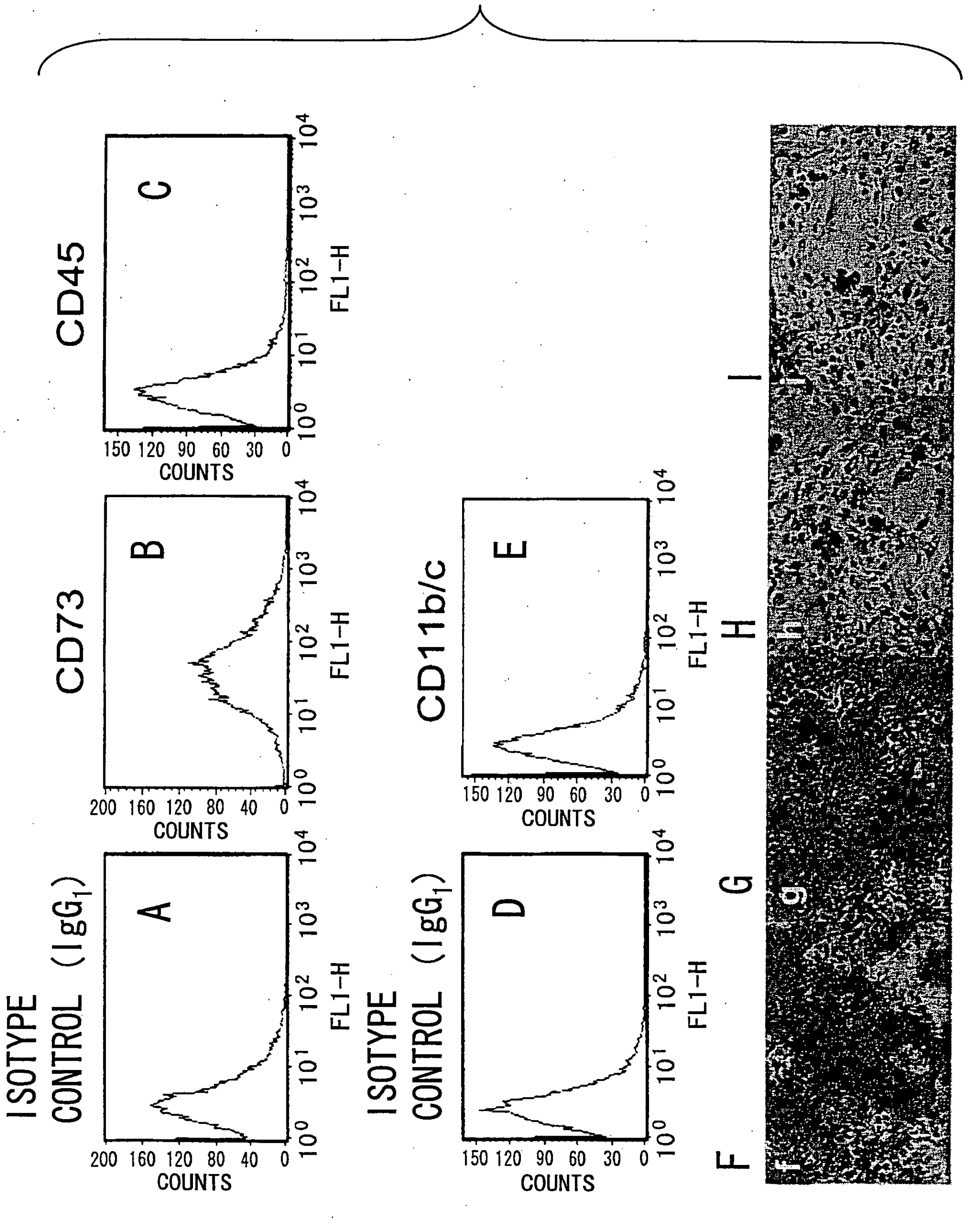
ISOTYPE CONTROL (IgG$_1$)
A
CD73
B
CD45
C
ISOTYPE CONTROL (IgG$_1$)
D
CD11b/c
E
COUNTS
FL1-H
F
G
H
I

FIG. 34

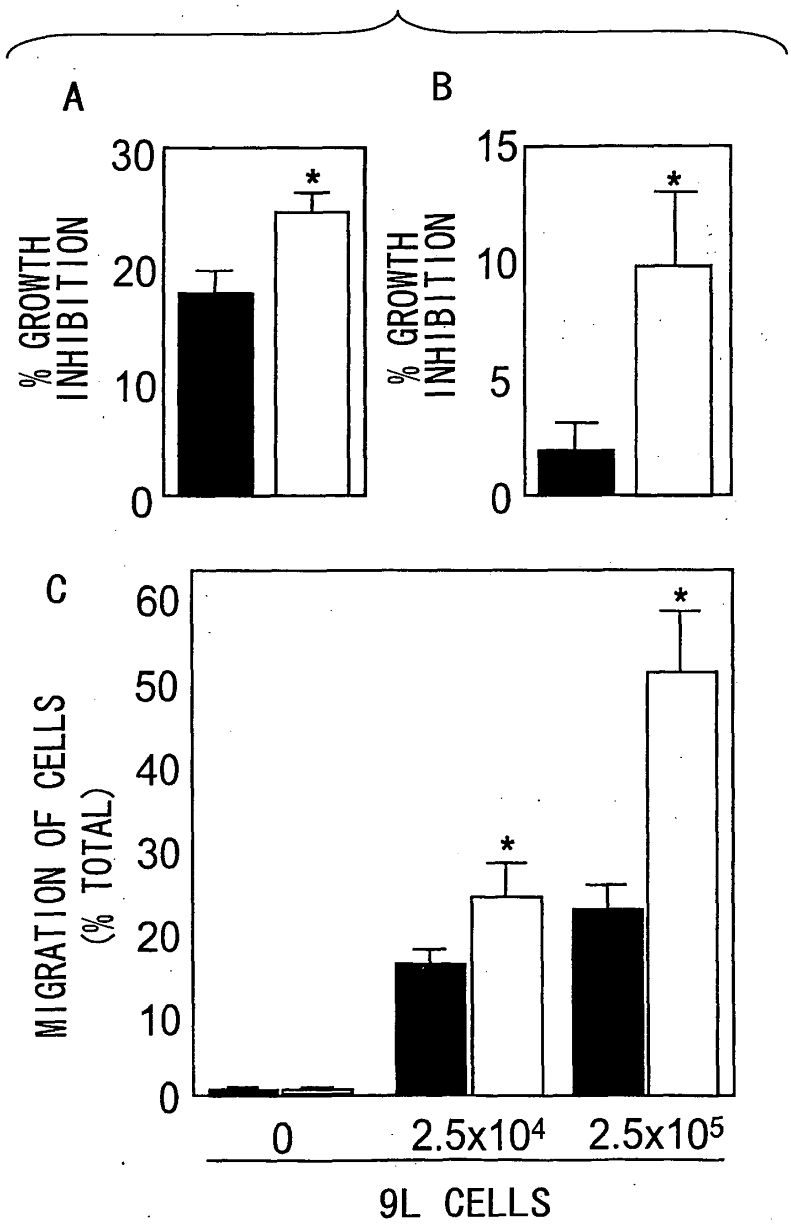
A
30
20
10
0
% GROWTH INHIBITION
*
B
15
10
5
0
% GROWTH INHIBITION
*
C
60
50
40
30
20
10
0
MIGRATION OF CELLS (% TOTAL)
*
*
0
2.5x10⁴
2.5x10⁵
9L CELLS

FIG. 35

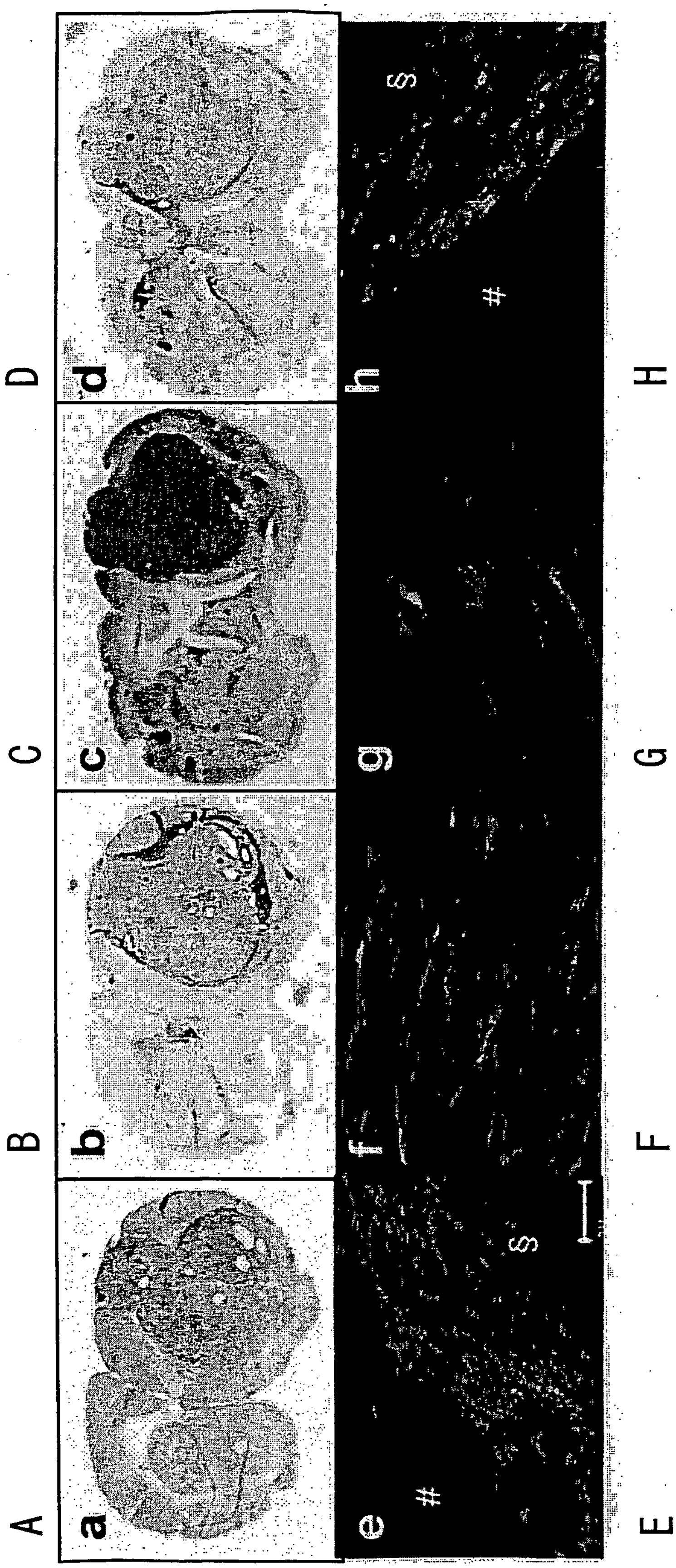

FIG. 36

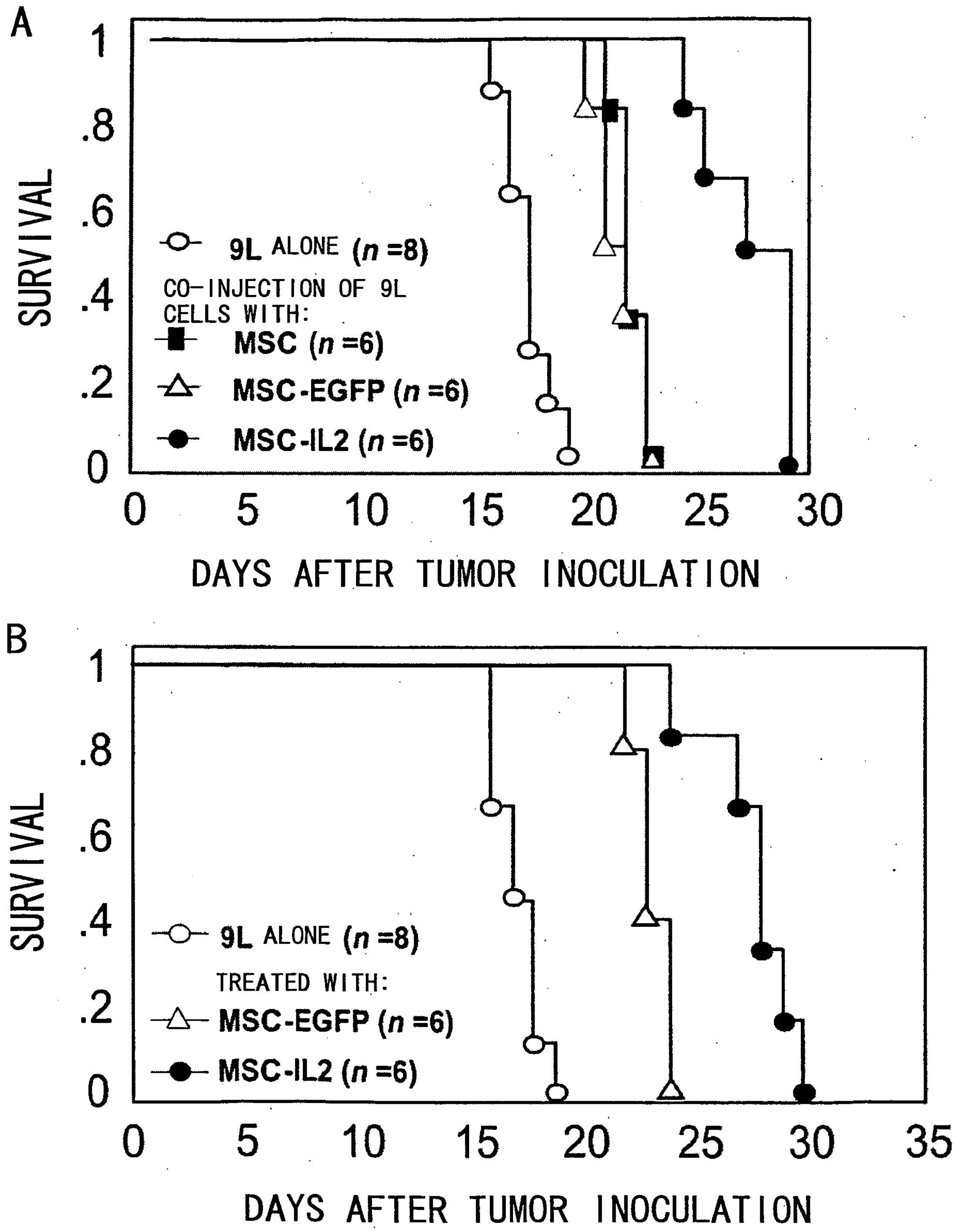
A
1
.8
.6
.4
.2
0
SURVIVAL
9L ALONE (n =8)
CO-INJECTION OF 9L CELLS WITH:
MSC (n =6)
MSC-EGFP (n =6)
MSC-IL2 (n =6)
0 5 10 15 20 25 30
DAYS AFTER TUMOR INOCULATION
B
1
.8
.6
.4
.2
0
SURVIVAL
9L ALONE (n =8)
TREATED WITH:
MSC-EGFP (n =6)
MSC-IL2 (n =6)
0 5 10 15 20 25 30 35
DAYS AFTER TUMOR INOCULATION

FIG. 37

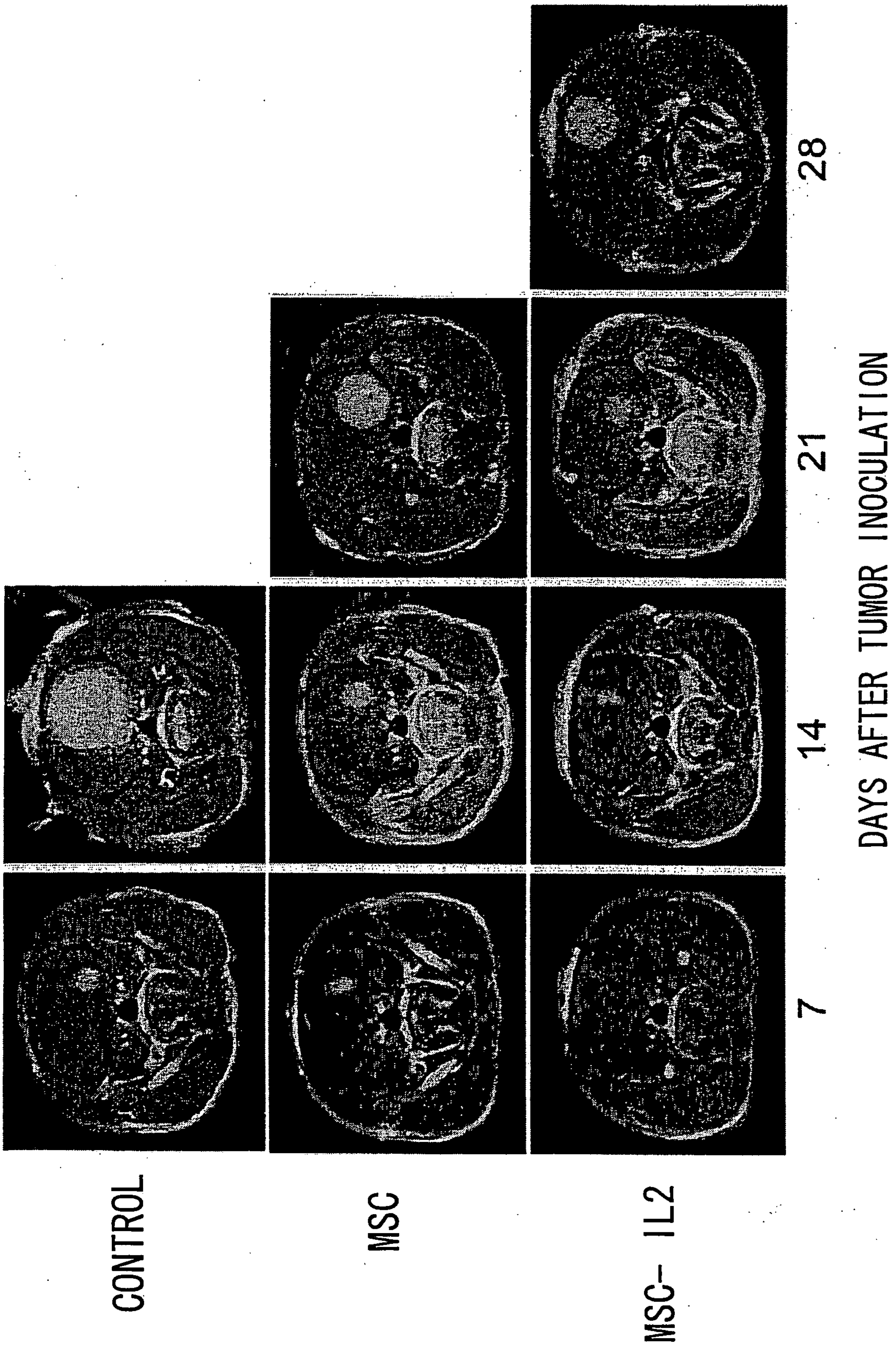

FIG. 38

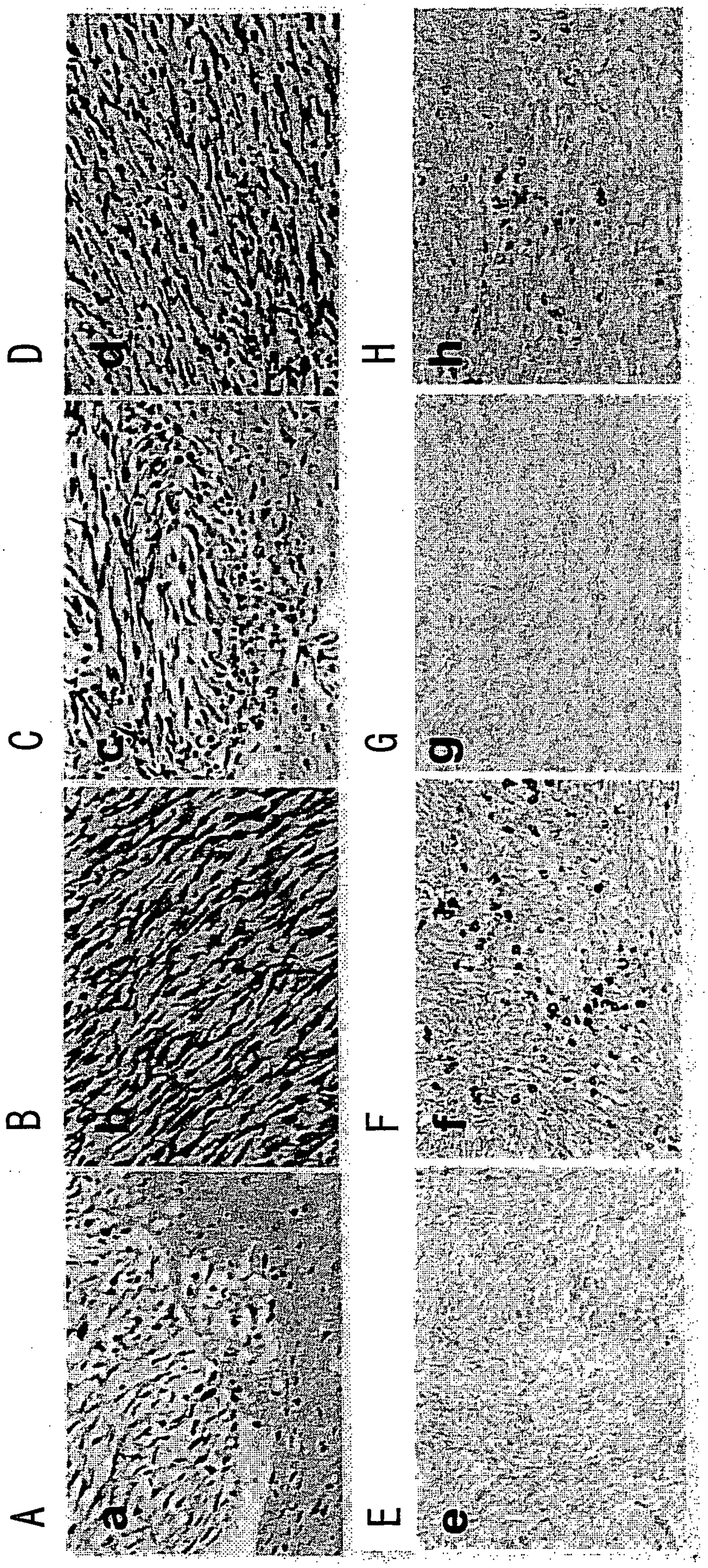

FIG. 39

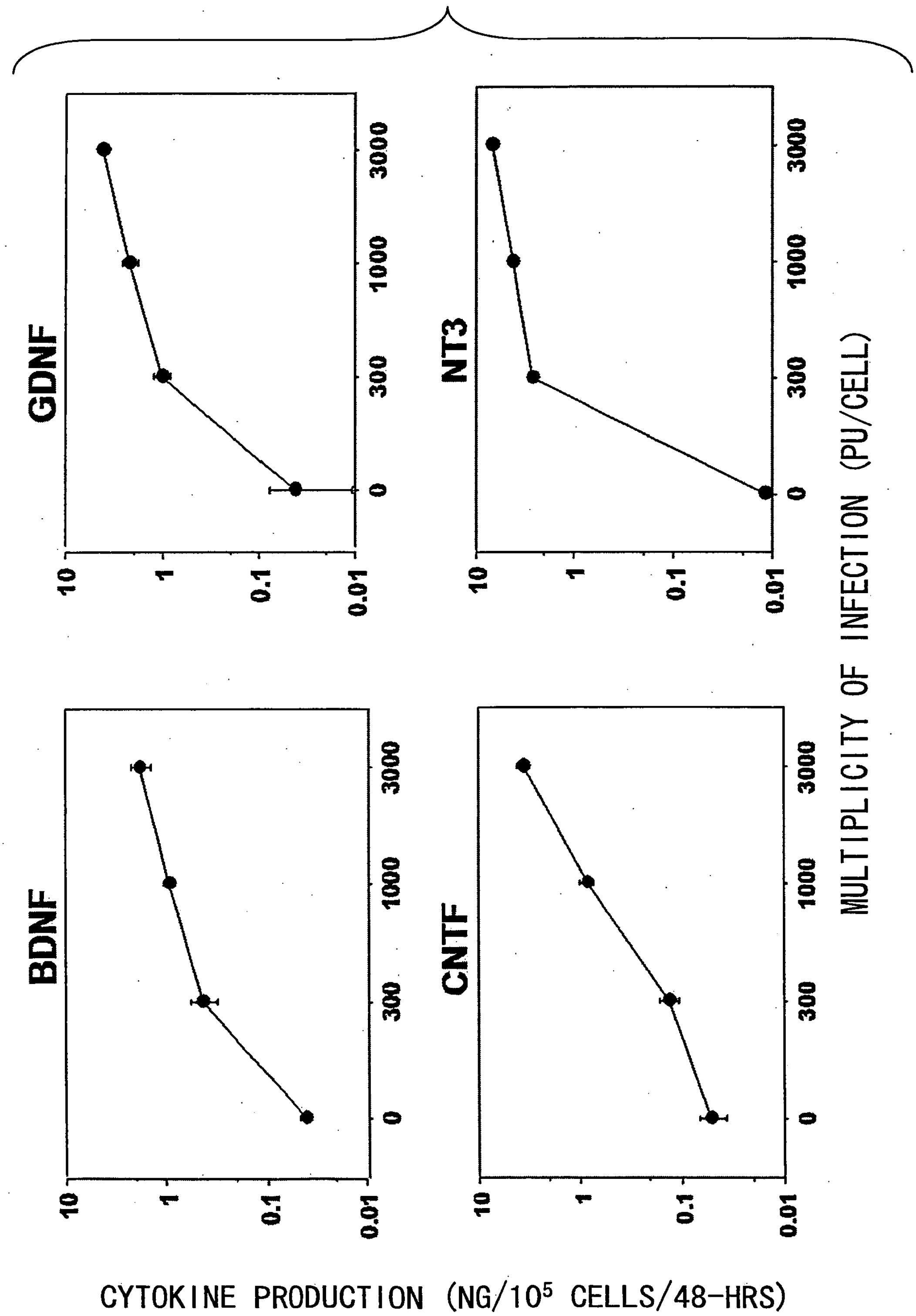
GDNF
NT3
BDNF
CNTF
10
1
0.1
0.01
0
300
1000
3000
MULTIPLICITY OF INFECTION (PU/CELL)
CYTOKINE PRODUCTION (NG/10$^5$ CELLS/48-HRS)

FIG. 40

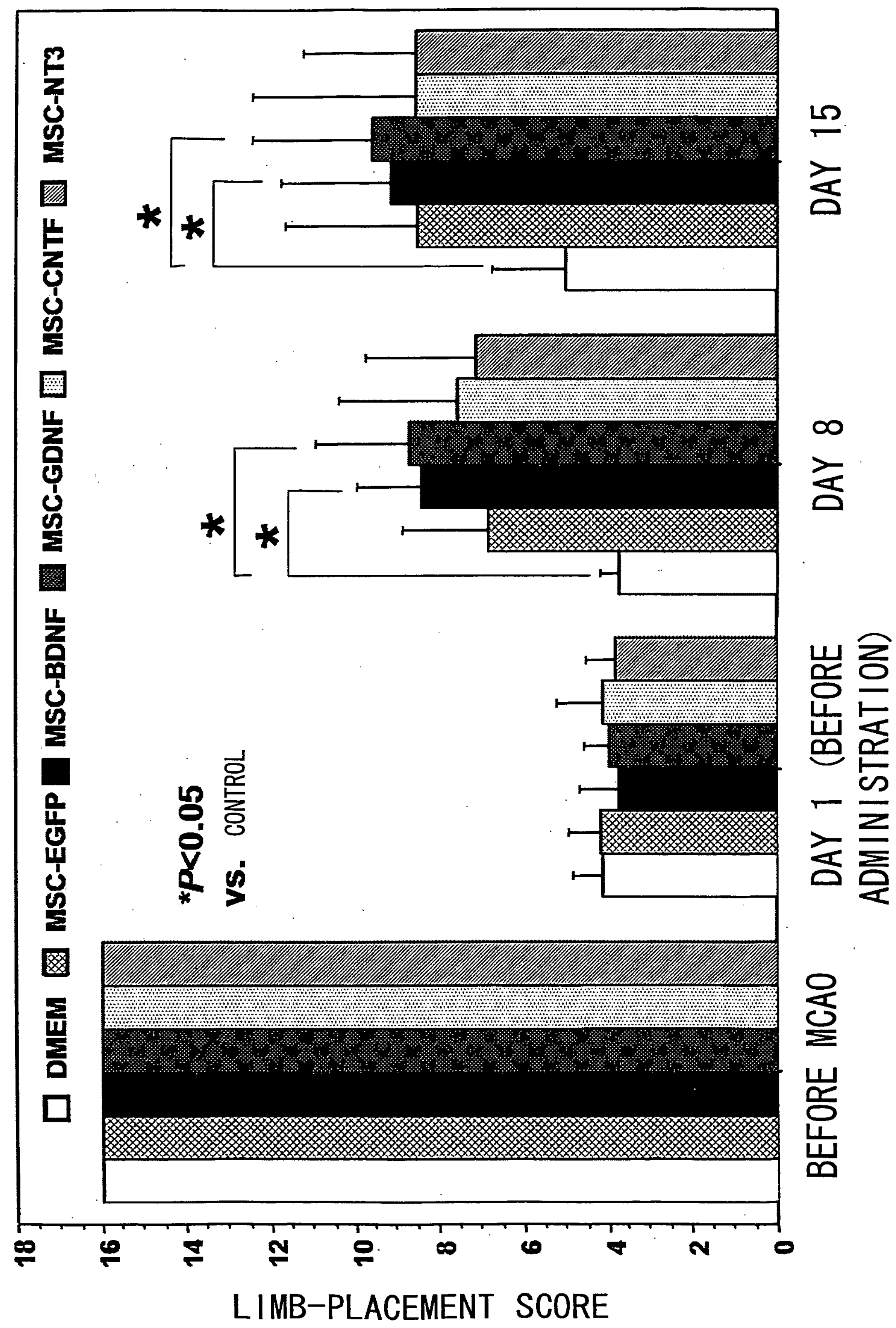
DMEM
MSC-EGFP
MSC-BDNF
MSC-GDNF
MSC-CNTF
MSC-NT3
*P<0.05
vs. CONTROL
BEFORE MCAO
DAY 1 (BEFORE ADMINISTRATION)
DAY 8
DAY 15
LIMB-PLACEMENT SCORE
0
2
4
6
8
10
12
14
16
18

FIG. 41

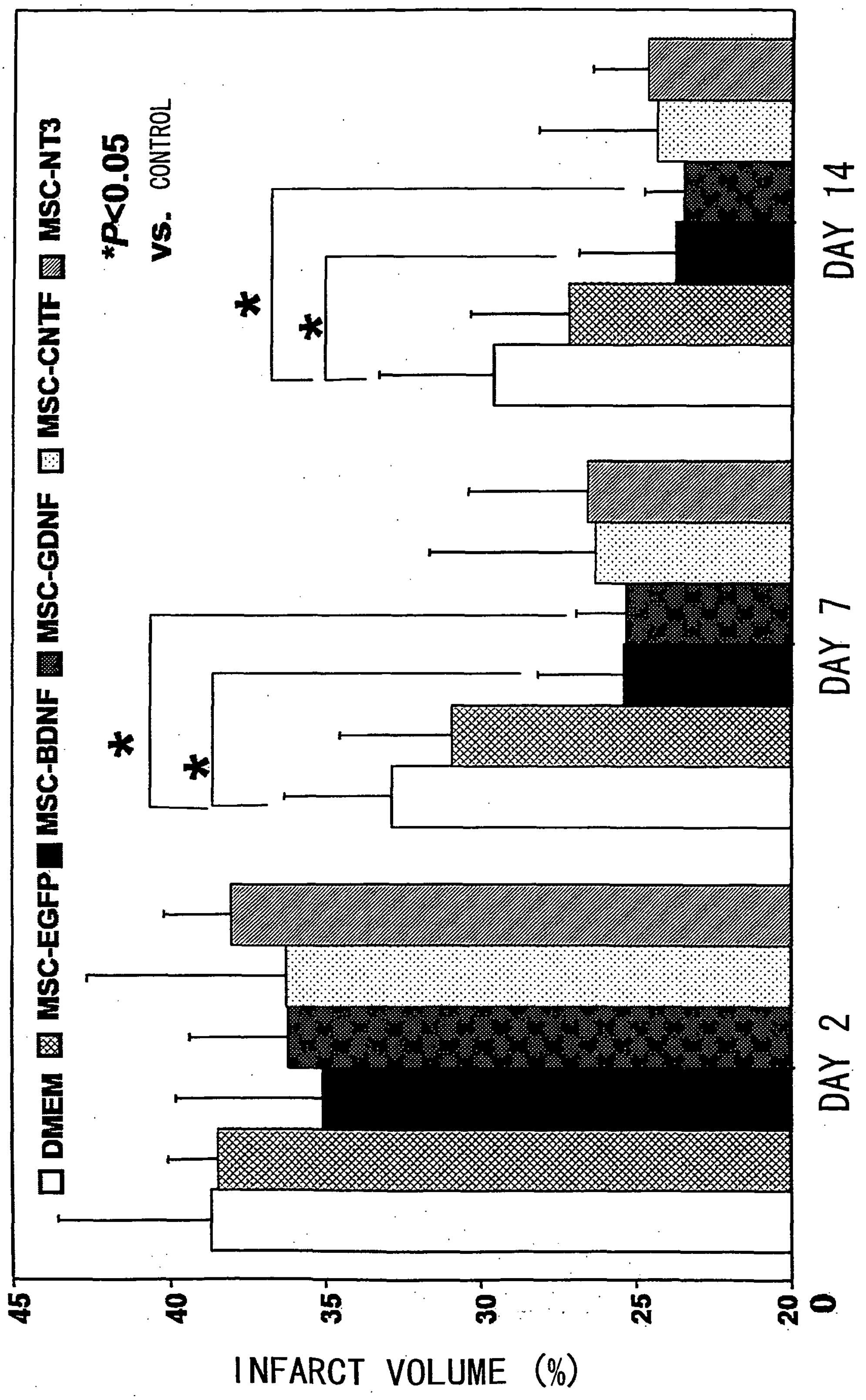
DMEM
MSC-EGFP
MSC-BDNF
MSC-GDNF
MSC-CNTF
MSC-NT3
*P<0.05
Vs. CONTROL
*
DAY 2
DAY 7
DAY 14
45
40
35
30
25
20
0
INFARCT VOLUME (%)

FIG. 42

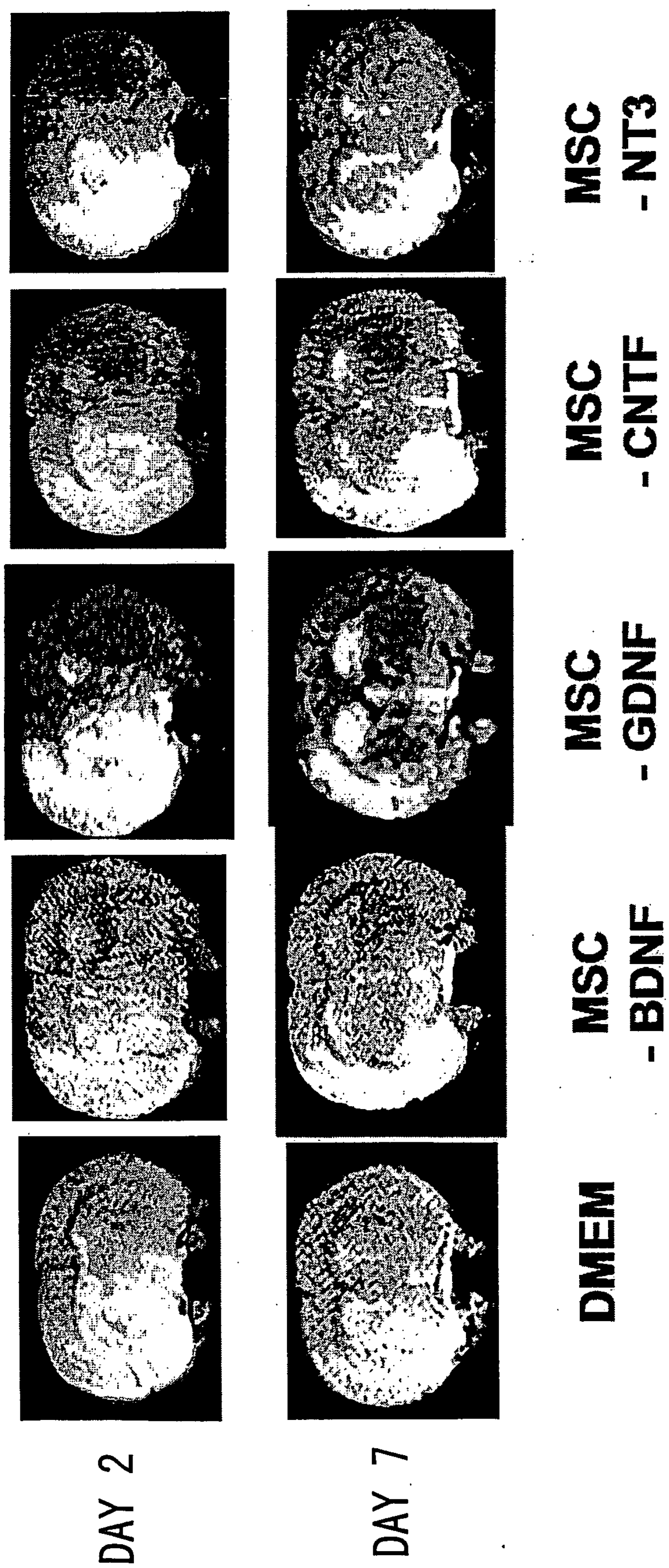

FIG. 43

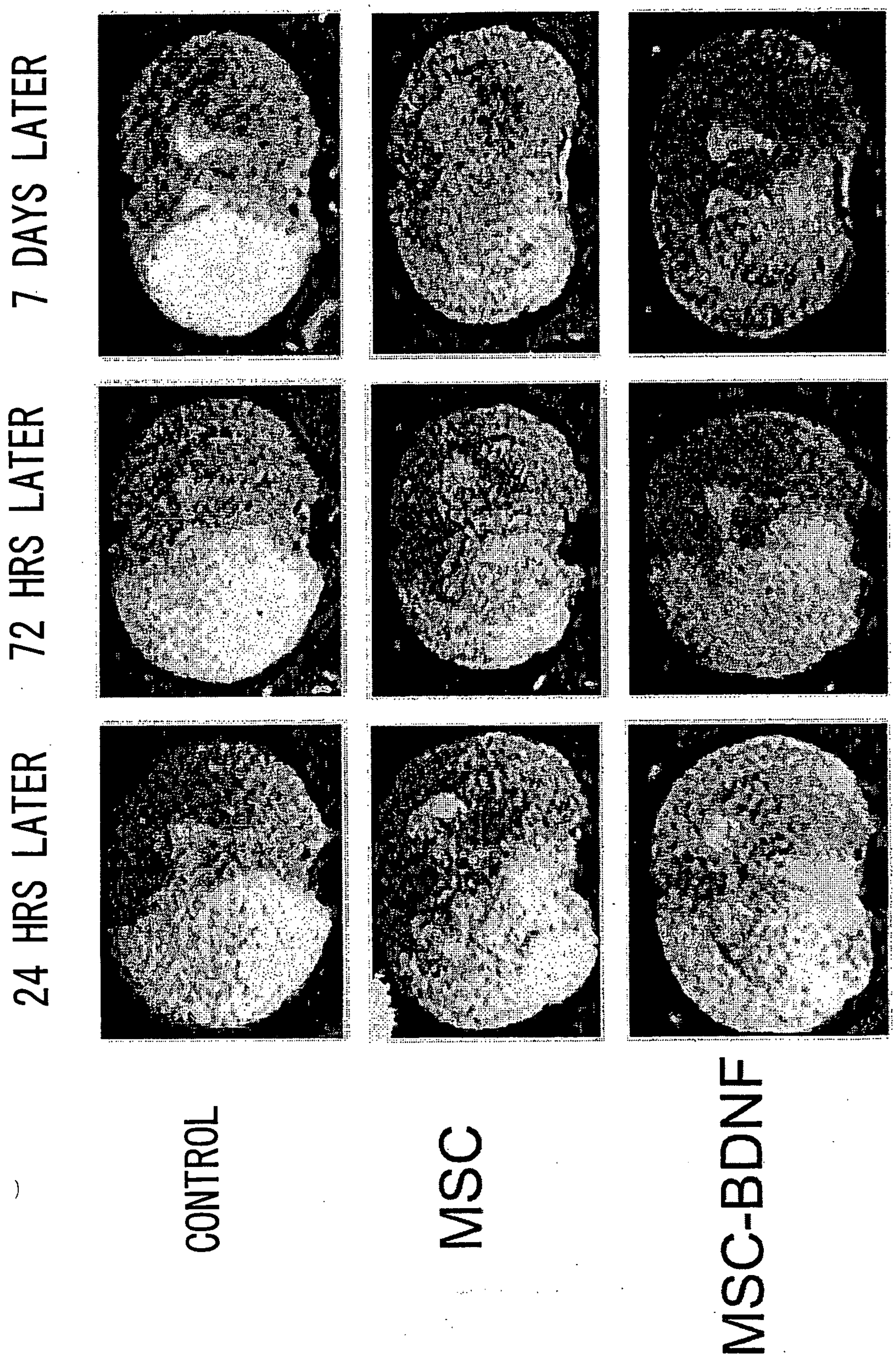

FIG. 44

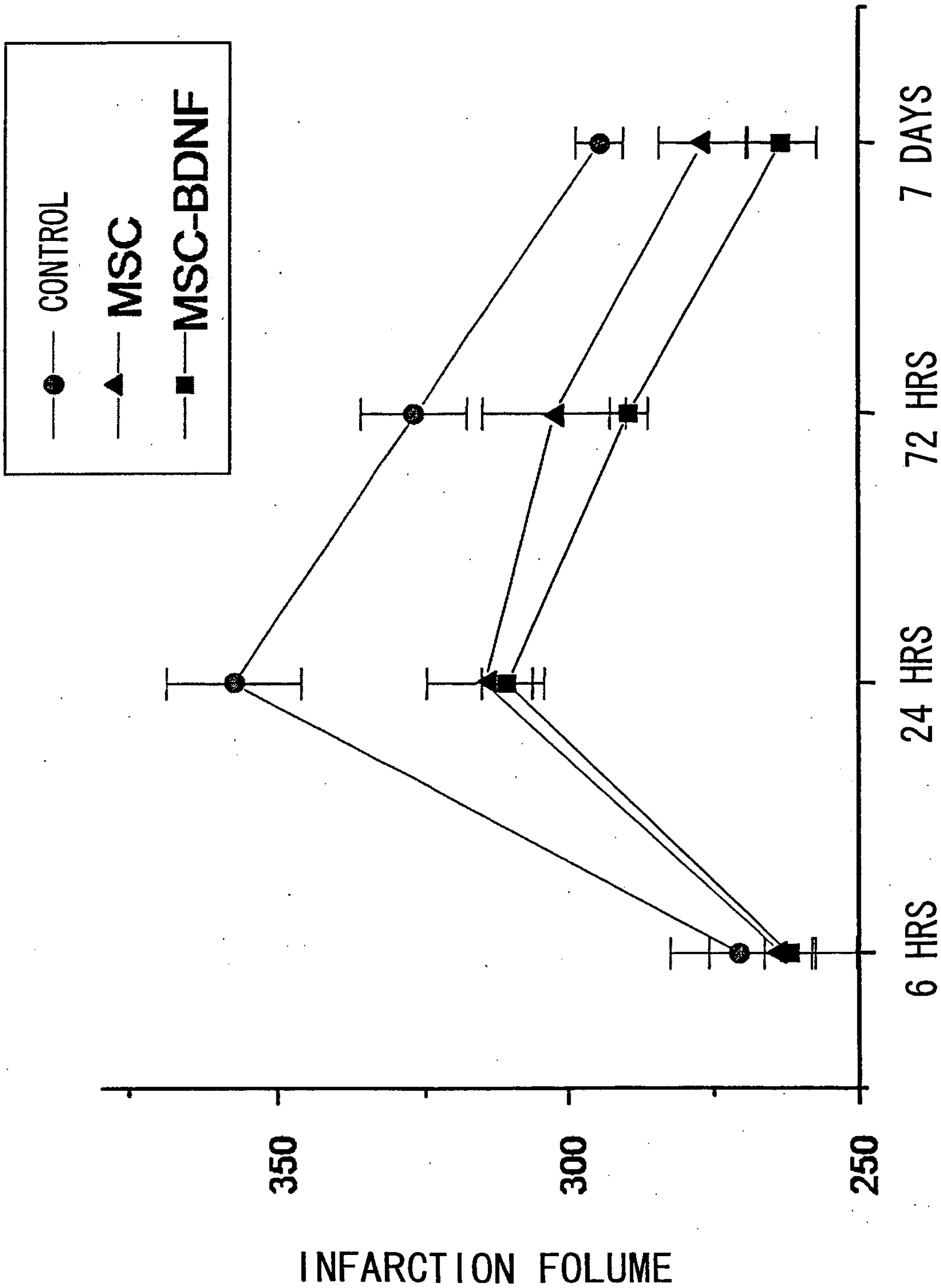
CONTROL
MSC
MSC-BDNF
6 HRS
24 HRS
72 HRS
7 DAYS
350
300
250
INFARCTION FOLUME

FIG. 45

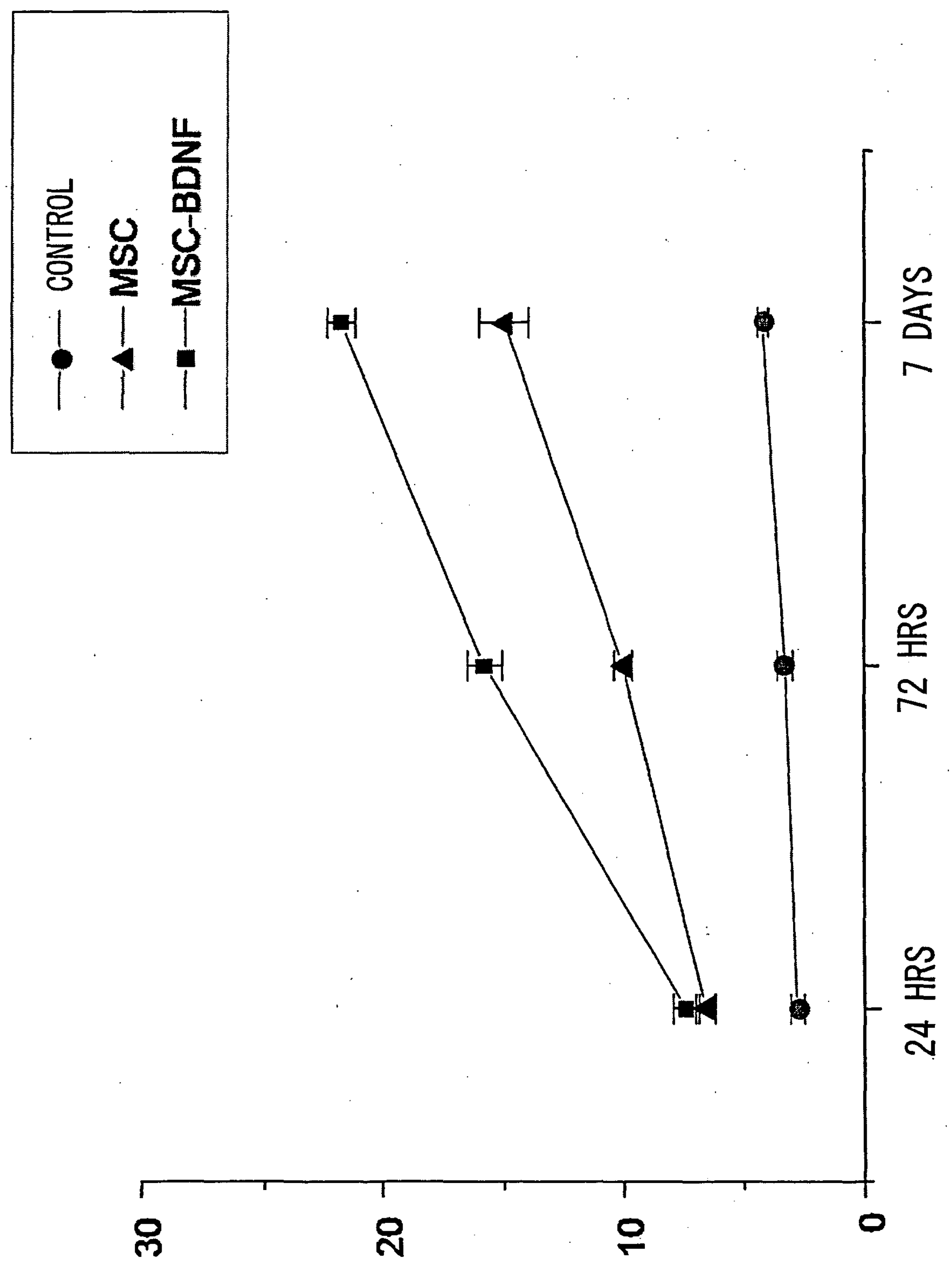

FIG. 46

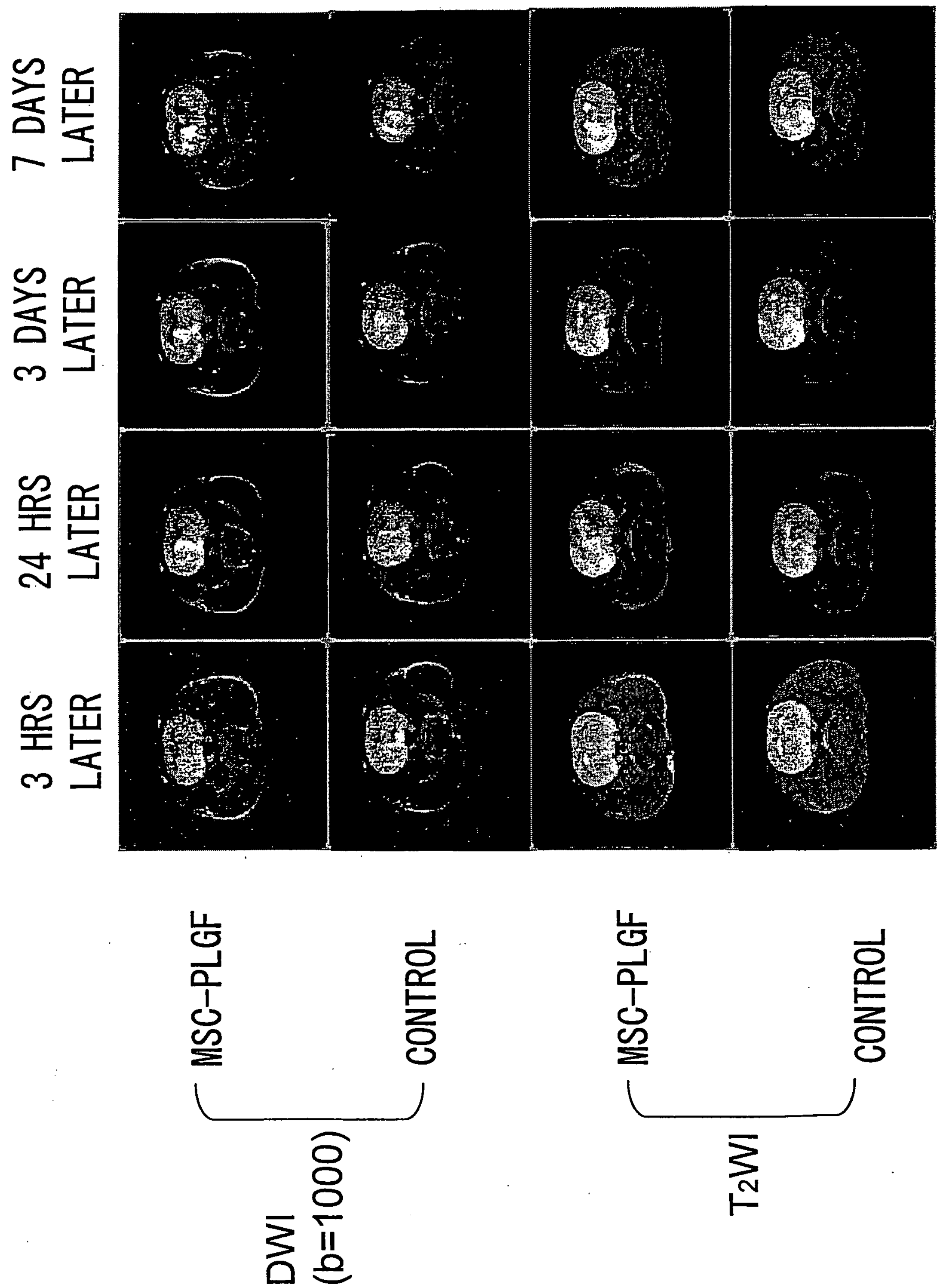
3 HRS LATER
24 HRS LATER
3 DAYS LATER
7 DAYS LATER
MSC-PLGF
CONTROL
DWI (b=1000)
MSC-PLGF
CONTROL
T2WI

FIG. 47

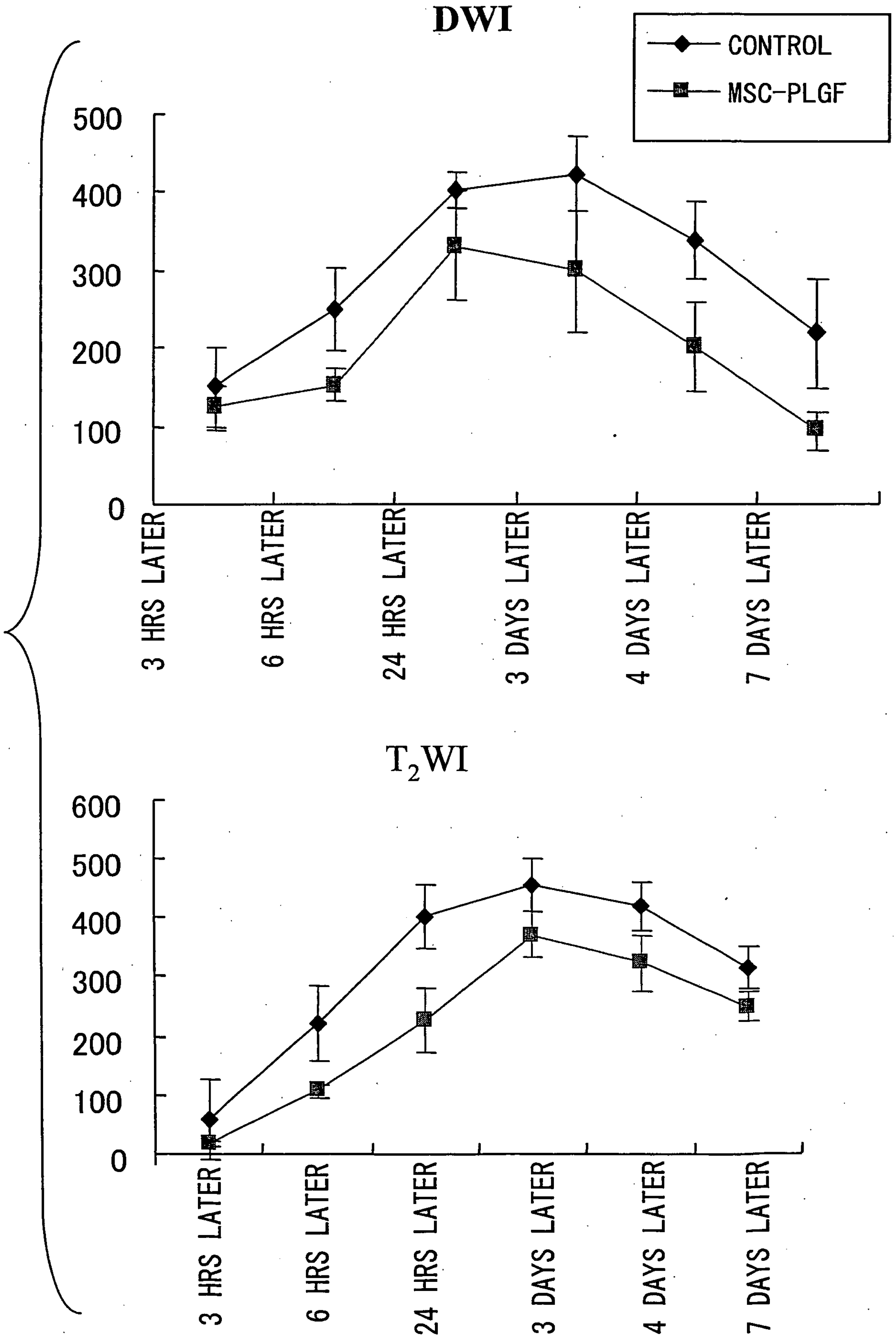
DWI
CONTROL
MSC-PLGF
500
400
300
200
100
0
3 HRS LATER
6 HRS LATER
24 HRS LATER
3 DAYS LATER
4 DAYS LATER
7 DAYS LATER
$T_2WI$
600
500
400
300
200
100
0
3 HRS LATER
6 HRS LATER
24 HRS LATER
3 DAYS LATER
4 DAYS LATER
7 DAYS LATER

FIG. 48

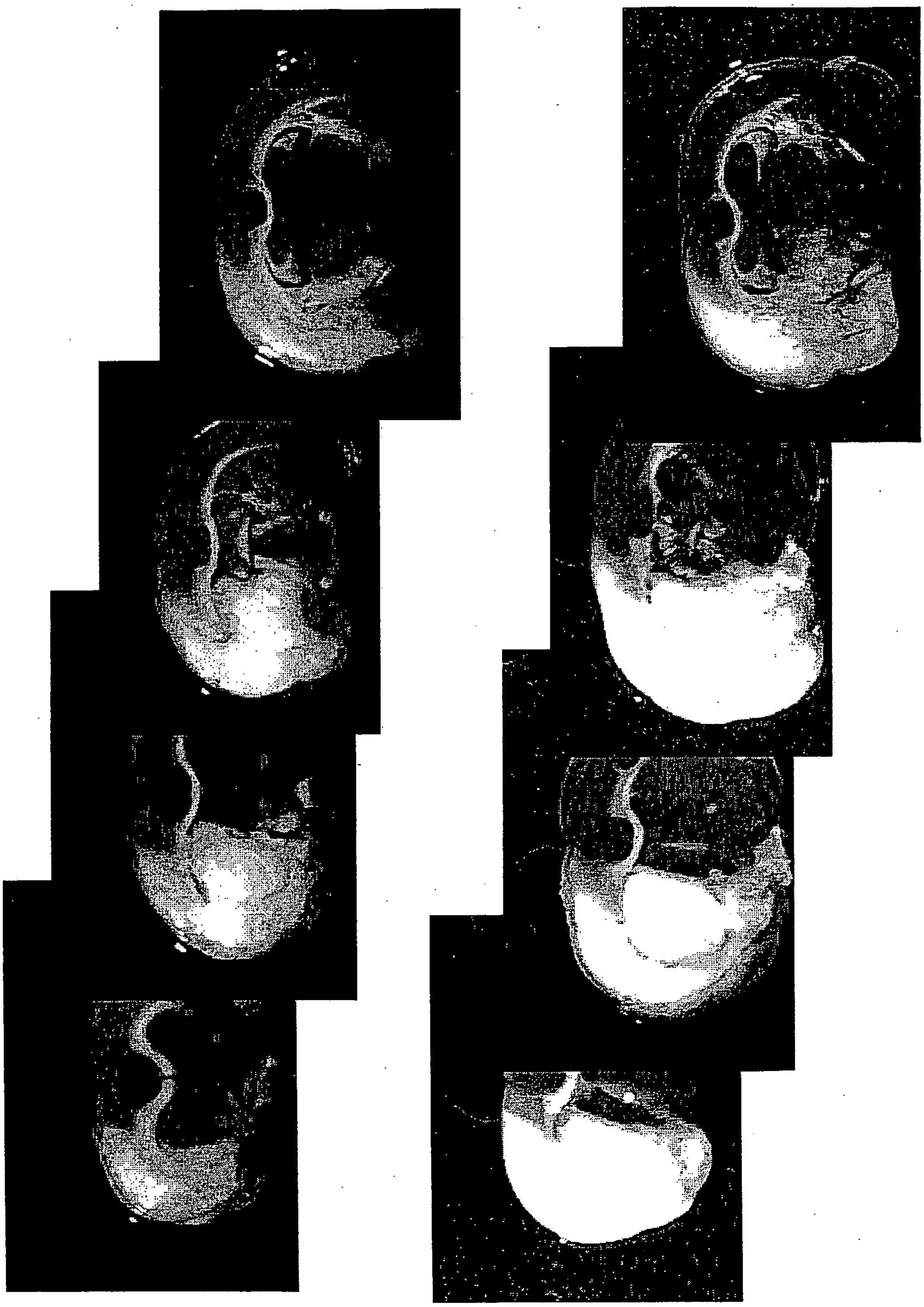

MSC-PLGF

CONTROL

FIG. 49

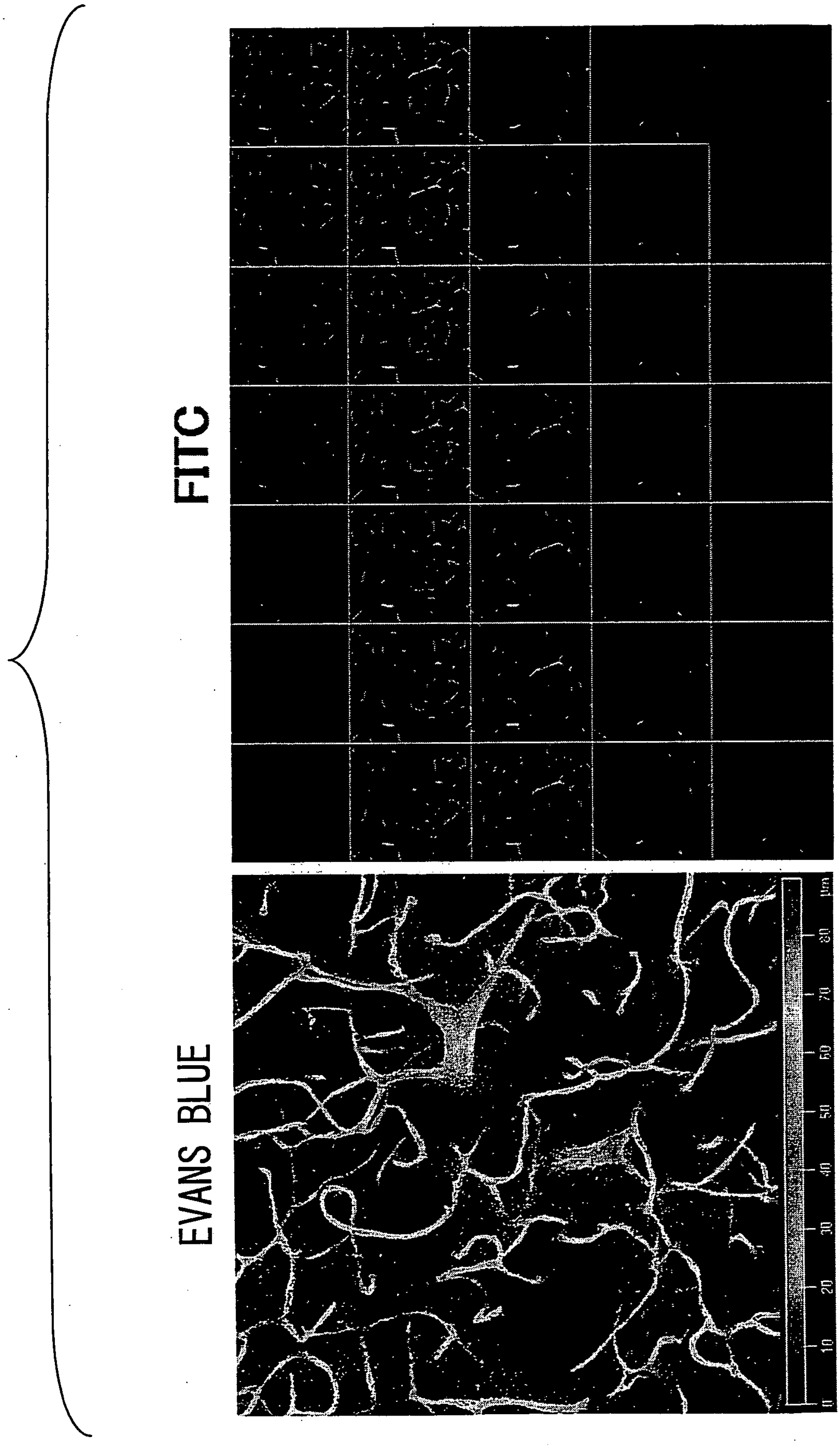

FIG. 50

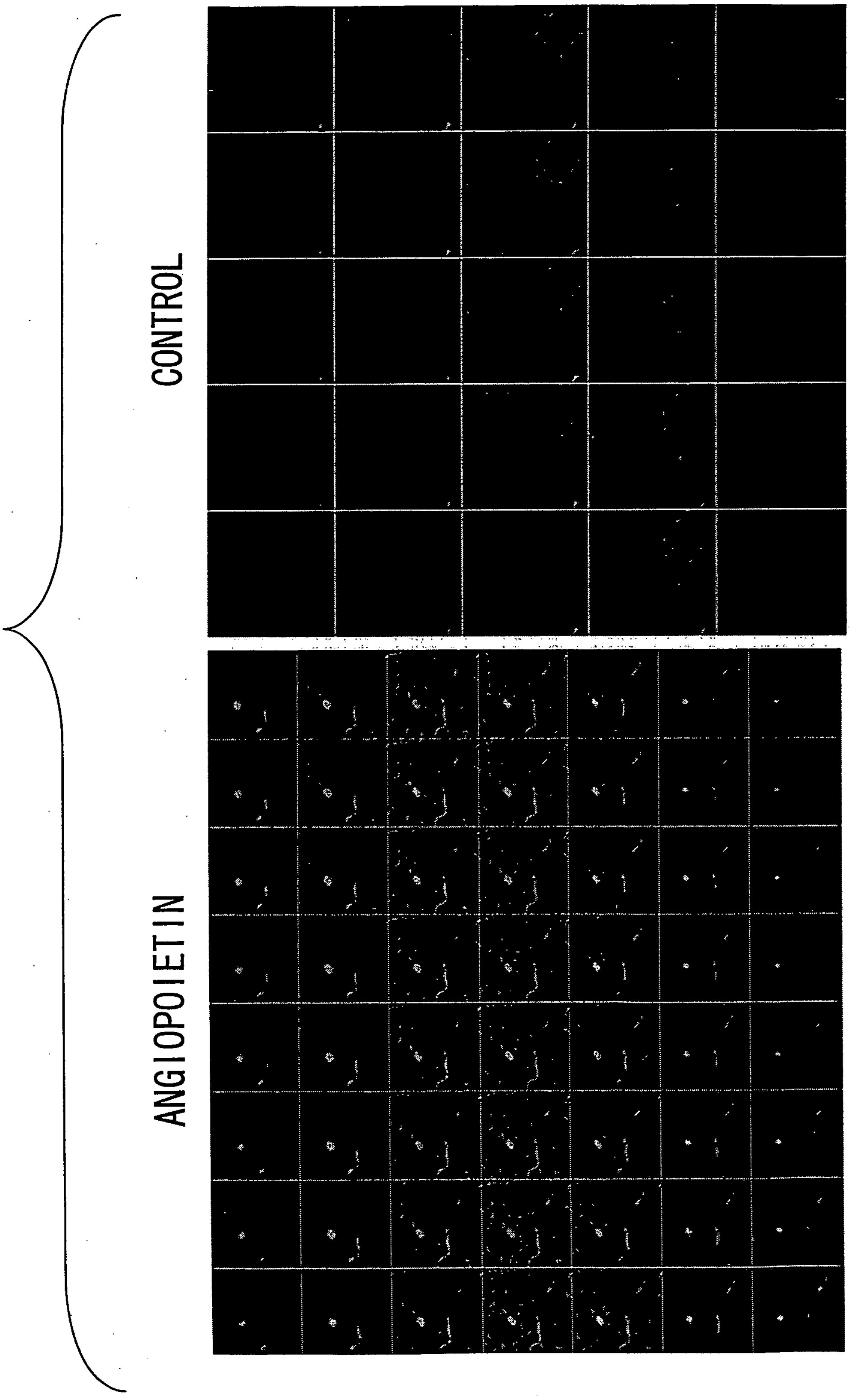

FIG. 51

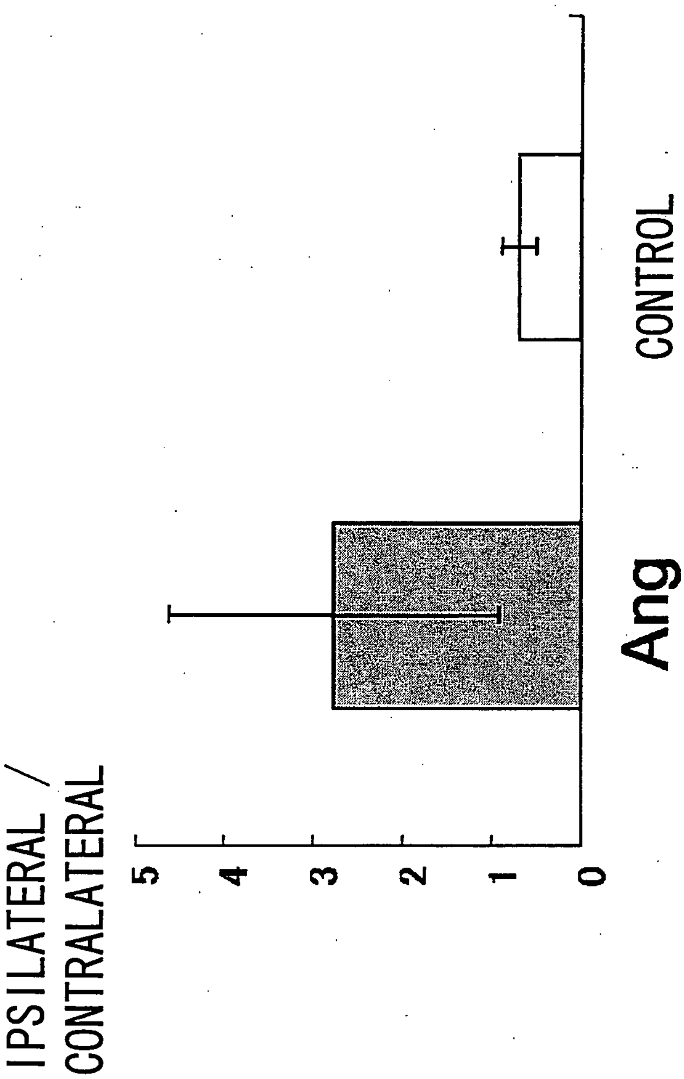

FIG. 52

CONTROL

ANGIOPOIETIN

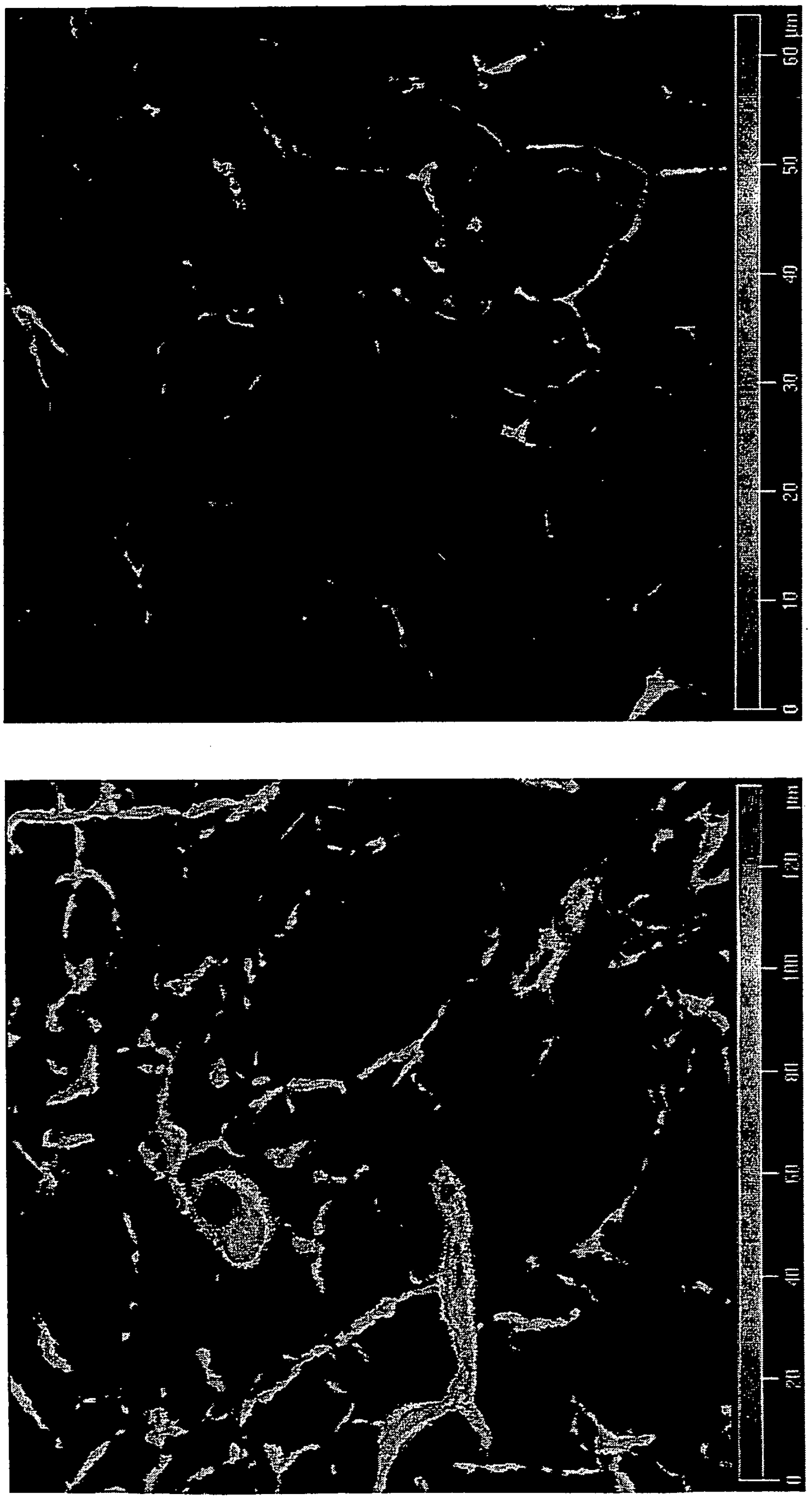

FIG. 53

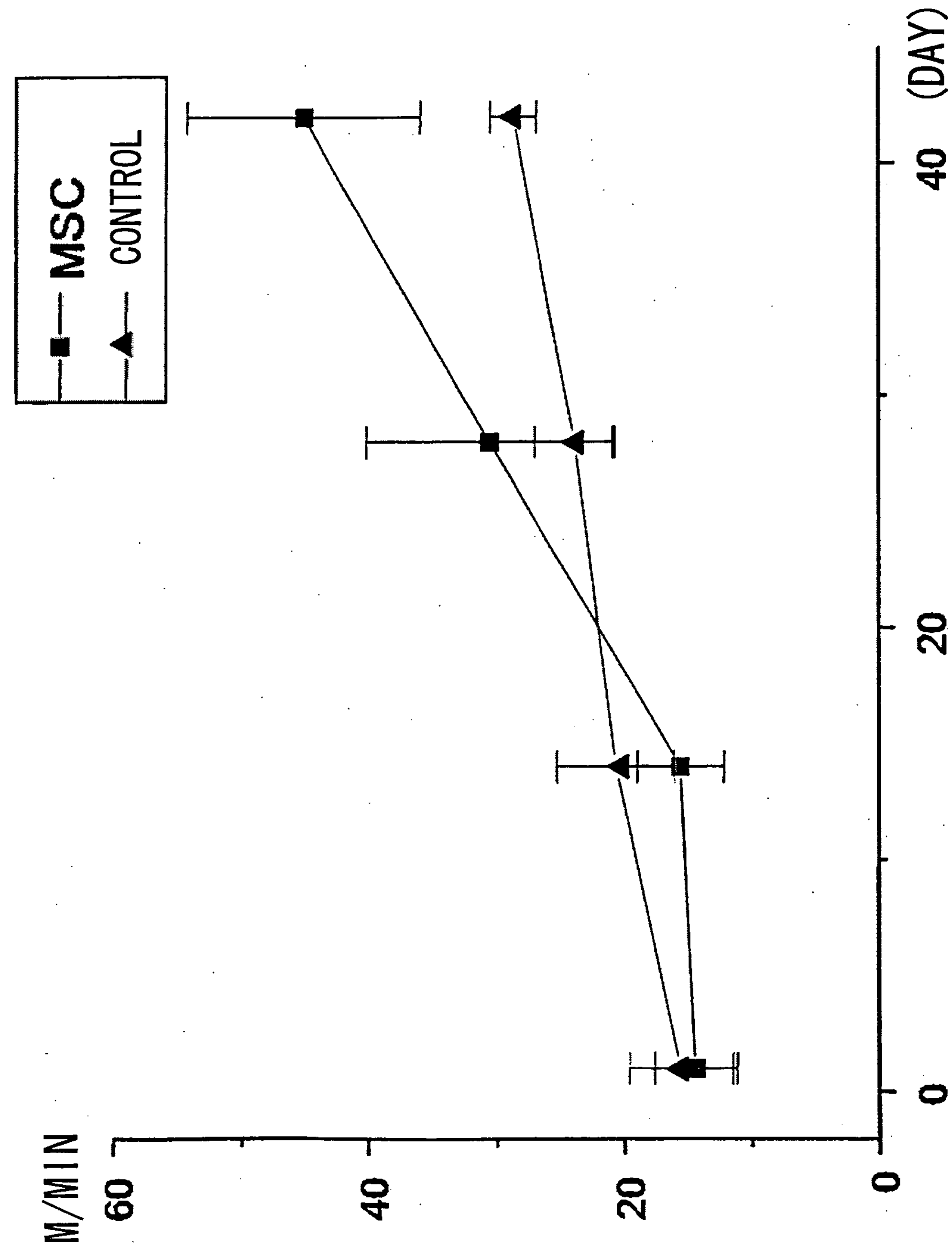

**INTERNATIONAL SEARCH REPORT**

International application No.
PCT/JP2004/009386

A. CLASSIFICATION OF SUBJECT MATTER
Int.Cl[7] A61K35/28, A61K35/14, A61K35/34, A61P25/00, A61P25/28

According to International Patent Classification (IPC) or to both national classification and IPC

B. FIELDS SEARCHED

Minimum documentation searched (classification system followed by classification symbols)
Int.Cl[7] A61K35/28, A61K35/14, A61K35/34, A61P25/00, A61P25/28

Documentation searched other than minimum documentation to the extent that such documents are included in the fields searched

Electronic data base consulted during the international search (name of data base and, where practicable, search terms used)
CAP(STN), BIOSIS(STN), MEDLINE(STN), EMBASE(STN), WPI, JOIS

C. DOCUMENTS CONSIDERED TO BE RELEVANT

| Category* | Citation of document, with indication, where appropriate, of the relevant passages | Relevant to claim No. |
|---|---|---|
| X<br>Y | DATABASE BIOSIS ON STN, (LAST UPDATED ON STN: 19 June, 2003 (19.06.03))abstract no.200300283245 & IIHOSHI, S. et al., 'Intravenous administration of autologous bone marrow cells repairs the ischemic lesions in the rat middle cerebral artery occlusion model.', Society for Neuroscience Abstract Viewer and Itinerary Planner, (2002) Vol.2002, abstract no.237.8., http://sfn.scholarone.com | 1-5,7,8<br>6-8 |
| X<br>Y | JP 2002-544234 A (HENRY FORD HEALTH SYSTEM), 24 December, 2002 (24.12.02), All references; Claims; Par. Nos. [0022] to [0026] & AU 2000/51300 B & WO 00/69448 A1 & EP 1183035 A1 | 1-5,7,8<br>6-8 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 September, 2004 (16.09.04) | 12 October, 2004 (12.10.04) |
| Name and mailing address of the ISA/<br>Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/009386

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | LU, D. et al., 'Adult bone marrow stromal cells administered intravenously to rats after traumatic brain injury migratein to brain and improve neurological outcome.', Neuroreport, (2001), Vol.12, No.3, pages 559 to 563, all references, abstract | 1-5,7,8<br>6-8 |
| X<br>Y | CHEN, J. et al., 'Therapeutic benefit of intravenous administ ratino of bone marrow stromal cells after cerebral ischemia in rats.', Stroke, (2001), Vol.32, No.4, pages 1005 to 1011, all references, abstract | 1-5,7,8<br>6-8 |
| X<br>Y | CHU, K. et al., 'Human neural stem cells can migrate, differentiate, and integrate after intravenous transplantation in adult rats with transient forebrain ischemia.', Neurosci.Lett., 05 June, 2003 (05.06.03), Vol.343, No.2, pages 129 to 133, all references, abstract | 1-5,7,8<br>6-8 |
| X<br>Y | AKIYAMA Y., et al., 'Remyelination of the spinal cord following intravenous delivery of bone marrow cells.', Glia, (2002), Vol.39, No.\ ) pages 229 to 236, all references, abstract | 1-5,7,8<br>6-8 |
| X<br>Y | WO 01/66698 A1 (CRYO-CELL INT. INC.), 13 September, 2001 (13.09.01), All references, page 56, 2nd line from the bottom to page 71<br>& AU 2001/43464 B & US 2002/28510 A<br>& EP 1263930 A1 & US 2003/36729 A<br>& JP 2004-500824 A | 1-5,7,8<br>6-8 |
| X<br>Y | MAHMOOD, A. et al,. 'Treatment of traumatic brain injury in female rats with intravenous administration of bone marrow stromal cells.', Neurosurgery, (2002), Vol.49, No.5, pages 1196 to 1203, all references, abstract | 1-5,7,8<br>6-8 |
| X<br>Y | DATABASE MEDLINE ON STN, (2002), abstract no. 2002333228 & LU, D. et al., 'Intravenous administration of human unbilical cord blood reduces neurological deficit in the rat after traumatic brain injury.', Cell Transplant., (2002), Vol.11, No.3, pages 275 to 281 | 1-5,7,8<br>6-8 |
| X<br>Y | DATABASE BIOSIS ON STN, (2001), abstract no. 200100547979 & AKIYAMA, Y. et al., 'Remyelination of spinal cord axons by intravenous delivery of bone marrow cells.', Society for Neuroscience Abstracts, (2001), Vol.27, No.2, page 1562 | 1-5,7,8<br>6-8 |

Form PCT/ISA/210 (continuation of second sheet) (January 2004)

**INTERNATIONAL SEARCH REPORT**

International application No.
PCT/JP2004/009386

C (Continuation). DOCUMENTS CONSIDERED TO BE RELEVANT

| Category* | Citation of document, with indication, where appropriate, of the relevant passages | Relevant to claim No. |
|---|---|---|
| Y | WO 03/25167 A2 (CENT TRANSLATIONAL RES IN CANCER), 27 March, 2003 (27.03.03), All references; Claims; examples IX & AU 2002/325758 B & EP 1456358 A2 | 6-8 |
| P,Y | DATABASE BIOSIS ON STN, (2003), abstract no. 200300315449 & LU, P. et al., 'Trans plantation of bone marrow stromal cells (MSCS), and BDNF-transduced MSCS promotoes robust axonal growth after spinal cord injury.', Society for Neuroscience Abstract Viewer and Itinerary Planner, (2002), Vol.202 abstract no.634.11 http://sfn.scholarone.com. | 6-8 |
| Y | KEANE-MOORE, MA. et al., 'Human mesenchymal stem cells can begenetically modified to function as antigen presenting cells.' Blood, (1998), Vol.92, No.10, suppl.1 part 1 to 2, p.338A, abstract #1388 | 6-8 |

Form PCT/ISA/210 (continuation of second sheet) (January 2004)

**INTERNATIONAL SEARCH REPORT**

International application No.
PCT/JP2004/009386

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 9-13
because they relate to subject matter not required to be searched by this Authority, namely:
The inventions 9 to 13 involve embodiments concerning methods for treatment of the human body by therapy and thus relates to a subject matter which this International Searching Authority is not required, under the provisions of Article 17(2)(a)(i) of the PCT (continued to extra sheet.)

2. ☐ Claims Nos.:
because they relate to parts of the international application that do not comply with the prescribed requirements to such an
extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable
claims.

2. ☐ As all searchable claims could be searched without effort justifying an additional fee, this Authority did not invite payment of any additional fee.

3. ☐ As only some of the required additional search fees were timely paid by the applicant, this international search report covers
only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
☐ The additional search fees were accompanied by the applicant's protest.
☐ No protest accompanied the payment of additional search fees.

Form PCT/ISA/210 (continuation of first sheet (2)) (January 2004)

**INTERNATIONAL SEARCH REPORT**

International application No.
PCT/JP2004/009386

Continuation of Box No.II-1 of continuation of first sheet(2)

and Rule 39.1(iv) of the Regulations under the PCT, to search.

Form PCT/ISA/210 (extra sheet) (January 2004)

SEQUENCE LISTING

<110> Renomedix Institute Inc.
Mitsui Sumitomo Insurance Company, Limited
TEIKOKU HORMONE MFG. CO., LTD.
Hitachi, Ltd.

<120> Pharmaceutical composition for brain diseases, that comprises mesodermal and mesenchymal cells

<130> SSK-X0301Y1P

<150> JP 2003-185260
<151> 2003-06-27

<150> JP 2003-432329
<151> 2003-12-26

<160> 2

<170> PatentIn version 3.1

<210> 1
<211> 40
<212> DNA
<213> Artificial

<220>
<223> an artificially synthesized primer sequence

<400> 1
cggaattcca ccatgaccat ccttttcctt actatggtta 40

<210> 2
<211> 34

<212> DNA
<213> Artificial

<220>
<223> an artificially synthesized primer sequence

<400> 2
ccagatctat cttcccctt taatggtcaa tgta 34

SEQUENCE LISTING

<110> Renomedix Institute Inc.
Mitsui Sumitomo Insurance Company, Limited
TEIKOKU HORMONE MFG. CO., LTD.
Hitachi, Ltd.

<120> Pharmaceutical composition for brain diseases, that comprises mesodermal and mesenchymal cells

<130> SSK-X0301Y1P

<150> JP 2003-185260
<151> 2003-06-27

<150> JP 2003-432329
<151> 2003-12-26

<160> 2

<170> PatentIn version 3.1

<210> 1
<211> 40
<212> DNA
<213> Artificial

<220>
<223> an artificially synthesized primer sequence

<400> 1
cggaattcca ccatgaccat ccttttcctt actatggtta 40

<210> 2
<211> 34

<212> DNA
<213> Artificial

<220>
<223> an artificially synthesized primer sequence

<400> 2
ccagatctat cttccccttt taatggtcaa tgta 34